(19) Europäisches Patentamt
European Patent Office
Office européen des brevets

(11) Publication number: **0 348 513 A1**

(12) # EUROPEAN PATENT APPLICATION

published in accordance with Art. 158(3) EPC

(21) Application number: **88910106.9**

(22) Date of filing: **22.11.88**

Data of the international application taken as a basis:

(86) International application number: **PCT/JP 88/01180**

(87) International publication number: **WO 89/04824 (01.06.89 89/12)**

(51) Int. Cl.⁴: **C 07 C 133/08**, C 07 C 159/00, C 08 F 226/00, C 09 K 3/00

(30) Priority: **24.11.87 JP 295861/87**
**08.01.88 JP 2995/88**

(43) Date of publication of application: **03.01.90 Bulletin 90/1**

(84) Designated Contracting States: **AT BE CH DE FR GB IT LI LU NL SE**

(71) Applicant: **EARTH CHEMICAL CO., LTD., 3218-12, Sakoshi, Ako-shi Hyogo 678-01 (JP)**

(72) Inventor: **NAKAYAMA, Tohru, 109-10, Kariya, Ako-shi Hyogo 678-02 (JP)**
Inventor: **KAJIFUSA, Noriyuki, 3143-33, Ozaki, Ako-shi Hyogo 678-02 (JP)**
Inventor: **HORINAKA, Akio, 215-6, Manago, Ako-shi Hyogo 678-01 (JP)**
Inventor: **FUJII, Setsuro, 323, Sanjosagaru Ebiya-cho Gokomachidori, Nakagyo-ku Kyoto-shi Kyoto 604 (JP)**

(74) Representative: **Türk, Gille, Hrabal, Bruckner Strasse 20, D-4000 Düsseldorf 13 (DE)**

(54) **STYRENE DERIVATIVE, POLYSTYRENE DERIVATIVE COMPRISING THE SAME, PROCESS FOR PREPARING SAID POLYSTYRENE DERIVATIVE, AND ULTRAVIOLET ABSORBER COMPRISING SAID STYRENE DERIVATIVE OR POLYSTYRENE DERIVATIVE.**

(57) The styrene derivative of the present invention is characterized by comprising a compound represented by general formula (I), wherein at least one of $R_1$, $R_2$, $R_3$, $R_4$ and $R_5$ is a carboxyl group, an alkyl group having 1 to 5 carbon atoms, a hydroxyalkyl group having 1 to 5 carbon atoms, a carboxyalkyl group having 2 to 6 carbon atoms, an alkoxycarbonyl group having 2 to 6 carbon atoms, an alkoxycarbonylalkyl group having 2 to 6 carbon atoms, or an aryl group and the remainders are each a hydrogen atom or an alkyl group having 1 to 5 carbon atoms, and $R_6$ is a sulfur atom or an oxygen atom. The styrene derivative has excellent capability of absorbing an ultraviolet ray having a wavelength form 290 nm, i.e., the shortest wavelength contained in sunrays reaching the surface of the earth, to 360 nm having an effect of causing the change in the plastics. The polystyrene derivative of the present invention is characterized by maintaining high water solubility through polymerization of said styrene derivative and causing no skin absorption, i.e., having high safety to the human body. Since the ultraviolet absorption wavelength region is the same as that which brings about a sunburn of the skin, which renders the polystyrene derivative suitable for use as an ultraviolet absorber. Further, according to the process for preparing the polystyrene derivative of the present invention, the polystyrene derivative can be purified in the stage of a monomer, which makes it possible to provide a polystyrene derivative having high quality and high stability.

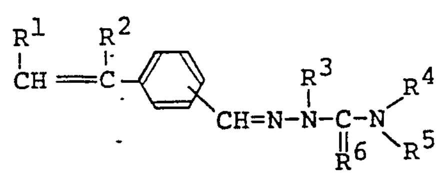

(I)

EP 0 348 513 A1

TITLE ... see front page



DESCRIPTION

STYRENE DERIVATIVE, POLYSTYRENE DERIVATIVE COMPRISING THE STYRENE DERIVATIVE AND METHOD FOR PREPARING THE SAME AND SUNSCREENING AGENT OF WHICH ACTIVE INGREDIENT IS THE SAME

TECHNICAL FIELD

The present invention relates to a novel styrene derivative, a polystyrene derivative and a method for preparing the same and a sunscreening agent or an ultraviolet absorber (hereinafter referred to as "a sunscreening agent") of which active ingredient is the styrene derivative or the polystyrene derivative.

BACKGROUND ART

Recently, a suntan cream and the like containing a sunscreening agent such as p-amino benzoic acid, an ester or a salt thereof as an active ingredient for preventing so-colled sunburn (acute solar dermatitis) have been developed. Usually, p-amino benzoic acid has been used together with a light scattering agent such as titanium oxide since p-amino benzoic acid does not absorb ultraviolet rays having a wavelength of nearby 305 nm which mainly effects erythema caused by sunburn. However, p-amino benzoic acid is not preferable since p-amino benzoic acid is absorbed through the skin and circulated in the body so that some people may suffer from diseases. Since the suntan cream and the like containing a light scattering agent are colored in many cases, when the suntan cream is rubbed in the skin of a face or hands, the appearance of them is not preferable.

Accordingly, a polyamino acid derivative composed of a polyamino acid and an aminobenzaldehyde (thio)semicarbazone derivative has been developed as a safe sunscreening agent for human bodies, which is not absorbed through the skin and does not have the defects of the above suntan cream and the like (refer to Japanese

Unexamined Patent Publication No. 65865/1981).

However, since aminobenzaldehyde(thio)-semicarbazone is insoluble in water, the obtained polyamino acid derivative becomes insoluble in water when the content of aminobenzaldehyde(thio)semicarbazone derivative is increased, that is, ultraviolet absorptive property is heightened. Therefore, a sunscreening agent having enough ultraviolet absorptive property could not be obtained in consideration of preparation of a sunscreening agent since there is a necessity to decrease the content of aminobenzaldehyde(thio)semicarbazone derivative in order to dissolve the polyamino acid derivative in water.

Further, aminobenzaldehyde(thio)semicarbazone derivative, which is prepared by combining a polyamino acid and an aminobenzaldehyde(thio)semicarbazone derivative, has defects that the polyamino acid which is a raw material is expensive, that purity thereof is low as the result of low reactivity and side reaction, and that the structure thereof cannot be apparently determined since the aminobenzaldehyde(thio)semicarbazone derivative is prepared by reacting a high molecular compound with a low molecular compound.

Accordingly, the present inventors have researched to give a sunscreening agent having an absorption band of wavelength corresponding to that of ultraviolet rays included in the direct rays of the sun and large absorption intensity of ultraviolet rays in view of the above conventional techniques. As the results, the inventors have found a novel polystyrene derivative having excellent ultraviolet absorptive property within a range of ultraviolet rays corresponding to the ultraviolet rays included in the direct rays of the sun, and that the polystyrene derivative can be suitably used as a sunscreening agent.

When organic compounds, particularly, plastics are used in an environment wherein the organic compounds are exposed to ultraviolet rays, a sunscreening agent

comprising, for instance, salicylic acid derivatives, benzophenone derivatives, benztriazole derivatives, and the like is added to the plastics and the like in industrial uses since the organic compounds are easily oxidized and deteriorated as the result of being exposed to ultraviolet rays.

However, ultraviolet absorptive property of these sunscreening agents is not so large. Then, as their researches of a styrene derivative which is a raw material of the polystyrene derivative, it is found that the styrene derivative is excellent in ultraviolet absorptive property.

Further, the present inventors have researched in consideration of the above-mentioned conventional techniques, and as the results, the inventors have found a novel polystyrene derivative which can be cheaply and easily prepared and has excellent water solubility and a method for preparing the polystyrene derivative, and also have found that the polystyrene derivative can be suitably used as a sunscreening agent and have completed the present invention.

DISCLOSURE OF INVENTION

The present ivention relates to

(1) a styrene derivative represented by the general formula (I):

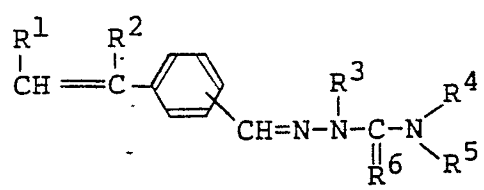

(I)

wherein at least one of $R^1$, $R^2$, $R^3$, $R^4$ and $R^5$ is carboxyl group, an alkyl group having 1 to 5 carbon atoms, a hydroxyalkyl group having 1 to 5 carbon atoms, a carboxyalkyl group having 2 to 6 carbon atoms, an alkoxycarbonyl group having 2 to 6 carbon atoms, an alkoxycarbonylalkyl group having 2 to 6 carbon atoms or

an aryl group, the residual groups of $R^1$, $R^2$, $R^3$, $R^4$ and $R^5$ are hydrogen atom or an alkyl group having 1 to 5 carbon atoms, $R^6$ is sulfur atom or oxygen atom;

(2) a polystyrene derivative represented by the general formula (II):

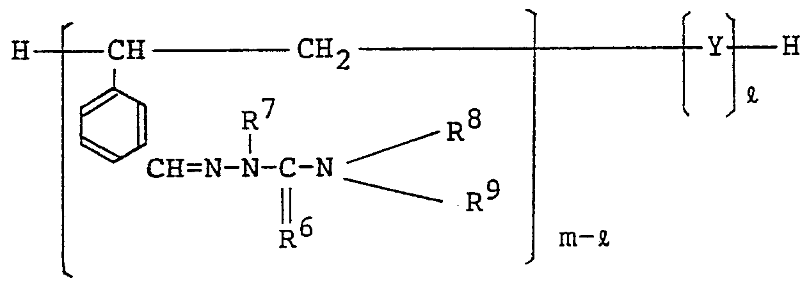

(II)

wherein at least one of $R^7$, $R^8$ and $R^9$ is carboxyl group, a carboxyalkyl group, an alkoxycarbonyl group or an alkoxycarbonylalkyl group having 2 to 6 carbon atoms, the residual groups of $R^7$, $R^8$ and $R^9$ are hydrogen atom, a lower alkyl group having 1 to 5 carbon atoms or an aryl group, $R^6$ is sulfur atom or oxygen atom, Y is 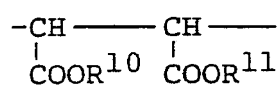 (wherein $R^{10}$ and $R^{11}$ are hydrogen atom or an alkyl group having 1 to 5 carbon atoms, respectively), 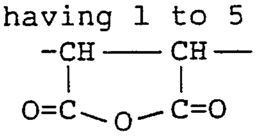, 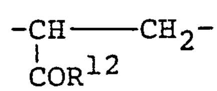 (wherein $R^{12}$ is $-OH$, $-NH_2$ or $-OC_pH_{2p+1}$ wherein p is an integer of 1 to 4), 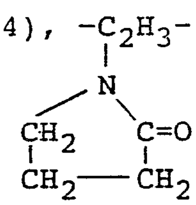 or 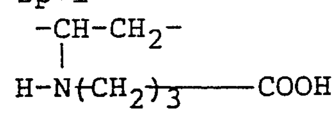, m is an integer of 10 to 1500, ℓ is an integer of 0 to 1100 which satisfies m>ℓ,

(3) a method for preparing the polystyrene derivative represented by the above general formula (II), characterized by using an azo-compound or a peroxide as a polymerization initiator and homopolymerizing in a solution, a styrene derivative represented by the general formula (III):

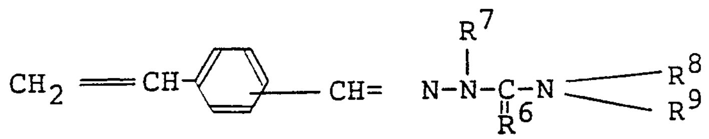 (III)

wherein $R^6$ to $R^9$ are the same as mentioned above, or copolymerizing at least two kinds of the styrene derivatives, or copolymerizing the styrene derivative and a compound containing unsaturated aliphatic groups having at least one double bond between carbon atoms, and at least one of the unsaturated aliphatic groups being terminated by carboxyl group or a salt thereof, anhydride, ester, amide or imide,

(4) a sunscreening agent containing as an active ingredient, the polystyrene derivative represented by the general formula (II),

(5) a polystyrene derivative represented by the general formula (IV):

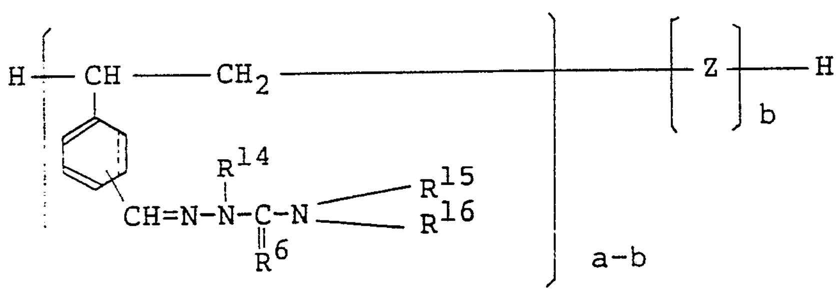 (IV)

wherein $R^{14}$, $R^{15}$ and $R^{16}$ are hydrogen atom, an alkyl group, a hydroxyalkyl group having 1 to 5 carbon atoms or an aryl group, respectively, $R^6$ is sulfur atom or oxygen atom, Z is 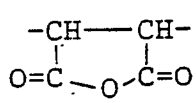 , 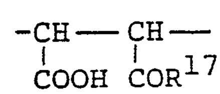 (wherein $R^{17}$ is $-OH$, $-NH_2$ or $-OC_pH_{2p+1}$ wherein p is an integer of 1 to 4, or 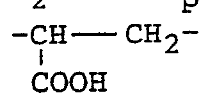 , a is an integer of 10 to 1500, b is an integer which satisfies that b/a is 0.2 to 0.9,

(6) a method for preparing the polystyrene derivative represented by the general formula (IV), characterized by using an azo-compound or a peroxide as a polymerization initiator and copolymerizing at least one kind of a

styrene derivative represented by the general formula (V):

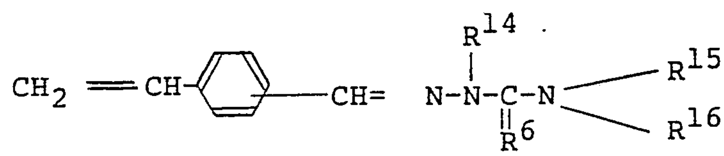

(V)

wherein $R^{14}$, $R^{15}$ and $R^{16}$ are the same as mentioned above, and a compound containing unsaturated aliphatic groups having at least one double bond between carbon atoms, at least one of the unsaturated aliphatic groups being terminated by carboxyl group or a salt thereof, anhydride, ester, amide or imide, and

(7) a sunscreening agent containing as an active ingredient, the polystyrene derivative represented by the general formula (IV).

## BRIEF DESCRIPTION OF THE DRAWINGS

Figs. 1 to 12 are graphs showing ultraviolet absorption spectrum of the styrene derivative obtained in Examples 1 to 12 of the present invention, respectively;

Fig. 13, Fig. 15, Fig. 17, Fig. 19, Fig. 21, Fig. 23, Fig. 25, Fig. 27, Fig. 29, Fig. 31, Fig. 33 and Fig. 35 are graphs showing infrared absorption spectrum of the polystyrene derivative obtained in Examples 21 to 32 of the present invention, respectively; Fig. 14, Fig. 16, Fig. 18, Fig. 20, Fig. 22, Fig. 24, Fig. 26, Fig. 28, Fig. 30, Fig. 32, Fig. 34 and Fig. 36 are graphs showing ultraviolet abosorption spectrum of the polystyrene derivative obtained in Examples 21 to 32 of the present invention, respectively; Fig. 38, Fig. 40, Fig. 42, Fig. 43, Fig. 45, Fig. 47, Fig. 49 and Fig. 51 are graphs showing infrared absorption spectrum of the polystyrene derivative obtained in Examples 46 to 53 of the present invention, respectively, Fig. 37, Fig. 39, Fig. 41, Fig. 44, Fig. 46, Fig. 48, Fig. 50 and Fig. 52 are graphs showing ultraviolet absorption spectrum of the polystyrene derivative obtained in Examples 46 to 53 of

the present ivnention, respectively.

BEST MODE FOR CARRYING OUT THE INVENTION

An styrene derivative of the present invention can be suitably used as a sunscreening agent since the styrene derivative of the present invention has desired absorption wavelength of ultraviolet rays and large molar extinction coefficient as a sunscreening agent.

The styrene derivative of the present invention is a compound represented by the general formula (I):

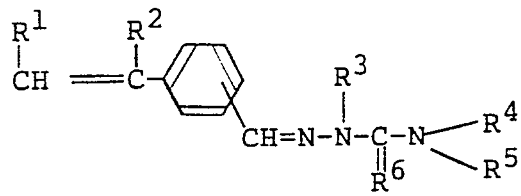

(I)

wherein at least one of $R^1$, $R^2$, $R^3$, $R^4$ and $R^5$ is carboxyl group, an alkyl group having 1 to 5 carbon atoms, a hydroxyalkyl group having 1 to 5 carbon atoms, a carboxyalkyl group having 2 to 6 carbon atoms, an alkoxycarbonyl group having 2 to 6 carbon atoms, an alkoxycarbonylalkyl group having 2 to 6 carbon atoms or an aryl gorup, the residual groups of the above $R^1$, $R^2$, $R^3$, $R^4$ and $R^5$ are hydrogen atom or an alkyl group having 1 to 5 carbon atoms, $R^6$ is sulfur atom or oxygen atom.

In the general formula (I), (thio)semicarbazone group may be sited at any position of o, m or p to the vinyl group bonded to the benzene ring. However, it is preferable that (thio)semicarbazone group is sited at p position since ultraviolet absorptive property becomes larger at that case.

In the general formula (I), at least one of $R^1$, $R^2$, $R^3$, $R^4$ and $R^5$ is hydrogen atom, carboxyl group, an alkyl group having 1 to 5 carbon atoms, a hydroxyalkyl group having 1 to 5 carbon atoms, a carboxyalkyl group having 2 to 6 carbon atoms, an alkoxycarbonyl group having 2 to 6 carbon atoms, an alkoxycarbonylalkyl group having 2 to 6 carbon atoms or an aryl group, and the

residual groups of the above $R^1$, $R^2$, $R^3$, $R^4$ and $R^5$ are hydrogen atom or an alkyl group having 1 to 5 carbon atoms.

It is preferable that at least one of the above $R^1$ to $R^5$ is carboxyl group, a carboxyalkyl group, an alkoxycarbonyl group or an alkoxycarbonylalkyl group since the styrene derivative of the present invention reacts with an alkali metal hydroxide and the like to form a carboxylic acid salt, so that water solubility is imparted to the styrene derivative. It is not preferable that all groups of $R^1$ to $R^5$ are hydrogen atoms since stability is deteriorated and absorptive property of ultraviolet rays is lowered when used in a solution.

The above $R^6$ is sulfur atom or oxygen atom. It is preferable that the $R^6$ is sulfur atom since the styrene derivative in which $R^6$ is sulfur atom has larger ultraviolet absorptive property than that of the styrene derivative in which $R^6$ is oxygen atom.

An example of the methods for preparing the styrene derivative of the present invention is explained hereinafter.

As the first method, there is a method wherein monomethylhydrazine and methylisothiocyanate acetate are reacted, then the obtained reaction product is reacted with p-vinylbenzaldehyde to give a styrene derivative, as represented by, for instance, the reaction formula (VI):

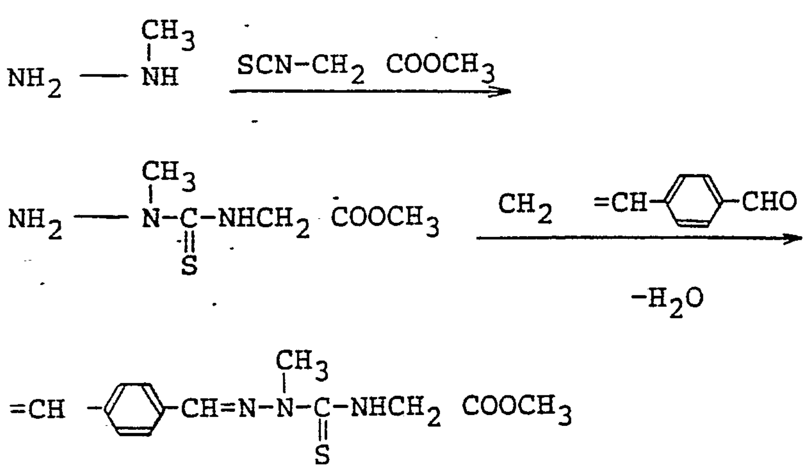


(VI)

and as the second method, there is a method wherein benzaldehyde and monomethylhydrazine are reacted, then the obtained reaction product is reacted with methylisothiocyanate acetate to give a styrene derivative as represented by, for instance, the reaction formula (VII):

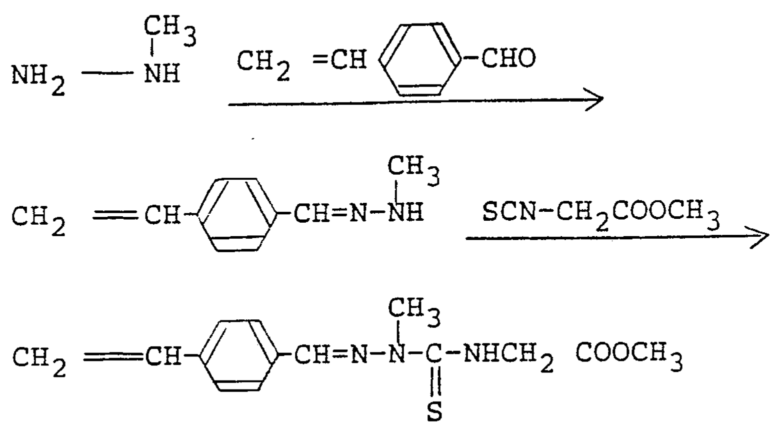


(VII)

The thus obtained styrene derivative of the present invention is collected by recrystallizing from a solvent such as methanol, ethanol, isopropanol, chloroform, or by passing the styrene derivative through a silica gel column to purify and isolate.

The styrene derivative of the present invention has excellent properties as a sunscreening agent since the styrene derivative absorbs ultraviolet rays in the range of 290 to 360 nm, which covers the wavelength from 290 nm which is the shortest wavelength contained in the sunlight reaching the surface of the earth to 360 nm which affects plastics in deterioration , and possesses high ultraviolet absorptive property, that is, molar extinction coefficient of 29,000 to 44,000.

Examples of a method for using styrene derivative of the present invention as a sunscreening agent are, for instance, a method for using by mixing and melting the styrene derivative with plastics as occasion demands and a method for using by coating the styrene derivative as a solution.

It is preferable that the styrene derivative is added in an amount of 0.01 to 20 % by weight,

particularly 0.1 to 5 % by weight based upon plastics when the styrene derivative of the present invention is used by mixing and melting with the plastics.

Further, the styrene derivative of the present invention can be also used as a homopolymer or a copolymer thereof prepared by radical polymerization of the vinyl group thereof.

The polystyrene derivative of the present invention is explained hereinafter. The polystyrene derivative can be suitably used as a sunscreening agent since the polystyrene derivative has an absorption wavelength desired as a sunscreening agent and large molar extinction coefficient.

The polystyrene derivative of the present invention is a compound represented by the general formula (II):

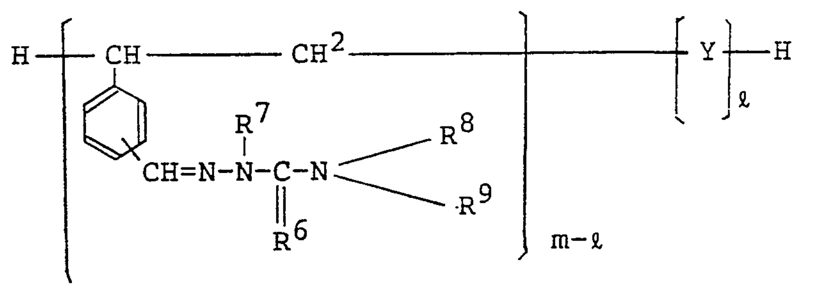

(II)

wherein at least one of $R^7$, $R^8$ and $R^9$ is carboxyl group, a carboxyalkyl group having 2 to 6 carbon atoms, an alkoxycarbonyl group having 2 to 6 carbon atoms or an alkoxycarbonylalkyl group having 2 to 6 carbon atoms, the residual groups of $R^7$, $R^8$ and $R^9$ are hydrogen atom, a lower alkyl group having 1 to 5 carbon atoms or an aryl group, $R^6$ is sulfur atom or oxygen atom, Y is

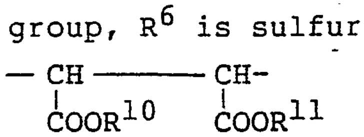

wherein $R^{10}$ and $R^{11}$ are hydrogen atom or an alkyl group having 1 to 5 carbon atoms, respectively,

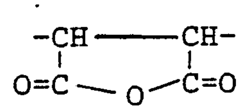

,

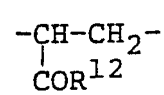

wherein $R^{12}$ is $-OH$, $-NH_2$ or $-OC_pH_{2p+1}$ wherein p is an integer of 1 to 4,

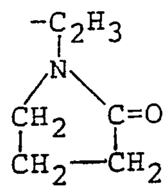 or 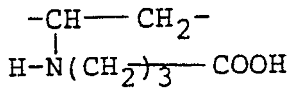 ,

m is an integer of 20 to 1500, ℓ is an integer of 0 to 1100 satisfying m>ℓ.

In the general formula (II), a partial constitutional formula:

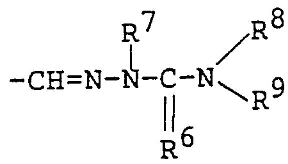

is a (thio)semicarbazone group which is bonded to the benzene nucleus. The (thio)semicarbazone group can be bonded to the alkylene group, which is bonded to the benzene nucleus, at any of o, m and p-position. It is preferable that the (thio)semicarbazone group is bonded to the benzene nucleus at p-position since ultraviolet absorptive property is heightened in that case. In the formula, at least one of $R^7$, $R^8$ and $R^9$ is carboxyl group, a carboxyalkyl group having 2 to 6 carbon atoms, an alkoxycarbonyl group having 2 to 6 carbon atoms or an alkoxycarbonylalkyl group having 2 to 6 carbon atoms, the residual groups of $R^7$, $R^8$ and $R^9$ are hydrogen atom, a lower alkyl group having 1 to 5 carbon atoms or an aryl group. It is particularly preferable that the lower alkyl group has 1 to 3 carbon atoms. The reason why it is necessary that at least one of $R^7$, $R^8$ and $R^9$ is carboxyl group, a carboxyalkyl group having 2 to 6 carbon atoms, an alkoxycarbonyl group having 2 to 6 carbon atoms or an alkoxycarbonylalkyl group having 2 to 6 carbon atoms in the present invention is to impart high water solubility. As mentioned above, $R^6$ is sulfur atom or oxygen atom. Examples of the (thio)semicarbazone group are, for instnace,

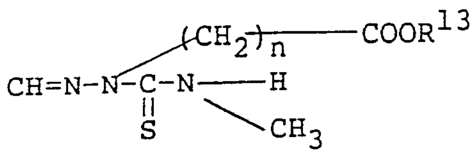

,

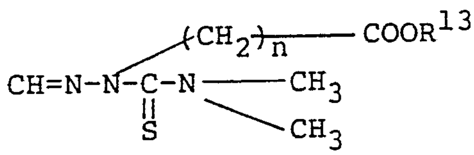

,

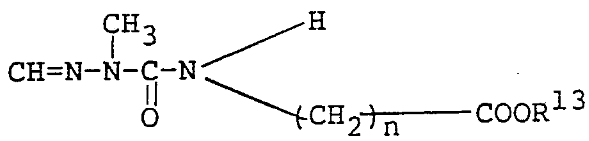

,

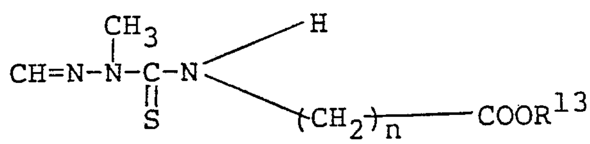

,

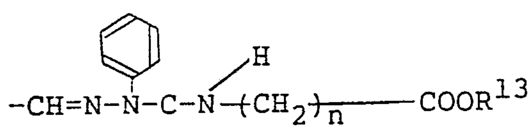

wherein $R^{13}$ is hydrogen atom, a lower alkyl group having 1 to 5 carbon atoms, ammonium group or an alkali metal, n is an integer of 0 to 3, and the like. It is preferable that the above $R^{13}$ is, for instance, a salt of alkaline metal such as lithium, sodium or potassium, ammonia, ethanolamine, propanolamine, an ammonium salt such as triethylamine in order to heighten water solubility of the obtained polystyrene derivative.

The reason why the recurring unit of the formula: $-[Y]-$ is introduced into the polystyrene derivative represented by the above general formula (II) as occasion demands is to impart more excellent water solubility to the obtained polystyrene derivative. Accordingly, depending on the kinds of the polystyrene derivative of the present invention, the polystyrene derivative can be substantially composed of the recurring unit of the formula:

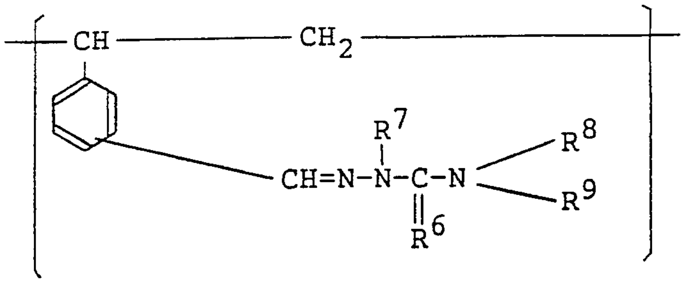

,

and when the polystyrene derivative of the present invention is used as a sunscreening agent required for high water solubility, it is preferable that the recurring unit represented by the formula: $\{Y\}$ is contained in the polystyrene derivative. It is preferable that the degree of polymerization of the recurring unit represented by the above formula: $\{Y\}$ is 0 to 1100, particularly 0 or 30 to 900. Also, it is a matter of course that the recurring unit represented by the formula: $\{Y\}$ can be contained although the polystrene derivative of the present invention is used as a sunscreening agent not required for water solubility.

It is preferable that the degree of polymerization (m) of the polystyrene derivative of the present invention is not less than 10 in order to avoid the absorption of the polystyrene derivative through the skin and not more than 1500 in order to avoid the lowering of utility caused by being imparted the extremely high viscosity. The degree of polymerization is more preferably 20 to 1100.

The polystyrene derivative of the present invention is a homopolymer or a random copolymer prepared by homopolymerizing a styrene derivative represented by the general formula (III):

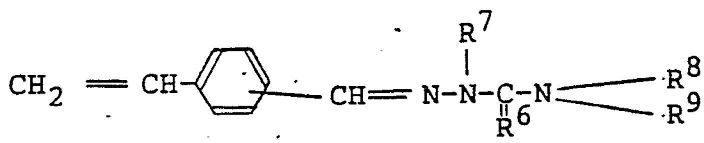

(III)

wherein $R^7$, $R^8$, $R^9$ and $R^6$ are the same as mentioned above, respectively, or copolymerising at least two of the above styrene derivatives, or copolymerising the

styrene derivative and a compound having unsaturated aliphatic groups having at least one double bond between carbon atoms and of which at least one unsaturated aliphatic group is terminated by carboxyl group or a salt thereof, anhydride, ester, amide or imide (hereinafter referred to as "monomer A"), by using an azo-compound or a peroxide as a polymerization initiator.

Examples of the azo-compound used as a polymerization initiator are, for instnace, α, α'-azobisisobutyronitrile, 2,2'-azobis (2,4-dimethylvaleronitrile), and the like. Examples of the peroxide are, for instance, ammonium persulfate, and the like.

The monomer A is added to the polystyrene derivative in order to improve water solubility of the polystyrene derivative. Examples of the monomer A are, maleic acid, maleic anhydride, fumaric acid or acrylic acid, a lower alkyl ester having 1 to 5 carbon atoms of maleic acid, fumaric acid or acrylic acid, N-vinylpyrrolidone, and the like.

The polymerization reaction is conducted in a solution. As the solvent, a solvent which is used in radical polymerization can be used. The solvent, which can dissolve starting monomers and a resulting polymer, are preferable. Examples of the solvent are, for instance, dimethylformamide, dioxane, tetrahydrofuran, N-methylpyrrolidone, and the like.

Also, the temperature at the polymerization can be room temperature to 100°C as well as the temperature at the general radical polymerization. It is also preferable that the atmosphere at polymerization is replaced by nitrogen gas.

The water solubility is imparted to the obtained polystyrene derivative by reacting the polystyrene derivative with a base such as hydroxides of alkaline metals, ammonia, amine in water to form a salt of carboxylic acid, or hydrolyzing the esters and amides thereof with a strong base such as hydroxides of alkaline

metals in water to form a salt of carboxylic acid.

One example of the method for preparing the polystyrene derivative of the present invention is explained hereinafter.

As the first method, there is a method for preparing comprising polymerizing or copolymerizing a styrene derivative containing a carboxyalkyl group. As the second method, there is a method for preparing comprising polymerizing or copolymerizing a styrene derivative containing an alkoxycarbonylalkyl group and then hydrolyzing the polymer or copolymer to convert the alkoxycarbonylalkyl group into a carboxyalkyl group. One embodiment of the reaction is explained hereinafter.

(One embodiment of the first method)

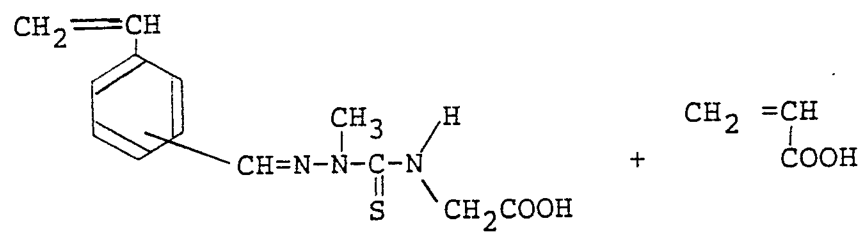

<u>Polymerization intiator (α ,α' -azobisisobutyronitrile)</u>
Dimethylformamide (DMF)

A random copolymer represented by

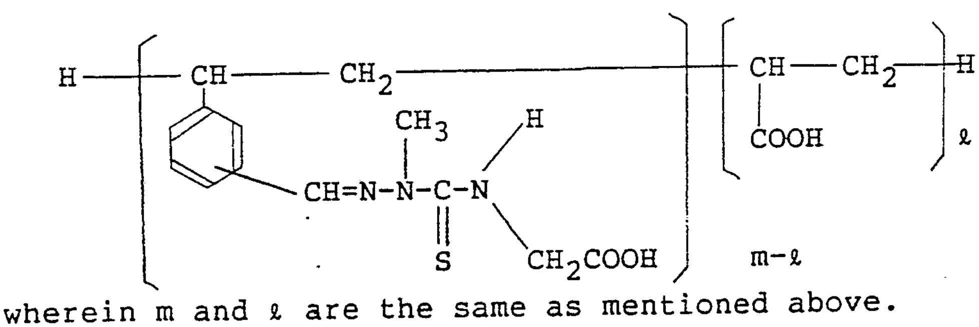

wherein m and ℓ are the same as mentioned above.

(The second method)

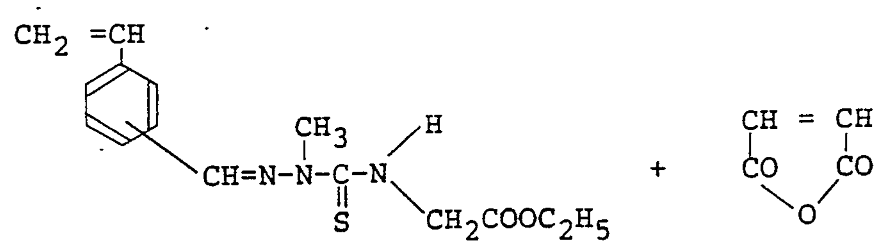

Polymerization initiator (α, α' -azobisisobutyronitrile)
Dimethyformamide (DMF)

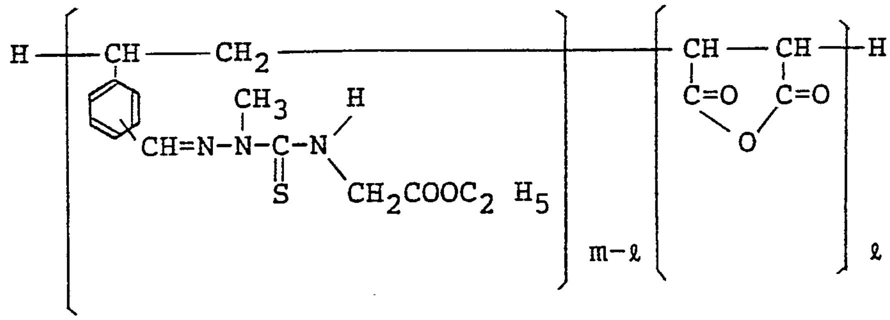

NaOH

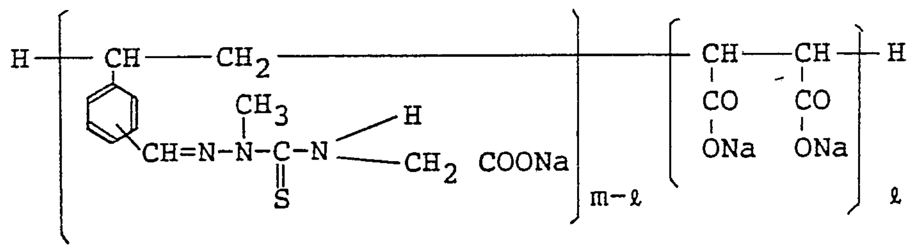

HCℓ

A random copolymer represented by

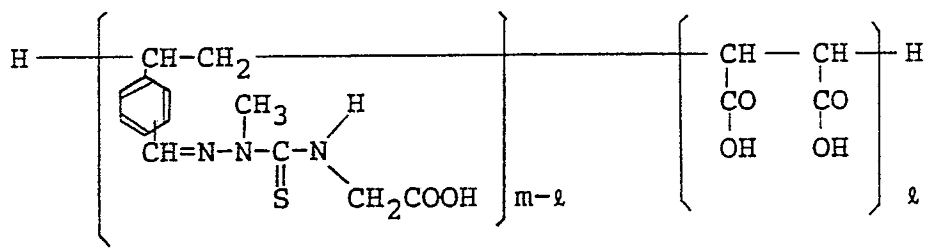

wherein m and ℓ are the same as mentioned above.

The thus obtained polystyrene derivative of the present invention is extremely suitably used as a sunscreening agent for preventing the skin from sunburn since the absorption wavelength of ultraviolet rays of the polystyrene derivative is within a range of 290 to 350 nm, which nearly corresponds to the most effective wavelength causing erythema (295 to 330 nm).

The polystyrene derivative exhibits hydrophilic property since in the general formula (II), at least one of $R^7$ to $R^9$ is a group having hydrophilic property. Accordingly, the Y group having hydrophilic property may not be existed in the polystyrene derivative.

Also, the polystyrene derivative of the present

invention can be either a random copolymer or a block copolymer comprising a portion represented by the formula:

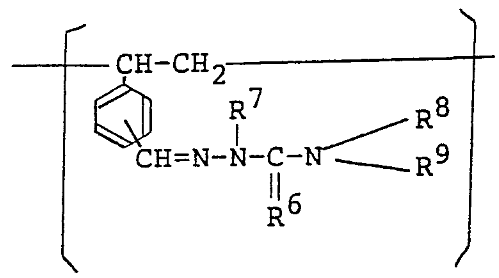

wherein $R^6$ to $R^9$ are the same as mentioned above and a portion represented by the formula: $-\!\!\left[ Y \right]\!\!-$, wherein Y is the same as mentioned above.

As the polystyrene derivative of the present invention, there is a polystyrene derivative represented by the general formula (IV):

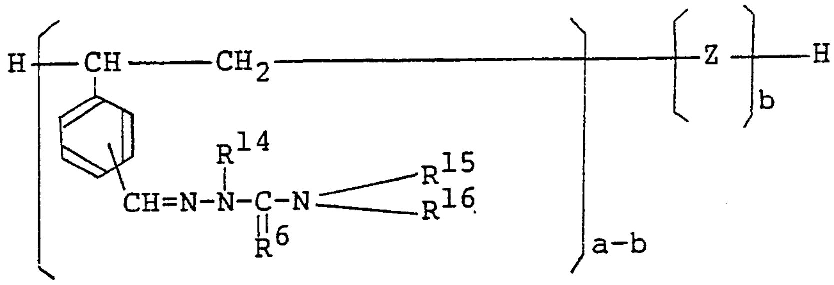

(IV)

wherein $R^{14}$, $R^{15}$ and $R^{16}$ are hydrogen atom, an alkyl group having 1 to 5 carbon atoms, a hydroxyalkyl group or an aryl group, $R^6$ is sulfur atom or oxygen atom, Z is

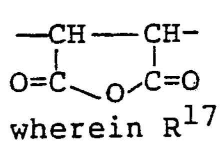 , 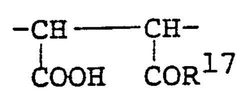

wherein $R^{17}$ is $-OH$, $-NH_2$ or $-OC_pH_{2p+1}$ (wherein p is an integer of 1 to 4) or 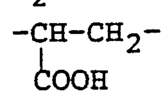 ,

a is an integer of 10 to 1500 which reveals the degree of polymerization of the polystyrene derivative, and b is an integer which satisfies that b/a is 0.2 to 0.9 and represents the number of a recurring unit of Z when the degree of polymerization is "a", and the polystyrene derivative can be suitably used as a sunscreening agent since the polystyrene derivative has desired absorption

wavelength as a sunscreening agent and a large extinction coefficient.

In the general formula (IV), a partial constituional formula:

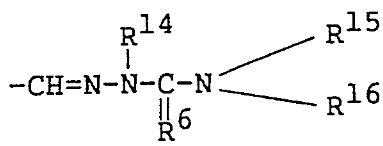

is a (thio)semicarbazone group bonded to the benzene nucleus. The (thio)semicarbazone group bonded to the benzene nucleus can be at any position of o, m and p to the vinyl group bonded to the benzene nucleus. It is particularly preferable that the (thio)semicarbazone group is at the p-position since ultraviolet absorptive property is heightened when the (thio)semicarbazone group is at the p-position. The $R^6$ is sulfur atom or oxygen atom.

In the present invention, in the formula, $R^{14}$, $R^{15}$ and $R^{16}$ are hydrogen atom, an alkyl group or a hydroxyalkyl group having 1 to 5 carbon atoms, or an aryl group, respectively, and the chemical structure of the partial constitutional formula is considered to be hydrophobic property. However, the polystyrene derivative has water solubility and high ultraviolet absorptive property owing to the carboxylic acid of the copolymerized portion of Z which is explained hereinafter.

The reason why the recurring unit of $-[Z]-$ is introduced into the polystyrene derivative of the general formula (IV) is to impart water solubility to the obtained polystyrene derivative.

The Z is, as mentioned above,

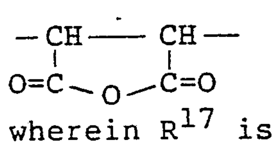 ,

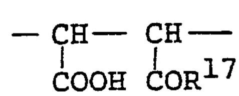

wherein $R^{17}$ is $-OH$, $-NH_2$ or $-OC_pH_{2p+1}$ (wherein p is an integer of 1 to 4) or 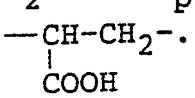.

However, it is preferable that $R^{17}$ is, for example, a salt of alkaline metal such as lithium, sodium, potassium or, ammonia, ethanolamines, propanolamines, ammonium salt such as triethylamine in order to heighten the water solubility of the obtained polystyrene derivative.

It is preferable that the constituting unit (b) represented by the above formula: $-[Z]-$ to "a" is 0.2 to 0.9, particularly 0.3 to 0.8. When b/a is less than 0.2, enough water solubility is not imparted, and when b/a is more than 0.9, ultraviolet absorptive property deteriorates.

It is preferable that the degree of polymerization (a) of the polystyrene derivative of the present invention is not less than 10 in order to prevent the obtained polystyrene derivative from being absorbed through the skin, and not more than 1500 in order to prevent the utility from lowering as the result of being extremely highly viscous. Particularly, it is preferable that the degree of polymerization of the polystyrene derivative is 20 to 1100.

The polystyrene derivative of the present invention is a copolymer prepared by copolymerizing one or not less than two of the styrene derivatives represented by the general formula (V):

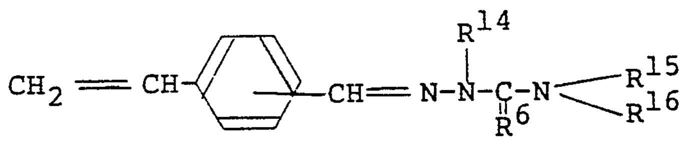

(V)

wherein $R^{14}$, $R^{15}$, $R^{16}$ and $R^6$ are the same as mentioned above, respectively, and a compound which has unsaturated aliphatic groups having at least one double bond between carbon atoms and of which at least one unsaturated aliphatic group is terminated by carboxyl group, an anhydride, an ester, an amide or imide (hereinafter referred to as "monomer B"), in a solution by using an azo-compound or a peroxide as a polymerization initiator.

Examples of the azo-compound used as a polymerization initiator are, for instance, α, α'-

azobisisobutyronitrile, 2,2'-azobis (2,4-dimethylvaleronitrile), and the like. Examples of the peroxide are, for instance, ammonium persulfate, and the like.

The monomer B is added in order to impart water solubility to the obtained polystyrene derivative. Examples of the monomer B are, for instance, maleic acid, maleic anhydride, fumaric acid or acrylic acid, or a lower alkyl ester having 1 to 5 carbon atoms or an amide of maleic acid, fumaric acid or acrylic acid, and the like.

The polymerization reaction is carried out in a solution. The solvent is a solvent which is generally used when radical polymerization is conducted. However, it is preferable that the solvent is the one which dissolves a starting monomer and a resulting polymer. Examples of the solvent are, for instance, dimethylformamide, dioxane, tetrahydrofuran, N-methylpyrrolidone, and the like.

The temperature at the polymerization is room temperature to 100°C as well as general radical polymerization. It is also preferable that the atmosphere at the polymerization is replaced by nitrogen gas.

The polystyrene derivative is reacted with a base such as hydroxide of an alkaline metal, ammonia or amine in water to form a salt of carboxylic acid, and esters or amides thereof are hydrolyzed with a strong base such as hydroxide of alkaline metal. As the results, water solubility is imparted to the obtained polystyrene derivative.

One embodiment of a method for preparing the polystyrene derivative of the present invention is explained hereinafter.

First, the reaction formula is as follows.

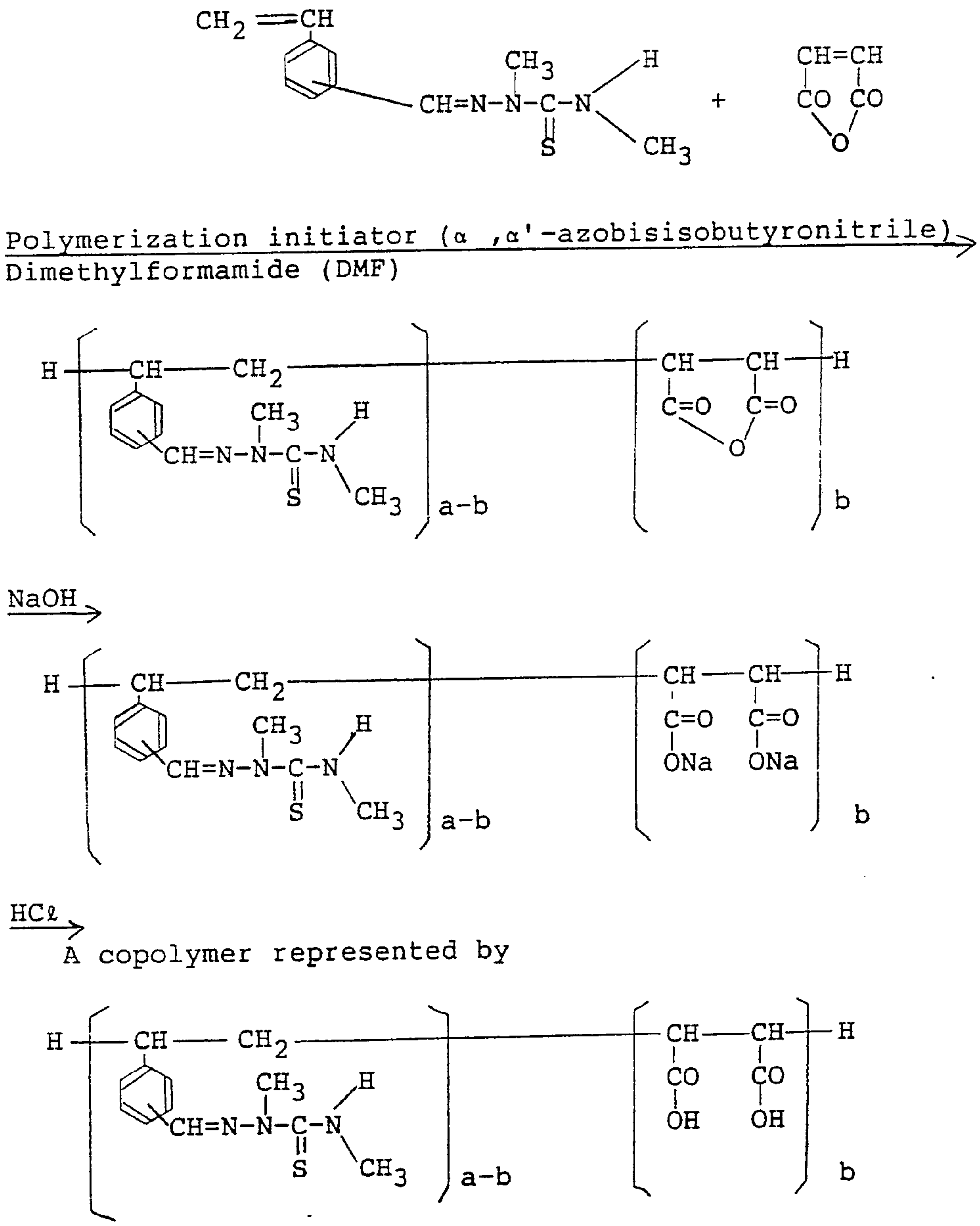


wherein a and b are the same as mentioned above.

The thus obtained polystyrene derivative of the present invention is either a random copolymer or a block copolymer comprising a portion represented by the formula:

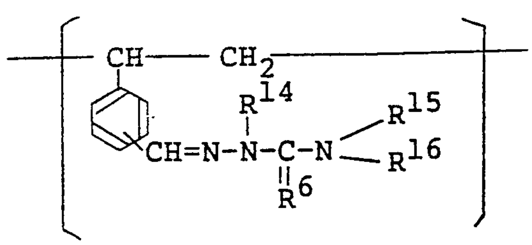

and a portion represented by the formula $-[Z]-$, and can be suitably used as a sunscreening agent for preventing sunburn since the ultraviolet absorption wavelength of the polystyrene derivative is within a range of 290 to 350 nm, which nearly corresponds to the most effective wavelength causing erythema (295 to 330 nm).

Any of the above-mentioned polystyrene derivatives of the present invention are used as a sunscreening agent, and the polystyrene derivatives are used in the form of liquids, ointment and the like, in accordance with their purposes.

When the polystyrene derivative of the present invention is used as a liquid sunscreening agent, after the polystyrene derivative is changed into a salt and dissolved in water, the styrene derivative can be used as liquids such as toilet water, tonic, lotion and milky lotion by using a base material such as glycerol, propylene glycol, vegetable oil. When the polystyrene derivative is used as a creamy sunscreening agent, after the polystyrene derivative is changed into a salt and dissolved in water in the same manner as mentioned above, the polystyrene derivative is used by adding into a base material such as an ointment prepared by emulsifying, for example, white vaseline, stearyl alcohol, propylene glycol, sodium lauryl sulfate, or the like, which is generally used.

Further, the content of the polystyrene derivative contained in a sunscreening agent cannot be absolutely determined since the content is different depending on the kinds of the polystyrene derivative, the ratio of the monomers at the copolymerization, kinds of the formulation, and the like. For example, when a liquid sunscreening agent is prepared , it is preferable that the content of the polystyrene derivative is adjusted to be 0.1 to 50 % by weight, particularly 2 to 25 % by weight. When the content is less than 0.1 % by weight, the effect exhibited by adding the polystyrene derivative is small, and when the content is more than 50

% by weight, dispersion property comes to be lowered. Also, for instance, when a solid or powdery sunscreening agent is prepared, it is preferable that the content of the polystyrene derivative is adjusted to be 0.1 to 50 % by weight, particularly 2 to 35 % by weight. When the content is less than 0.1 % by weight, the effect exhibited by adding the polystyrene derivative is small, and when the content is more than 50 % by weight, the obtained sunscreening agent can be used, but improvements of the effects cannot be expected and it becomes economically disadvantageous.

To the sunscreening agent, perfume, colorant, vehicle and other sunscreening agents, and the like can be suitably added.

Next, the styrene derivative and the polystyrene derivative of the present invention are more specifically explained by means of the following Examples. The present invention cannot be limited to the Examples.

Example 1

[Preparation of p-vinylbenzaldehyde 4-methoxycarbonylmethyl-2-methylthiosemicarbazone]

In 100 mℓ of methanol was dissolved 20.0 g of methyl isothiocyanateacetate, and 50 mℓ of a methanol solution containing 7.0 g of methylhydrazine was added thereto while cooling with ice. Then, the reaction was carried out at room temperature for 2 hours. After the reaction, the precipitated crystals were filtered and collected, and were recrystallized by using methanol to give 21.7 g of 4-methoxycarbonylmethyl-2-methylthiosemicarbazide in the form of colorless needle-like crystals (yield 80 %). Then, 21.7 g of the obtained 4-methoxycarbonylmethyl-2-methylthiosemicarbazide was dissolved in 250 mℓ of methanol, 100 mℓ of a methanol solution of 16.2 g of p-vinylbenzaldehyde was added thereto, and the reaction was carried out at room temperature for 4.5 hours. After the reaction, the

precipitated crystals was collected and recrystallized by using methanol to give 28.6 g of colorless needle-like crystals (yield 80 %). It was identified that the obtained product was p-vinylbenzaldehyde 4-methoxycarbonylmethyl-2-methylthiosemicarbazone according to the following methods.

(Infrared absorption spectrum)

The measurement was carried out by using a diffraction grating infrared spectrophotometer (IRA-1 type, made by JAPAN SPECTROSCOPIC Co., Ltd.) according to KBr method. The results are shown below.

3370 $cm^{-1}$ (N-H stretching vibration)
1745 $cm^{-1}$ (C=O stretching vibration)
1510 $cm^{-1}$ (C=N stretching vibration)
1340 $cm^{-1}$ (C-H scissoring deformation vibration)
1065 $cm^{-1}$ (C=S, C-N stretching vibration)
980 $cm^{-1}$ and 900 $cm^{-1}$ ($CH=CH_2$ out-of-plane deformation vibration)

(Nuclear magnetic resonance spectrum)

The measurement was carried out by using a high resolution nuclear magnetic resonance apparatus (R-40 type, made by Hitachi, Ltd.) with tetramethylsilane as a standard substance, as $CDC\ell_3$ (deuterated chloroform) solution. The results are shown below.

$\delta$ 3.81 (3H, S, $-OC\underline{H}_3$)
3.85 (3H, S, $-NC\underline{H}_3$)
4.99 (2H, d, J=5.00 Hz, $-NHC\underline{H}_2-$)
5.32 (1H, d, J=10.00 Hz, $-CH=C\underline{H}_2-$)
5.80 (1H, d, J=17.50 Hz, $-CH=C\underline{H}_2$)
6.75 (1H, d-d, J=10.00, 17.50 Hz, $-C\underline{H}=CH_2$)
7.45 and 7.67 (2H, d, J=7.00 Hz, aromatic ring, respectively)
7.67 (1H, S, $-C\underline{H}=N-$)
8.60 to 8.83 (1H, broad, $-N\underline{H}CH_2-$)

(Mass spectrometry)

The measurement was carried out by using a mass spectrometer (RMU-6MG type, made by Hitachi, Ltd.)

according to direct insertion method (El-20eV). The results are shown below.

m/z: 291 ($M^+$, 78), 130 (100)

(Elementary analysis)

Carbon, hydrogen and nitrogen were analyzed by using an elementary analyzer (240 C, made by Perkin-Elmer Corp.) at the same time, and sulfur was analyzed according to oxygen-flask method. The results are shown below.

Calcd.: C 59.0 %, H 6.3 %, N 13.8 %, S 10.5 %

Found: C 58.9 %, H 6.4 %, N 13.7 %, S 10.0 %

Next, the melting point and ultraviolet absorption spectrum of the obtained product were measured according to the following methods.

(Melting point)

The measurement was carried out by using a melting point measuring apparatus (MP type, made by Yanagimoto Co., Ltd). The results are shown below.

Melting point: 129.5° to 130.0 °C

(Ultraviolet absorption spectrum)

The measurement was carried out by using a spectrophotometer (UV-200S, made by SHIMADZU CORPORATION) as an ethanol solution of the sample having a suitable concentration with a quartz cell having an optical path of 10 mm. The result of the measurement is shown in Fig. 1. Also, the wavelength at the maximum absorption and the molar extinction coefficient were found from the results of the measurement as the ultraviolet absorptive properties. The results are shown below.

Wavelength at the maximum absorption: 332 nm

Molar extinction coefficient: 44,000

Example 2

[Preparation of p-vinylbenzaldehyde 4-carboxymethyl-2-methylthiosemicarbazone]

To 200 mℓ of a 1,4-dioxane solution containing 20.0 g of p-vinylbenzaldehyde 4-methoxycarbonylmethyl-2-methylthiosemicarbazone was added 95 mℓ of a 0.75N-NaOH

aqueous solution while cooling with ice, and the reaction was carried out at room temperature for 1.5 hours. After the reaction, 100 mℓ of water was added. The water layer was washed with ethyl acetate and adjusted to pH 2 with dilute hydrochloric acid, then extracted with ehtyl acetate. The ethyl acetate layer was washed with water and then with a saturated salt solution, and was dried over anhydrous sodium sulfate.

The ethyl acetate solution from which sodium sulfate was filtered off, was concentrated, and the residue was recrystallized by using methanol to give 14.6 g of colorless needle-like crystals (yield 76 %). The identification of the obtained product was conducted in the same manner as in Example 1, and it was confirmed that the obtained product was p-vinylbenzaldehyde4-carboxymethyl-2-methylthiosemicarbazone. The data of the identification are mentioned below.

(Infrared absorption spectrum)

3350 $cm^{-1}$ (N-H stretching vibration)
3000 to 2800 $cm^{-1}$ (O-H stretching vibration)
1710 $cm^{-1}$ (C=O stretching vibration)
1510 $cm^{-1}$ (C=N stretching vibration)
1060 $cm^{-1}$ (C=S, C-N stretching vibration)
980 $cm^{-1}$ and 900 $cm^{-1}$ ($CH=CH_2$ out-of-plane deformation vibration)

(Nuclear magnetic resonance spectrum)

The measurement was carried out as deuterated acetone solution.

$\delta$ 3.90 (3H, S, $-NHC\underline{H}_3$)
4.50 (2H, d, J=5.00 Hz, $-NC\underline{H}_2-$)
5.31 (1H, d, J=10.00 Hz, $-CH=C\underline{H}_2$)
5.90 (1H, d; J=17.50 Hz, $-CH=C\underline{H}_2$)
6.82 (1H, d-d, J=10.00, 17,50 Hz, $-C\underline{H}=CH_2$)
7.54 and 7.85 (2H, d, J=7.00 Hz, aromatic ring)
8.00 (1H, S, $-C\underline{H}=N-$)
8.90 to 9.10 (1H, broad, $-N\underline{H}CH_2-$)

(Mass spectrometry)

m/z: 277 ($M^+$, 13), 160 (100)

(Elementary analysis)

Calcd.: C 56.2 %, H 5.5 %, N 15.2 %, S 11.6 %

Found: C 56.1 %, H 5.4 %, N 15.1 %, S 11.0 %

Next, the melting point and ultraviolet absorption spectrum of the obtained product were measured.

(Melting point)

178.0° to 180.0°C

(Ultraviolet absorption spectrum)

The result of the measurement is shown in Fig. 2. Also, the ultraviolet absorptive properties found from the result are as follows:

Wavelength at the maximum absorption: 332 nm

Molar extinction coefficient: 44,000

## Example 3

[Preparation of p-vinylbenzaldehyde 4-ethoxy carbonylmethyl-2-methylsemicarbazone]

In 100 mℓ of ethanol was dissolved 20.0 g of p-vinylbenzaldehyde, and 10 mℓ of an ethanol solution containing 7.3 g of methylhydrazine was added dropwise thereto. Then, the reaction was carried out at room temperature for 3.5 hours. After the reaction, 30 mℓ of an ethanol solution of 21.5 g of ethyl isocyanateacetate was added thereto, and the reaction was carried out at room temperature for 15 hours. After the reaction was completed, the precipitated crystals were filtered and collected, and were recrystallized by using ethanol to give colorless plate-like crystals (total yield 65 %). The identification of the obtained product was conducted in the same manner as in Example 1, and it was confirmed that the obtained product was p-vinylbenzaldehyde 4-ethoxycarbonylmethyl-2-methylsemicarbazone. The data of the identification are shown below.

(Infrared absorption spectrum)

3380 $cm^{-1}$ (N-H stretching vibration)

1780 $cm^{-1}$ (C=O stretching vibration)

1685 $cm^{-1}$ (absorption band of amide I)
1510 $cm^{-1}$ (C=N stretching vibration)
1195 $cm^{-1}$ (C-O stretching vibration)
980 $cm^{-1}$ and 910 $cm^{-1}$ (C=C out-of-plane deformation vibration)

(Nuclear magnetic resonance spectrum)

The measurement was carried out as a deuterated chloroform solution.

δ 1.30 (3H, t, J=7.25 Hz, $-OCH_2C\underline{H}_3$)
3.36 (3H, S, $-NC\underline{H}_3$)
4.13 (2H, d, J=5.00 Hz, $-NHC\underline{H}_2-$)
4.25 (2H, q, J=7.25 Hz, $-OC\underline{H}_2CH_3$)
5.30 (1H, d, J=10.00 Hz, $-CH=C\underline{H}_2$)
5.80 (1H, d, J=17.50 Hz, $-CH=C\underline{H}_2$)
6.75 (1H, d-d, J=10.00, 17.50 Hz, $-C\underline{H}=CH_2$)
7.05 to 7.15 (1H, broad, $-N\underline{H}CH_2-$)
7.43 and 7.63 (2H, d, J=7.00 Hz, aromatic ring, respectively)
7.51 (1H, S, $-C\underline{H}=N-$)

(Mass spectrometry)

m/z: 289 ($M^+$, 40), 160 (100)

(Elementary analysis)

Calcd.: C 62.3 %, H 6.6 %, N 14.5 %
Found : C 62.1 %, H 6.5 %, N 14.4 %

Next, the melting point and ultraviolet absorption spectrum of the obtained product were measured. The results are shown below.

(Melting point)

126.5° to 127.0°C

(Ultraviolet absorption spectrum)

The result of the measurement is shown in Fig. 3. The ultraviolet absorptive properties found from the result are as follows:

Wavelength at the maximum absorption: 311 nm
Molar extinction coefficient: 33,000

Example 4

[Preparation of vinylbenzaldehyde 2-carboxymethyl-4-methylthiosemicarbazone]

The reaction of 19.5 g of vinylbenzaldehyde with 12.0 g of acetohydrazide was carried out in 30 mℓ of ethanol for 4 hours. Then, 20.0 g (yield 72 %) of the obtained vinylbenzaldehyde acetohydrazone was dissolved in 50 mℓ of dimethylformamide (hereinafter referred to as DMF), and the solution was added, while cooling with ice, to 30 mℓ of a DMF solution suspended 4.3 g of sodium hydride in oil (trade name, a suspension of sodium hydride in mineral oil made by WAKO PURE CHEMICAL INDUSTRIES, LTD., content of sodium hydride: 60 %) was dissolved in an amount of 4.3 g based on 32 g of DMF. Then, 30 mℓ of a DMF solution of 17.6 g of ethyl chloroacetate was added dropwise thereto, and the reaction was carried out at room temperature for 1.5 hours. After the reaction, the residue obtained by distilling away DMF, was dissolved in a mixed solution of ethanol (200 mℓ) and water (50 mℓ), 450 mℓ of a 2N-NaOH aqueous solution was added thereto, and then hydrolysis was conducted by refluxing for 5 hours. After the reaction, the pH value was adjusted to 8 with acetic acid, 15 mℓ of a ethanol solution of 7.8 g of methyl isothiocyanate was added thereto, and then the reaction was carried out at room temperature for 15 hours. After the reaction, ethanol was distilled away and the resultant was treated in the same manner as in Example 2 to give 19.4 g of colorless needle-like crystals (yield 68 %). The identification of the obtained product was conducted in the same manner as in Example 1, and it was confirmed that the obtained product was vinylbenzaldehyde 2-carboxymethyl-4-methylthiosemicarbazone. The data of the identification are shown below.

(Infrared absorption spectrum)

3260 $cm^{-1}$ (N-H stretching vibration)
3000 to 2800 $cm^{-1}$ (O-H stretching vibration)
1690 $cm^{-1}$ (C=O stretching vibration)
1520 $cm^{-1}$ (C=N stretching vibration)
1050 $cm^{-1}$ (C=S, C-N stretching vibration)

980 $cm^{-1}$ and 900 $cm^{-1}$ ($CH=CH_2$ out-of-plane deformation vibration)

(Nuclear magnetic resonance spectrum)

The measurement was carried out as a deuterated dimethyl sulfoxide solution.

δ 3.11 (3H, d, J=5.00 Hz, $-NHC\underline{H}_3-$)

5.41 (2H, S, $-NC\underline{H}_2-$)

5.31 (1H, d, J=10.00 Hz, $-CH=C\underline{H}_2$)

5.93 (1H, d, J=17.50 Hz, $-CH=C\underline{H}_2$)

6.80 (1H, d-d, J=10.00, 17.50 Hz, $-C\underline{H}=CH_2$)

7.30 to 7.63 and 7.80 to 7.95 (2H, m, aromatic ring, respectively)

8.00 (1H, S, $-C\underline{H}=N-$)

8.97 (1H, q, J=5.00 Hz, $-N\underline{H}CH_3$)

(Mass spectrometry)

m/z: 277 ($M^+$, 17), 130 (100)

(Elementary analysis)

Calcd.: C 56.2 %, H 5.5 %, N 15.2 %, S 11.6 %

Found : C 56.1 %, H 5.3 %, N 15.0 %, S 11.0 %

Next, the melting point and ultraviolet absorption spectrum of the obtained product were measured. The results are shown below.

(Melting point)

160.0° to 161.0°C

(Ultraviolet absorption spectrum)

The result of the measurement is shown in Fig. 4. The ultraviolet absorptive properties found from the result are as follows:

Wavelength at the maximum absorption: 315 nm

Molar extinction coefficient: 34,000

Example 5

[Preparation of p-vinylbenzaldehyde 2-(γ-carboxypropyl)-4-methylthiosemicarbazone]

The procedure of Example 4 was repeated except that ethyl bromobutyrate was used instead of ethyl chloroacetate to give colorless needle-like crystals (yield: 51 %). The identification of the obtained

product was conducted in the same manner as in Example 1, and it was confirmed that the obtained product was p-vinylbenzaldehyde 2-(γ-carboxypropyl)-4-methylthiosemicarbazone. The data of the identification are shown below.

(Infrared absorption spectrum)

3300 $cm^{-1}$ (N-H stretching vibration)
3020 to 2880 $cm^{-1}$ (O-H stretching vibration)
1690 $cm^{-1}$ (C=O stretching vibration)
1510 $cm^{-1}$ (C=N stretching vibration)
1050 $cm^{-1}$ (C=S, C-N stretching vibration)
980 $cm^{-1}$ and 910 $cm^{-1}$ ($CH=CH_2$ out-of-plane deformation vibration)

(Nuclear magnetic resonance spectrum)

The measurement was carried out as deuterated chloroform solution.

δ 1.70 to 2.13 (2H, m, $-NCH_2C\underline{H}_2CH_2-$)
2.55 (2H, t, J=7.25 Hz, $-NCH_2CH_2C\underline{H}_2-$)
3.25 (3H, d, J=5.00 Hz, $-NHC\underline{H}_3$)
4.50 to 4.73 (2H, m, $-NCH_2C\underline{H}_2CH_2-$)
5.33 (1H, d, J=10.00 Hz, $-CH=C\underline{H}_2$)
5.83 (1H, d, J=17.50 Hz, $-CH=C\underline{H}_2$)
6.76 (1H, d-d, J=10.00, 17.50 Hz, $-C\underline{H}=CH_2$)
7.45 and 7.68 (2H, d, J=7.00 Hz, aromatic ring, respectively)
8.23 (1H, q, J=5.00 Hz, $-N\underline{H}CH_3$)
9.73 (1H, S, $-CO_2\underline{H}$)

(Mass spectrometry)

m/z: 305 ($M^+$, 15), 130 (100)

(Elementary analysis)

Calcd.: C 58.9 %, H 6.3 %, N 13.8 %, S 10.5 %
Found : C 59.0 %, H 6.3 %, N 13.6 %, S 10.3 %

Next, the melting point and ultraviolet absorption spectrum of the obtained product were measured.

(Melting point)

147.0° to 148.0°C

(Ultraviolet absorption spectrum)

The result of the measurement is shown in Fig. 5. The ultraviolet absorptive properties found from the result of the measurement are as follows:

Wavelength at the maximum absorption: 332 nm

Molar extinction coefficient: 38,000

Example 6

[Preparation of p-vinylbenzaldehyde 2-ethoxycarbonylmethyl-4,4-dimethylthiosemicarbazone]

In the same manner as in Example 1, 3.8 g of ethyl hydrazinoacetate hydrochloride and 3.3 g of dimethylcarbamoyl chloride were dissolved in 25 mℓ of ether, and 3.7 g of triethylamine was added thereto, and then the reaction was carried out at room temperature for 2 hours. Then, 4.1 g of the obtained 2-ethoxycarbonylmethyl-4,4-dimethylthiosemicarbazide was reacted with 6.3 g of p-vinylbenzaldehyde to give 5.1 g of colorless needle-like crystals (yield 79 %). The identification of the obtained product was conducted in the same manner as in Example 1, and it was confirmed that the obtained product was p-vinylbenzaldehyde 2-ethoxycarbonyl-4,4-dimethylthiosemicarbazone. The data of the identification are shown below.

(Infrared absorption spectrum)

1720 $cm^{-1}$ (C=O stretching vibration)
1500 $cm^{-1}$ (C=N stretching vibration)
1070 $cm^{-1}$ (C=S, C-N, stretching vibration)
980 $cm^{-1}$ and 900 $cm^{-1}$ ($CH=CH_2$ out-of-plane deformation vibration)

(Nuclear magnetic resonance spectrum)

The measurement was carried out as a deuterated chloroform solution.

δ 1.28 (3H, t, J=7.25 Hz, $-OCH_2C\underline{H}_3$)
3.39 (6H, S, $-N(C\underline{H}_3)_2$)
4.25 (2H, q, J=7.25 Hz, $-OC\underline{H}_2CH_3$)
5.28 (2H, S, $-NC\underline{H}_2-$)
5.30 (1H, d, J=10.00 Hz, $-CH=C\underline{H}_2$)
5.80 (1H, d, J=17.50 Hz, $-CH=C\underline{H}_2$)

6.75 (1H, d-d, J=10.00, 17.50 Hz, $-C\underline{H}=CH_2$)

7.43 and 7.63 (2H, d, J=7.00 Hz, aromatic ring, respectively)

(Mass spectrometry)

m/z: 319 ($M^+$, 18), 88 (100)

(Elementary analysis)

Calcd.: C 60.1 %, H 6.6 %, N 13.1 %, S 10.0 %

Found : C 60.2 %, H 6.6 %, N 13.0 %, S 9.5 %

Next, the melting point and ultraviolet absorption spectrum of the obtained product were measured. The results are shown below.

(Melting point)

81.0 ° to 82.0 °C

(Ultraviolet absorption spectrum)

The result of the measurement is shown in Fig. 6. The ultraviolet absorption properties found from the result of the measurement are as follows:

Wavelength at the maxinum absorption: 381 nm

Molar extinction coefficient: 34,000

Example 7

[Preparation of vinylbenzaldehyde 2,4-dimethylthiosemicarbazone]

In 30 mℓ of ethanol was dissolved 9.9 g of 2,4-dimethylthiosemicarbazide, and 40 mℓ of an ethanol solution containing 11.0 g of vinylbenzaldehyde was added thereto. The reaction was carried out at room temperature for 6 hours. After the reaction, the precipitated crystals were filtered and collected, and were recrystallized from an ethanol solution to give 17.1 g of colorless needle-like crystals (yield 88 %). The identification of the obtained product was conducted in the same manner as in Example 1, and it was confirmed that the obtained product was vinylbenzaldehyde-2,4-dimethylthiosemicarbazone.

(Infrared absorption spectrum)

3310 $cm^{-1}$ (N-H stretching vibration)

1510 $cm^{-1}$ (C=N stretching vibration)

1050 $cm^{-1}$ (C=S, C-N stretching vibration)
980 $cm^{-1}$ and 900 $cm^{-1}$ ($CH=CH_2$ out-of-plane deformation vibration)

(Nuclear magnetic resonance spectrum)

The measurement was carried out as a deuterated chloroform solution.

δ 3.23 (3H, d, J=5.00 Hz, $-NHC\underline{H}_3$)
3.87 (3H, S, $-NC\underline{H}_3$)
5.31 (1H, d, J=10.00 Hz, $-CH=C\underline{H}_2$)
5.79 (1H, d, J=17.5 Hz, $-CH=C\underline{H}_2$)
6.76 (1H, d-d, J=10.00, 17.50 Hz, $-C\underline{H}=CH_2$)
7.31 to 7.65 (4H, m, aromatic ring)
7.63 (1H, S, $-C\underline{H}=N-$)
8.07 to 8.31 (1H, broad, $-N\underline{H}CH_3$)

(Mass spectrometry)

m/z:233 ($M^+$, 93), 71 (100)

(Elementary analysis)

Calcd.: C 61.8 %, H 6.5 %, N 18.0 %, S 13.7 %
Found : C 61.8 %, H 6.5 %, N 17.9 %, S 13.3 %

Next, the melting point and ultraviolet absorption spectrum of the obtained product were measured. The results are shown below.

(Melting point)

136.5° to 137.5°C

(Ultraviolet absorption spectrum)

The result of the measurement is shown in Fig. 7. The ultraviolet absorptive properties found from the result are as follows:

Wavelength at the maximum absorption: 315 nm
Molar extinction coefficient: 34,000

## Example 8

[Preparation of p-vinylbenzaldehyde 2,4,4-trimethylthiosemicarbazone]

In the same manner as in Example 7, 7.0 g of 2,4,4-trimethylthiosemicarbazide was reacted with 7.1 g of p-vinylbenzaldehyde to give 11.0 g of colorless plate-like crystals (yield 84 %). The identification of the

obtained product was conducted in the same manner as in Example 1, and it was confirmed that the obtained product was p-vinylbenzaldehyde 2,4,4-trimethylthiosemicarbazone. The data of the identification are shown below.

(Infrared absorption spectrum)

1510 $cm^{-1}$ (C=N stretching vibration)

1070 $cm^{-1}$ (C=S, C-N stretching vibration)

980 $cm^{-1}$ and 900 $cm^{-1}$ ($CH=CH_2$ out-of-plane deformation vibration)

(Nuclear magnetic resonance spectrum)

The measurement was carried out as a deuterated chloroform solution.

δ 3.33 (6H, S, $-N(C\underline{H}_3)_2$)
3.65 (3H, S, $-NC\underline{H}_3$)
5.28 (1H, d, J=10.00 Hz, $-CH=C\underline{H}_2$)
5.80 (1H, d, J=17.50 Hz, $-CH=C\underline{H}_2$)
6.75 (1H, d-d, J=10.00, 17.50 Hz, $-CH=C\underline{H}_2$)
7.41 to 7.63 (2H, d, J=7.00 Hz, aromatic ring, respectively)
7.59 (1H, S, $-C\underline{H}=N-$)

(Mass spectrometry)

m/z: 247 ($M^+$, 30), 188 (100)

(Elementary analysis)

Calcd.: C 63.1 %, H 6.9 %, N 16.9 %, S 12.8 %
Found : C 63.0 %, H 6.8 %, N 17.0 %, S 12.7 %

Next, the melting point and ultraviolet absorption spectrum of the obtained product were measured. The results are shown below.

(Melting point)

143.0° to 144.0°C

(Ultraviolet absorption spectrum)

The result of the measurement is shown in Fig. 8. The ultraviolet absorptive properties found from the result are as follows:

Wavelength at the maximum absorption: 331 nm
Molar extinction coefficient: 29,000

Exampel 9

[Preparation of p-vinylbenzaldehyde 2-(β-hydroxyethyl)-4-methylthiosemicarbazone]

In the same manner as in Example 1, 3.8 g of hydrazinoethanol was reacted with 3.7 g of methyl isothiocyanate in ethanol at room temperature for 3 hours. There was reacted 6.1 g of the obtained 2-(β-hydroxyethyl)-4-methylthiosemicarbazide with 5.3 g of p-vinylbenzaldehyde in ethanol for 6 hours to give 8.9 g of colorless crystals (yield 85 %). The identification of the obtained product was conducted in the same manner as in Example 1, and it was confirmed that the obtained product was p-vinylbenzaldehyde 2-(β-hydroxyethyl)-4-methylthiosemicarbazone.

(Infrared absorption spectrum)

3080 to 3040 $cm^{-1}$ (O-H stretching vibration)
1520 $cm^{-1}$ (C=N stretching vibration)
1040 $cm^{-1}$ (C=S, C-N stretching vibration)
990 $cm^{-1}$ and 905 $cm^{-1}$ ($CH=CH_2$ out-of-plane deformation vibration)

(Nuclear magnetic resonance spectrum)

The measurement was carried out as a deuterated acetone solution.

δ 2.83 (1H, S, -OH)
3.17 (3H, d, J=5.00 Hz, $-NHC\underline{H}_3$)
3.80 to 4.07 (2H, m, $-CH_2C\underline{H}_2OH$)
4.73 (2H, t, J=7.25 Hz, $-C\underline{H}_2CH_2OH$)
5.30 (1H, d, J=10.00 Hz, $-CH=C\underline{H}_2$)
5.85 (1H, d, J=17.50 Hz, $-CH=C\underline{H}_2$)
6.80 (1H, d-d, J=10.00, 17.50 Hz, $-C\underline{H}=CH_2$)
7.50 and 7.83 (2H, d, J=7.00 Hz, aromatic ring, respectively)
8.20 (1H, S, $-C\underline{H}=N-$)
8.55 and 8.87 (1H, broad $-N\underline{H}CH_3$)

(Mass spectrometry)

m/z: 263 ($M^+$, 47), 126 (100)

(Elementary analysis)

Calcd.: C 59.3 %, H 6.5 %, N 15.9 %, S 12.2 %

Found : C 59.5 %, H 6.3 %, N 15.7 %, S 12.0 %

Next, the melting point and ultraviolet absorption spectrum of the obtained product were measured. The results are shown below.

(Melting point)

169.0° to 170.0°C

(Ultraviolet absorption spectrum)

The result of the measurement is shown in Fig. 9. The ultraviolet absorptive properties found from the result are as follows:

Wavelength at the maximum absorption: 322 nm

Molar extinction coefficient: 40,000

Example 10

[Preparation of p-formylcinnamic acid methyl 2,4-dimethylthiosemicarbzaone]

In the same manner as in Example 7, 2.4 g of 2,4-dimethylthiosemicarbazide was reacted with 3.8 g of p-formylcinnamic acid methyl ester in methanol to give 4.8 g of colorless needle-like crystals (yield 82 %). The identification of the obtained product was conducted in the same manner as in Example 1, and it was confirmed that the obtained product was p-formylcinnamic acid methyl 2,4-dimethylthiosemicarbazone. The data of the identification are shown below.

(Infrared absorption spectrum)

3380 $cm^{-1}$ (N-H stretching vibration)

1710 $cm^{-1}$ (C=O stretching vibration)

1520 $cm^{-1}$ (C=N stretching vibration)

1045 $cm^{-1}$ (C=S, C-N stretching vibration)

980 $cm^{-1}$ and 930 $cm^{-1}$ ($CH=CH_2$ out-of-plane deformation vibration)

(Nuclear magnetic resonance spectrum)

The measurement was carried out as a deuterated chloroform solution.

δ 3.27 (3H, d, J=5.00 Hz, $-NHC\underline{H}_3$)

3.83 (3H, S, $-OC\underline{H}_3$)

3.93 (3H, S, $-NC\underline{H}_3$)

6.47 (1H, d, J=6.25 Hz, -C<u>H</u>=CHCO-)

7.71 (1H, d, J=6.25 Hz, -CH=C<u>H</u>CO-)

7.63 and 7.73 (2H, d, J=6.25 Hz, aromatic ring, respectively)

(Mass spectrometry)

m/z: 291 ($M^+$, 42), 71 (100)

(Elementary analysis)

Calcd.: C 59.0 %, H 6.3 %, N 13.8 %, S 10.5 %

Found : C 59.0 %, H 6.3 %, N 13.7 %, S 10.2 %

Next, the melting point and ultraviolet absorption spectrum of the obtained product were measured. The results are shown below.

(Melting point)

188.0° to 189.0°C

(Ultraviolet absorption spectrum)

The result of the measurement is shown in Fig. 10. The ultraviolet absorptive properties found from the result are as follows:

Wavelength at the maximum absorption: 350 nm

Molar extinction coefficient: 44,000

## Example 11

[Preparation of p-vinylbenzaldehyde 4-methyl-2-phenylthiosemicarbazone]

In the same manner as in Example 1, 7.3 g of methyl isothiocyanate was reacted with 10.8 g of phenylhydrozine in 100 mℓ of methanol, and then 15 mℓ of a methanol solution of 13.2 g of p-vinylbenzaldehyde was added thereto. After the reaction, the precipitated crystals were collected and recrystallized from N,N-dimethylformamide-ethanol to give 25.0 g of colorless crystals (yield 84 %). The identification of the obtained product was conducted in the same manner as in Example 1, and it was confirmed that the obtained product was p-vinylbenzaldehyde 4-methyl-2-phenylthiosemicarbazone. The data of the identification are shown below.

(Infrared absorption spectrum)

The measurement was carried out by using a diffraction grating infrared spectrophotometer (IR-700 type, made by JAPAN SPECTROSCOPIC CO., LTD.) according to KBr method.

3310 $cm^{-1}$ (N-H stretching vibration)
1520 $cm^{-1}$ (C=N stretching vibration)
1050 $cm^{-1}$ (C=S, C-N stretching vibration)
980 $cm^{-1}$ and 900 $cm^{-1}$ ($CH=CH_2$ out-of-plane deformation vibration)

(Nuclear magnetic resonance spectrum)

The measurement was carried out as a deuterated trifluoroacetic acid solution.

δ 3.15 (3H, S, $-NHC\underline{H}_3$)
5.35 (1H, d, J=10.00 Hz, $-CH=C\underline{H}_2$)
5.81 (1H, d, J=17.50 Hz, $-CH=C\underline{H}_2$)
6.57 (1H, S, $-N\underline{H}CH_3$)
6.75 (1H, d-d, J=10.00, 17.50 Hz, $-C\underline{H}=CH_2$)
7.30 to 7.65 (9H, m, aromatic ring, $-C\underline{H}-N-$)

(Mass spectrometry)

m/z: 295 ($M^+$, 1), 130 (100)

(Elementary analysis)

Carbon, hydrogen and nitrogen were analyzed at the same time by using an elementary analyzer (CHN CORDER MF3, made by Yanagimoto CO., LTD.), and sulfur was analyzed according to oxygen-flask method.

Calcd.: C 69.1 %, H 5.8 %, N 14.2 %, S 10.9 %
Found : C 68.7 %, H 6.1 %, N 14.1 %, S 10.8 %

Next, the melting point and ultraviolet absorption spectrum of the obtained product were measured. The results are shown below.

(Melting point)

248.0° to 250.0°C

(Ultraviolet absorption spectrum)

The measurement was carried out as a N,N-dimethylformamide solution. The results of the measurement is shown in Fig. 11. The ultraviolet absorptive properties found from the result are as follows:

Wavelength at the maximum absorption: 335 nm
Molar extinction coefficient: 38,000

Example 12

[Preparation of p-vinylbenzaldehyde 2-methyl-4-phenylthiosemicarbazone]

In the same manner as in Example 1, 4.3 g of methylhydrazine was reacted with 13.5 g of phenyl isothiocyanate in 100 mℓ of methanol, and then 15 mℓ of a methanol solution of 13.2 g of p-vinylbenzaldehyde was added thereto. After the reaction, the mixture was heated to be homogeneous solution, and recrystallized to give 28.6 g of colorless crystals (yield 97 %). The identification of the obtained product was conducted in the same manner as in Example 17, and it was confirmed that the obtained product was p-vinylbenzaldehyde 2-methyl-4-phenylthiosemicarbazone.

(Infrared absorption spectrum)

3270 $cm^{-1}$ (N-H stretching vibration)
1530 $cm^{-1}$ (C=N stretching vibration)
1050 $cm^{-1}$ (C=S, C-N stretching vibration)
980 $cm^{-1}$ and 900 $cm^{-1}$ ($CH=CH_2$ out-of-plane deformation vibration)

(Nuclear magnetic resonance spectrum)

δ 3.90 (3H, S, $-NC\underline{H}_3$)
5.30 (1H, d, J=10.00 Hz, $-CH=C\underline{H}_2$)
5.80 (1H, d, J=17.50 Hz, $-CH=C\underline{H}_2$)
6.73 (1H, d-d, J=10.00, 17.50 Hz, $-C\underline{H}=CH_2$)
7.20 to 7.75 (9H, m, aromatic ring)
7.70 (1H, S, $-C\underline{H}=N-$)

(Mass spectrometry)

m/z: 295 ($M^+$, 5), 130 (100)

(Elementary analysis)

Calcd.: C 69.1 %, H 5.8 %, N 14.2 %, S 10.9 %
Found : C 69.0 %, H 5.8 %, N 14.3 %, S 10.8 %

Next, the melting point and ultraviolet absorption spectrum of the obtained product were also measured in the same manner as in Example 1.

(Melting point)

135.5° to 136.5°C

(Ultraviolet absorption spectrum)

The measurement was carried out as a N,N-dimethylformamide solution. The result of the measurement is shown in Fig. 12.

Wavelength at the maximum absorption: 338 nm

Molar extinction coefficient: 40,000

## Comparative Example 1

Wavelength at the maximum absorption and molar absorptivity of ultraviolet rays were measured by using homomenthyl salicylate as a commercially available sunscreening agent. As the result, the wavelength at the maximum absorption was 308 nm, and the molar absorption wavelength was 4,400.

## Comparative Example 2

Wavelength at the maximum absorption and molar extinction coefficient of ultraviolet rays were measured by using 2-(2'-hydroxy-5'-methylphenylbenzotriazole) as a commercially available sunscreening agent. As the result, the wavelengths at the maximum absorption were present at 297 nm and 337 nm, and the molar extinction coefficients were 12,000 and 13,000, respectively.

## Comparative Example 3

Wavelength at the maxima absorption and molar extinction coefficient of ultraviolet rays were measured by using 2-hydroxy-4-methoxybenzophenone as a commercially available sunscreening agent. As the result, the wavelengths at the maxima absorption were 287 nm and 326 nm, and the molar extinction coefficients were 13,000 and 8,200, respectively.

## Example 13

A mixture of 0.5 g of p-vinylbenzaldehyde 4-methoxycarbonylmethyl-2-methylthiosemicarbazone obtained

in Example 1 and 99.5 g of a high density polyethylene (made by Aldrich Chemical Company, Inc.) was extruded at 170°C and pressed at 170°C for 15 minutes to prepare a sheet having a thickness of 0.5 mm, and the sheet was cut into pieces having a size of 2.5 cm X 18 cm. Aside from this, control pieces were prepared by using only high density polyethylene and conducting the high density polyethylene to the treatments in the same manner as above.

Two test pieces and two control pieces were prepared and irradiated with carbon arc by using Sunshine weathermeter 65/XW-WR (made by ATRAS company, Inc.) for 600 hours.

Then, the test pieces and the control pieces were examined the bending strength by bending 180° using a forefinger. As the result, while the control pieces had broken, either the test pieces had not broken and had not shown remarkable deterioration.

## Examples 14 to 17

The compounds obtained in Example 3 and Examples 8 to 10 were used in an amount of 0.5 g, respectively, and treated in the same manner as in Example 13. As the result, none of the test pieces broke and showed remarkable deterioration.

## Example 18

There was dissolved 2.8 g of p-vinylbenzaldehyde 4-carboxymethyl-2-methylthiosemicarbazone in 1.5 g of triethanolamine and 23.7 g of water. The solution was applied onto a polyethylene sheet having a thickness of 0.5 mm (which was prepared in the same manner as the preparation process for the control pieces in Example 11) in an amount of 2.0 $mg/cm^2$, and dried at room temperature to give a ultraviolet absorbent sheet.

The obtained ultraviolet absorbent sheet and untreated polyethylene sheet were cut into pieces having

a size of 1 cm X 4 cm, respectively. The untreated polyethylene sheet was used as control piece, and transmittance of ultraviolet rays at a wavelength of 330 nm was measured by using a spectrophotometer. As the result, the transmittance of ultraviolet rays of the ultraviolet absorbent sheet was not more than 0.1 %. Accordingly, it was confirmed that not less than 99.9 % of ultraviolet rays were absorbed under the above condition.

Examples 19 and 20

The compounds obtained in Example 11 and Example 12 were used in an amount of 0.5 g, respectively, and treated in the same manner as in Example 13. As the result, none of the test pieces broke and showed remarkable deterioration.

Example 21

[Preparation of poly(p-vinylbenzaldehyde 4-carboxymethyl-2-methylthiosemicarbazone)]

In 35 mℓ of dry N-methylpyrrolidone was dissolved 5.00 g of p-vinylbenzaldehyde 4-carboxymethyl-2-methylthiosemicarbazone. While passing dry nitrogen gas and stirring, the solution was heated to 60° to 62°C. Then, 5 mℓ of a solution of 0.15 g of α, α'-azobisisobutyronitrile in N-methylpyrrolidone previously prepared was added thereto, and the reaction was carried out at 60° to 62°C for 20 hours. After the reaction, the reaction solution was cooled with ice water, poured into 500 mℓ of methanol, and thus the polymer was precipitated and collected by filtration. The polymer was dissolved again in 10 mℓ of N-methylpyrrolidone, and the solution was poured into 500 mℓ of methanol to reprecipite the polymer. Then, the polymer was collected by filteration, and was dried under vacuum to give 4.94 g of light yellow powder (yield 99 %). After the powder was dissolved in 1.4 ℓ of a 0.05 % NaOH aqueous solution, circulating filtration was conducted by means of ultrafiltration to

concentrate the solution to about 300 mℓ. After the concentrated solution was adjusted to pH 2 by adding dilute hydrochloric acid, the produced precipitate was collected by filteration, and was washed with water and then with methanol, and dried under vacuum to give 4.80 g of light yellow powder (yield 97 %).

The physical properties and the elementary analysis of the obtained product were measured according to the following methods. The structural formula, the values of the elementary analysis, the values of the differential thermal analysis and the result of the measurement of ultraviolet absorptive properties are shown in Table 1. Also, the infrared absorption spectrum is shown in Fig. 13 and the ultraviolet absorption spectrum is shown in Fig. 14.

(Differential thermal analysis)

The variation in weight and temperature of the sample from room temperature to 475°C was measured with thermal analysis station (TAS-100 type, made by Rigaku Denki Co., Ltd).

(Infrared absorption spectrum)

The measurement was carried out by using a diffraction grating infrared spectrophotometer (IRA-1 type, made by JAPAN SPECTROSCOPIC CO., LTD.) according to KBr method.

(Elementary analysis)

Carbon, hydrogen and nitrogen were analyzed at the same time by using an elementary analyzer (240 C, made by Perkin-elmer Corp.), and sulfur was analyzed according to oxygen-flask method.

(Ultraviolet absorption spectrum)

The measurement was carried out by using a spectrophotometer (UV-200S, made by SHIMADZU CORPORATION), as a dimethylformamide solution of the sample having a suitable concentration and with a quartz cell having an optical path of 10 mm.

(Average molecular weight)

Suitable amount of the sample was dissolved in

a 0.01N NaOH aqueous solution, and then analysis was carried out by using a high performance liquid chromatography (made by TOSOH CO., LTD., pump: CCPE, column: TSK gel G 4000SWXL 7.8 X 300 mm + G 3000$SW_{XL}$ 7.8 X 300 mm), and using water-soluble polystyrenes (made by SHOWA DENKO K.K., weight average molecular weight: 690,000 to 1,600) as standard substances.

Next, with respect to the polystyrene derivative obtained in Example 21, acute toxicity and local stimulation were examined. The results are as follows:

[Acute toxicity]

Oral administration was conducted against mice, and $LD_{50}$ was found. Concerning $LD_{50}$, no mouse died even by the administration of 5000 mg/kg, and the mice recovered as good as normal mice. And sex difference was not observed.

[Local stimulation]

(a) Into the conjunctival sac of a rabbit, 0.1 mℓ of a 10 % aqueous solution of the polystyrene derivative was dropped, and the stimulation was measured according to the criteria for the judgement of Draize, and Kay and Calandra. As the result, it was judged that there was no stimulation. (b) A scratch was given to the notal skin of a guinea pig with a sterilized injection needle of 18 gauge, and thereon an adhesive tape used for a patch test impregnated with 1 m of a 10 % aqueous solution of the polystyrene derivative was applied. Then, the estimation of the stimulation was conducted according to the criteria of Draize. As the result, it was judged that the stimulation was mild, and in fact, there was no stimulation.

[Percutaneous absorption]

The skin of a quinea pig was peeled, washed with physiological saline and then set in a percutaneous absorption apparatus which was made in accordance with the description in Franz, T. J, J. Invest, Dermatol, 64, 190, 1975, by using a diffusion cell. The sample room

was filled with 0.5 mℓ of a 1 % aqueous solution of the polystyrene derivative, and the acceptor was filled with physiological saline. After maintaining at 33°C for 20 hours while stirring with a magnetic stirrer, the physiological saline in the acceptor was taken out, and the ultraviolet absorbency was measured. In the same manner as above, ultraviolet absorbency was measured, using water alone as a negative control substance and a 1 % aqueous solution of p-aminobenzoic acid as a positive control substance. As the result, the substance of the polystyrene derivative showed an absorption curve similar to that of the negative control substance. To the contrary, the spectrum of the positive control substance was shown the corresponding to an amount of 87.0 ppm of the compound.

From the results mentioned above, it is confirmed that the polystyrene does not permeate into skin.

From the results mentioned above, it is confirmed that the polystyrene derivative of the present invention does not involve stimulation or percutaneous absorption, and is useful not only for an ordinary sunscreen but also for protecting the exposed skin of patients of hypersensitiveness against sunlight.

## Example 22

[Preparation of poly(p-vinylbenzaldehyde 4-carboxymethyl-2-methylthiosemicarbazone)]

After 20.00 g of p-vinylbenzaldehyde 4-methoxycarbonylmethyl-2-methylthiosemicarbazone was dissolved in 100 mℓ of dry N-methylpyrrolidone, 10 mℓ of an aqueous solution of 1.00 g of ammonium persulfate previously prepared was added thereto while passing dry nitrogen gas and stirring, and the reaction was carried out at room temperature (about 20°C) for 48 hours. After the reaction, the reaction solution was cooled with ice water. Then, the solution was poured into 1.0 ℓ of methanol to precipitate the polymer, and the polymer was

collected by filteration. The polymer was dissolved again in 40 mℓ of N-methylpyrrolidone and the solution was poured into 1.0 ℓ of methanol to reprecipitate the polymer. The polymer was collected by filteration, and dried under vacuum to give 19.8 g of light yellow powder (yield 99 %). The obtained powder and 3.50 g of sodium methoxide were dissolved in 600 mℓ of dry dimethyl sulfoxide, and hydrolysis was carried out while passing dry nitrogen gas and stirring at room temperature for 15 hours. After the reaction, the reaction solution was poured into 3 ℓ of ice water, and subjected to circulating filtration by means of ultrafiltration to concentrate to about 500 mℓ. After the concentrated solution was adjusted to about pH 2 by adding dilute hydrochloric acid, the obtained precipitate was filtered and collected, washed with water and then with methanol, and dried under vacuum to give 16.83 g of light yellow powder (yield 85 %). The physical properties and elementary analysis of the obtained product were measured in the same manner as in Example 21. The results are shown in Table 1. Also, the infrared absorption spectrum measured is shown in Fig. 15 and the ultraviolet absorption specturm measured is shown in Fig. 16.

## Example 23

[Preparation of poly(vinylbenzaldehyde 4-carboxymethyl-2-methylthiosemicarbazone)]

There was dissolved 7.00 g of vinylbenzaldehyde 4-ethoxycarbonylmethyl-2-methylthiosemicarbazone in 10 mℓ of dry dioxane. After heating the solution to 60° to 62°C while passing dry nitrogen gas and stirring, 5 mℓ of a solution of 0.07 g of α,α'-azobisisobutyronitrile in dioxane previously prepared was added thereto, and the reaction was carried out at 60° to 62°C for 48 hours. After the reaction, the reaction solution was cooled with ice water. The solution was poured into 500 mℓ of methanol to precipitate the polymer, and the polymer was collected by filteration. The polymer was dissolved

again in 20 mℓ of N,N-dimethylformamide, and the solution was poured into 500 mℓ of methanol to reprecipitate the polymer. Then the product was collected by filteration, and was dried under vacuum to give 6.75 g of light yellow powder (yield 96%). After the powder was dissloved in 120 mℓ of N,N-dimethylformamide, 3.00 g of powdered NaOH was added thereto and the mixture was stirred at room temperature for 15 hours to conduct hydrolysis. After the reaction, the reaction solution was poured into 3 ℓ of ice water. After adjusting the pH to 8 to 9 by adding dilute hydrochloric acid, the solution was subjected to circulating filtration by means of ultrafiltration to concentrate to about 300 mℓ. After the concentrated solution was adjusted to about pH 2 by adding dilute hydrochoric acid, the produced precipitate was collected by filteration, and was washed with water and then with methanol, and dried under vacuum to give 5.40 g of light yellow powder (yield 80 %). The physical properties and elementary analysis of the obtained product were examined in the same manner as in Example 21.

The structural formula, the values of elementary analysis, the values of differential thermal analysis and the measurement results of the ultraviolet absorptive properties are shown in Table 1. Also, the measurement result of the infrared absorption spectrum is shown in Fig. 17, and the measurement result of the ultraviolet absorption spectrum is shown in Fig. 18.

Example 24

[Preparation of a copolymer of poly(p-vinylbenzaldehyde 4-carboxymethyl-2-methylthiosemicarbazone) and maleic acid]

There were dissolved 2.77 g of p-vinylbenzaldehyde 4-carboxymethyl-2-methylthiosemicarbazone and 0.49 g of maleic anhydride in 15 mℓ of dry dioxane. After heating the solution to 60° to 62°C while passing dry nitrogen gas and stirring,

5 mℓ of a solution of 0.16 g of α,α'-azobisisobutyronitrile in dioxane previously prepared was added dropwise thereto, and the reaction was carried out at 60° to 62°C for 20 hours. After the reaction, the reaction solution was cooled with ice water, and poured into 300 mℓ of methanol. Thus the produced polymer was precipitated, and collected by filteration. The polymer was dissolved again in 10 mℓ of N,N-dimethylformamide, and the solution was poured into 300 mℓ of methanol to reprecipitate the polymer. The polymer was collected by filteration, and dried under vacuum to give 2.46 g of light yellow powder (yield 76 %). After 2.46 g of the obtained copolymer was dissolved in 1.0 ℓ of a 0.05 % NaOH aqueous solution, the solution was treated in the same manner as in Example 21 to give 1.97 g of light yellow powder (yield 80 %). The physical properties and elementary analysis of the obtained product were measured in the same manner as in Example 21.

The structural formula, the values of elementary analysis, the values of differential thermal analysis and the measurement results of the ultraviolet absorptive properties are shown in Table 1. Also, the measurement result of the infrared absorption spectrum is shown in Fig. 19, and the measurement result of the ultraviolet absorption spectrum is shown in Fig. 20.

## Example 25

[Preparation of a copolymer of poly(vinylbenzaldehyde 4-carboxymethyl-2-methylthiosemicarbazone) and maleic acid]

There were dissolved 50.00 g of vinylbenzaldehyde 4-carboxymethyl-2-methylthiosemicarbazone and 8.03 g of maleic anhydride in 75 mℓ of dry dioxane. After heating the solution to 60° to 62°C while passing dry nitrogen gas and stirring, 10 mℓ of a solution of 0.50 g of α,α'-azobisisobutyronitrile in dioxane previously prepared was added thereto, and the reaction was carried out at 60° to 62°C for 48 hours. After the reaction, the reaction solution was cooled with ice

water, and poured into 2.0 mℓ of methanol. Thus the produced polymer was precipitated, and collected by filteration. The polymer was dissolved again in 100 mℓ of N,N-dimethylformamide, and the solution was poured into 2.0 ℓ of methanol to reprecipitate the polymer. The polymer was collected by filteration, and dried under vacuum to give 57.00 g of light yellow powder (yield 98 %). Then, 55.00 g of the obtained copolymer was dissolved in 500 mℓ of a 1N-NaOH aqueous solution. After the solution was hydrolyzed by stirring at room temperature for 15 hours, the solution was diluted 5 times with water, and then treated in the same manner as in Example 21 to give 48.00 g of light yellow powder (yield 87 %). The physical properties and elementary analysis of the obtained product were measured in the same manner as in Example 21.

The structural formula, the values of elementary analysis, the values of differential thermal analysis and the measurement results of the ultraviolet absorptive properties are shown in Table 1. Also, the measurement result of the infrared absorption spectrum is shown in Fig. 21, and the measurement result of the ultraviolet absorption spectrum is shown in Fig. 22.

Example 26

[Preparation of poly(p-vinylbenzaldehyde 2-(γ-carboxypropyl)-4-methylthiosemicarbozone)]

There was dissolved 1.00 g of p-vinylbenzaldehyde 2-(γ-carboxypropyl)-4-methyl-thiosemicarbazone 7 mℓ of dry N-methylpyrrolidone. After heating the solution to 40° to 42°C while passing dry nitrogen gas and stirring, 1 mℓ of a solution of 0.03 g of 2,2'-azobis-(2,4-dimethylvaleronitrile) in N-methylpyrrolidone previously prepared was added thereto, and the reaction was carried out at 40° to 42°C for 48 hours. After the reaction, the reaction solution was cooled with ice water, and poured into 100 mℓ of methanol. Thus the polymer was precipitated, and was

collected by filteration. The polymer was dissolved again in 2 mℓ of N,N-dimethylformamide, the solution was poured into 100 mℓ of methanol to reprecipitate the polymer. The polymer was collected by filteration, and dried under vacuum to give 0.90 g of light yellow powder (yield 90 %). Then, the obtained powder was treated in the same manner as in Example 21 to give precipitate. The precipitate was filtered and collected, and was washed with water and then with methanol to give 0.80 g of light yellow powder (yield 88 %). The physical properties and elementary analysis of the obtained product were measured in the same manner as in Example 21.

The structural formula, the values of elementary analysis, the values of differential thermal analysis and the measurement results of the ultraviolet absorptive properties are shown in Table 1. Also, the measurement result of the infrared absorption spectrum is shown in Fig. 23, and the measurement result of the ultraviolet absorption spectrum is shown in Fig. 24.

Example 27

[Preparation of a copolymer of poly(vinylbenzaldehyde 4-carboxymethyl-2-methylthiosemicarbazone) and acrylic acid]

There were dissolved 3.00 g of vinylbenzaldehyde 4-ethoxycarbonylmethyl-2-methylthiosemicarbazone and 0.84 g of methyl acrylate in 5 mℓ of dry dioxane. After heating the solution to 60° to 62°C while passing dry nitrogen gas and stirring, 2 mℓ of a solution of 0.03 g of α,α'-azobisisobutyronitrile in dioxane previously prepared was added dropwise thereto, and the reaction was carried out at 60° to 62°C for 48 hours. After the reaction, the reaction solution was cooled with ice water, and was poured into 300 mℓ of methanol. Thus the produced polymer was precipitated, and was collected by filteration. The polymer was dissolved again in 10 mℓ of N,N-dimethylformamide, the solution was poured into

300 mℓ of methanol to reprecipitate the polymer. The polymer was collected by filteration, and was dried under vacuum to give 3.80 g of light yellow powder (yield 99 %). Then, 3.50 g of the obtained copolymer was dissolved in 50 mℓ of dimethyl sulfoxide. While cooling with ice and stirring, 10 mℓ of a 8 % aqueous solution of sodium hydroxide was added dropwise thereto, and hydrolysis was conducted at room temperature for 48 hours. Then, the solution was treated in the same manner as in Example 2 to give 3.40 g of light yellow powder (yield 97 %). The physical properties and elementary analysis of the obtained product were measured in the same manner as in Example 21.

The structural formula, the values of elementary analysis, the values of differential thermal analysis and the measurement results of the ultraviolet absorptive properties are shown in Table 1. And the measurement result of the infrared absorption spectrum is shown in Fig. 25, and the measurement result of the ultraviolet absorption spectrum is shown in Fig. 26.

Example 28

[Preparation of a copolymer of poly(vinyl-aldehyde 4-carboxymethyl-2-methylthiosemicarbazone) and N-vinylaminobutyric acid]

In the same manner as in Example 27, 1.00 g of vinylbenzaldehyde 4-ethoxycarbonylmethyl-2-methylthiosemicarbazone and 0.36 g of N-vinyl-2-pyrrolidone were dissolved in 2.5 mℓ of dry dioxane. After 1 mℓ of a solution of 15 mg of α,α'-azobisisobutyronitrile in dioxane was added dropwise thereto to copolymerize them, the obtained 1.20 g of light yellow powder was hydrolyzed and treated with dilute hydrochloric acid to give 1.00 g of light yellow powder (yield 73 %). The physical properties and elementary analysis of the obtained product were measured in the same manner as in Example 21.

The structural formula, the values of

elementary analysis, the values of differential thermal analysis and the measurement results of the ultraviolet absorptive properties are shown in Table 1. Also, the measurement result of the infrared absorption spectrum is shown in Fig. 27, and the measurement result of the ultraviolet absorption spectrum is shown in Fig. 28.

Example 29

[Preparation of a copolymer of poly(p-vinylbenzaldehyde 2-carboxymethyl-4,4-dimethylthiosemicarbazone) and maleic acid]

After a solution of 2.93 g of p-vinylbenzaldehyde 2-caroxymethyl-4,4-dimethylthiosemicarbazone and 0.49 g of maleic anhydride in 10 mℓ of dry N,N-dimethylformamide was heated to 60° to 62°C while passing dry nitrogen gas and stirring, 2 mℓ of a solution of 0.07 g of α,α'-azobisisobutyronitrile in N,N-dimethylformamide previously prepared was added dropwise to the solution, and the reaction was carried out at 60° to 62°C for 24 hours. After the reaction, the reaction solution was cooled with ice water, and poured into 200 mℓ of methanol. Thus the produced polymer was precipitated, and collected by filteration. The polymer was dissolved again in 5 mℓ of N,N-dimethylformamide, and the solution was poured into 200 mℓ of methanol to reprecipitate the polymer. The polymer was collected by filteration, and dried under vacuum to give 2.60 g of light yellow powder (yield 76 %). Then, 2.60 g of the obtained copolymer was treated in the same manner as in Example 25 to give 2.58 g of light yellow powder (yield 99 %). The physical properties and elementary analysis of the obtained product were measured in the same manner as in Example 21.

The structural formula, the values of elementary analysis, the values of differential thermal analysis and the measurement results of the ultraviolet absorptive properties are shown in Table 1. Also, the measurement result of the infrared absorption spectrum is

shown in Fig. 29, and the measurement result of the ultraviolet absorption spectrum is shown in Fig. 30.

Example 30

[Preparation of poly(p-vinylbenzaldehyde 4-ethoxycarbonylmethyl-2-methylthiosemicarbazone)]

There was dissolved 20.0 g of p-vinylbenzaldehyde 4-ethoxycarbonylmethyl-2-methylthiosemicarbazone in 100 mℓ of dry N-methylpyrrolidone. After heating the solution to 60° to 62°C while passing dry nitrogen gas and stirring, 10 mℓ of a solution of 0.70 g of α,α'-azobisisobutyronitrile in N-methylpyrrolidone previously prepared was added dropwise thereto, and the reaction was carried out at 60° to 62°C for 20 hours. After the reaction, the reaction solution was cooled with ice water, and poured into 1.0 ℓ of methanol. Thus the polymer was precipitated, and collected by filteration. The polymer was dissolved again in 40 mℓ of N-methylpyrrolidone, the solution was poured into 1.0 ℓ of methanol to reprecipitate the polymer. The polymer was collected by filteration, and dried under vacuum to give 19.80 g of light yellow powder (yield 99 %). The physical properties and elementary analysis of the obtained powder were measured in the same manner as in Example 21. Also, the weight average molecular weight was measured in the same manner as in Example 21 after hydrolysis was conducted in the same manner as in Example 22.

The structural formula, the values of elementary analysis, the values of differential thermal analysis and the measurement results of the ultraviolet absorptive properties are shown in Table 1. Also, the measurement result of the infrared absorption spectrum is shown in Fig. 31, and the measurement result of the ultraviolet absorption spectrum is shown in Fig. 32.

Example 31

[Preparation of a copolymer of poly(vinyl-benzaldehyde 4-carboxymethyl-2-methylsemicarbazone) and maleic acid]

In the same manner as in Example 25, 5.00 g of vinylbenzaldehyde 4-carboxymethyl-2-methylsemicarbazone and 0.93 g of maleic anhydride were dissolved in 13 mℓ of dry dioxane. After adding 0.06 g of α,α'-azobisisobutyronitrile thereto to copolymerize them, the obtained 1.50 g (yield 25 %) of light yellow powder was hydrolyzed and then treated with dilute hydrochloric acid to give 1.34 g of light yellow powder (yield 89 %). The physical properties and elementary analysis of the obtained powder were measured in the same manner as in Example 21.

The structural formula, the values of elementary analysis, the values of differential thermal analysis and the measurement results of the ultraviolet absorptive properties are shown in Table 1. Also, the measurement result of the infrared absorption spectrum is shown in Fig. 23, and the measurement result of the ultraviolet absorption spectrum is shown in Fig. 24.

Table 1

| Ex. | Structural formula | Weight average molecular weight | ℓ/m |
| --- | --- | --- | --- |
| 21 | 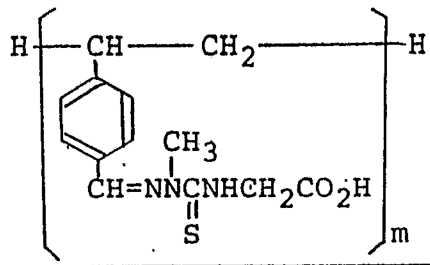 | 10000 | – |
| 22 | 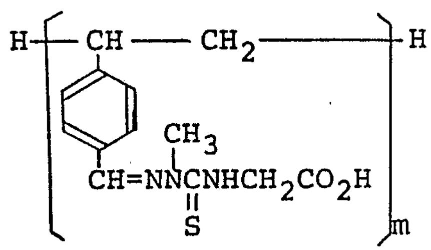 | 16000 | – |
| 23 | 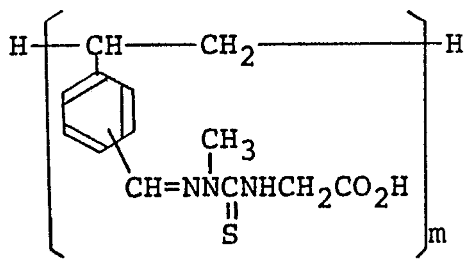 | 300000 | – |

- continued -

- continued -

| Ex. | Structural formula | Weight average molecular weight | ℓ/m |
|---|---|---|---|
| 24 | A random copolymer represented by 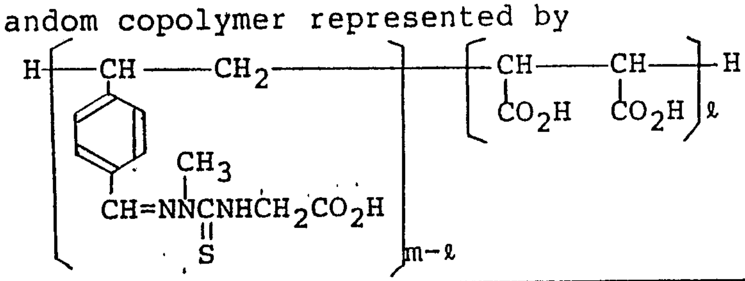 | 15000 | 0.47 |
| 25 | A random copolymer represented by 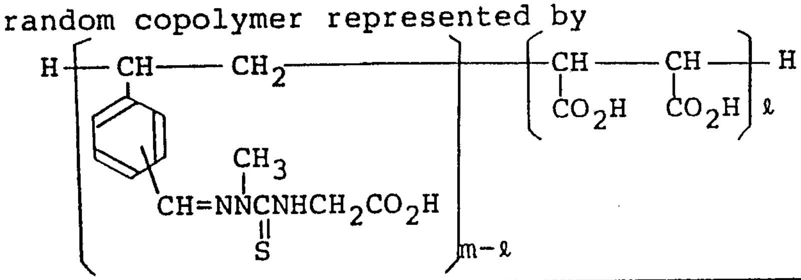 | 200000 | 0.42 |
| 26 | 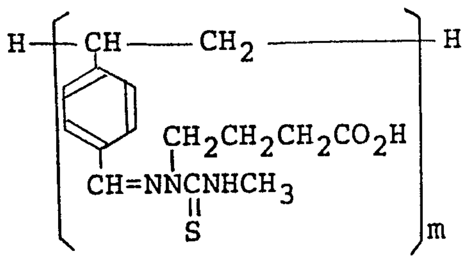 | 10000 | - |

- continued -

- continued -

| Ex. | Structural formula | Weight average molecular weight | ℓ/m |
|---|---|---|---|
| 27 | A random copolymer represented by 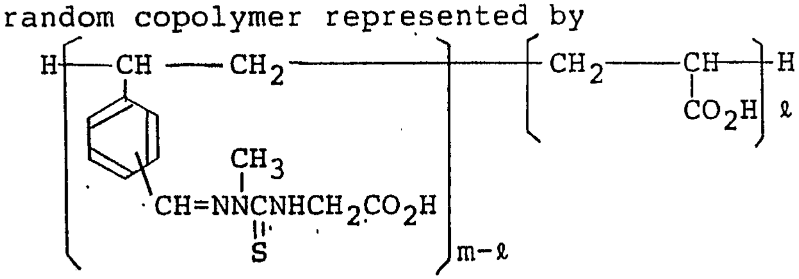 | 200000 | 0.40 |
| 28 | A random copolymer represented by 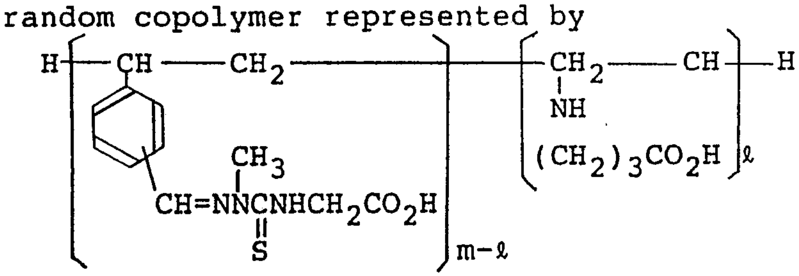 | 150000 | 0.56 |
| 29 | A random copolymer represented by 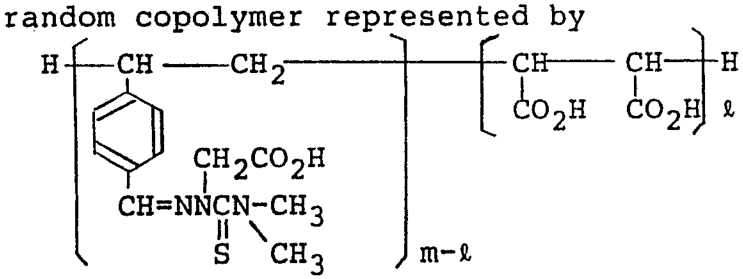 | 25000 | 0.53 |

- continued -

- continued -

| Ex. | Structural formula | Weight average molecular weight | ℓ/m |
| --- | --- | --- | --- |
| 30 | 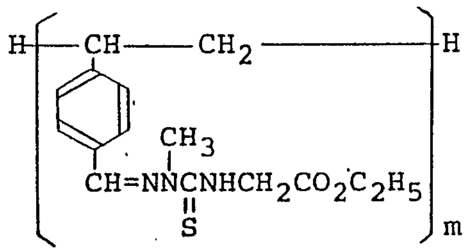 | 16000 | - |
| 31 | A random copolymer represented by 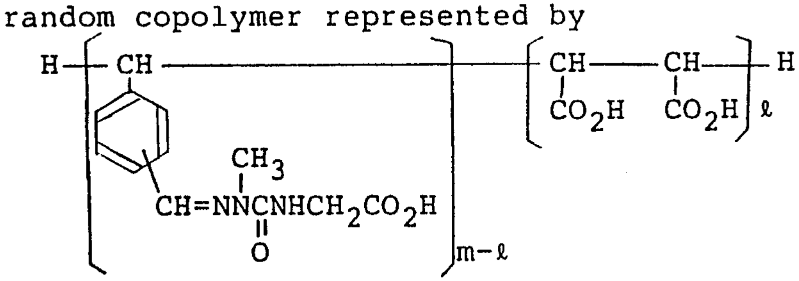 | 1000000 | 0.61 |

-continued-

- continued -

| Ex. | Elementary analysis (%) C | H | N | S | Differential thermal analysis | Ultraviolet absorptive properties: Wavelength of ultraviolet at the maximum absorption and the extinction coefficient (a) |
|---|---|---|---|---|---|---|
| 21 | 55.6 | 6.3 | 14.6 | 8.4 | 60.0°C (Endothermic)<br>131.0°C (Endothermic)<br>240.5°C (Exothermic)<br>377.4°C (Exothermic) | 323 nm (DMF)<br>a 116.3 |
| 22 | 55.6 | 6.3 | 14.6 | 8.4 | 60.0°C (Endothermic)<br>131.0°C (Endothermic)<br>240.5°C (Exothermic)<br>377.4°C (Exothermic) | 323 nm (DMF)<br>a 116.3 |
| 23 | 55.3 | 6.0 | 14.4 | 9.4 | 220.0°C (Exothermic)<br>370.0°C (Exothermic) | 323 nm (DMF)<br>a 86.9 |
| 24 | 51.9 | 6.2 | 11.1 | 7.8 | 260.0°C (Exothermic)<br>380.0°C (Exothermic) | 322 nm (DMF)<br>a 87.6 |

- continued -

- continued -

| | Elementary analysis (%) | | | | Differential thermal analysis | Ultraviolet absorptive properties |
|---|---|---|---|---|---|---|
| Ex. | C | H | N | S | | Wavelength of ultraviolet at the maximum absorption and the extinction coefficient (a) |
| 25 | 50.9 | 5.6 | 11.6 | 7.9 | 220.0°C (Exothermic)<br>380.0°C (Exothermic) | 320 nm (DMF)<br>a 80.2 |
| 26 | 56.5 | 6.4 | 12.9 | 8.3 | 167.5°C (Exothermic)<br>363.0°C (Exothermic) | 324 nm (DMF)<br>a 89.1 |
| 27 | 54.5 | 5.9 | 13.2 | 9.2 | 227.5°C (Exothermic)<br>367.5°C (Exothermic) | 321 nm (DMF)<br>a 70.4 |
| 28 | 54.9 | 5.9 | 13.6 | 9.1 | 227.5°C (Exothermic)<br>359.0°C (Exothermic) | 322 nm (DMF)<br>a 37.3 |
| 29 | 53.5 | 6.3 | 10.3 | 6.9 | 150.0°C (Exothermic)<br>357.6°C (Exothermic) | 316 nm (DMF)<br>a 54.9 |

- continued -

- continued -

| Ex. | Elementary analysis (%) C | H | N | S | Differential thermal analysis | Ultraviolet absorptive properties: Wavelength of ultraviolet at the maximum absorption and the extinction coefficient (a) |
|---|---|---|---|---|---|---|
| 30 | 58.7 | 6.5 | 13.8 | 9.6 | 173.4°C (Exothermic)<br>267.4°C (Exothermic)<br>367.5°C (Exothermic) | 323 nm (DMF)<br>a 118.5 |
| 31 | 51.6 | 6.0 | 9.2 | - | 185.0°C (Exothermic)<br>365.6°C (Exothermic) | 292 nm (DMF)<br>a 43.9 |

(Note) The polymers obtained in Examples 23, 25, 27, 28 and 31 are mixtures wherein (thio)semicarbazone group is combined with a benzene ring, at the m- or p- position to the alkylene group.

From the results mentioned above, it is confirmed that the polystyrene derivative of the prevsent invention excellently absorbs ultraviolet rays particularly having a wavelength of 290 to 350 nm.

Example 32

[Preparation of poly(p-vinylbenzaldehyde 4-carboxymethyl-2-phenylthiosemicarbazone)]

There was dissolved 6.00 g of p-vinylbenzaldehyde 4-carboxymethyl-2-phenylthiosemicarbazone in 36 mℓ of dry tetrahydrofuran. After heating the solution to 60° to 62°C while passing through dry nitrogen gas and stirring, 4 mℓ of a solution of 0.12 g of α,α'-azobisisobutyronitrile in tetrahydrofuran previously prepared was added thereto, and the reaction was carried out at 60° to 62°C for 24 hours. After the reaction, the reaction solution was treated in the same manner as in Example 21 to give 4.20 g of light yellow powder (yield 70 %). The physical properties and elementary analysis of the obtained product were conducted in the same manner as in Example 21 except that the following apparatuses were used.

As apparatuses for the analysis, a diffraction grating infrared spectrometer (1R-700 type, made by JAPAN SPECTROSCOPIC Co., LTD.), an elementary analyzer (CHN CORDER MT-3, Yanagimoto Co., Ltd.) and a differential thermal analyzer (TG-DTA 200, made by Seiko Instruments Inc.) were used.

The results are as follows:

Weight average molecular weight: 17,000

Elementary analysis: C 57.6 %, H 4.6 %, N 11.2 %, S 8.5 %

Differential thermal analysis:

271.9°C (Exothermic)

387.2°C (Exothermic)

Ultraviolet absorptive properties:

Wavelength at the maximum absorption: 322 nm (DMF)

Extinction coefficient (a): 84.0

Hereinafter the sunscreening agent of the present invention is specifically explained by means of Examples. However, it is to be understood that the present invention is not limited to the Examples.

Example 33

On a water bath, 4.8 g of white vaseline and 4.3 g of stearyl alcohol were mixed with heating (75°C). Next, 3.0 g of propylene glycol, 0.4 g of sodium lauryl sulfate, 1.0 g of the polystyrene derivative obtained in Example 21, 0.5 g of triethanolamine and 11.0 g of purified water were mixed, and the mixture was heated (75°C) to give a uniform solution. Then, the solution was added to the mixture of white vaseline and stearyl alcohol, thoroughly mixed with stirring, and cooled to room temperature to give a sunscreen ointment.

The obtained sunscreen ointment had properties like those of a hydrophilic ointment given in Japanese pharmacopoeia. Then, the effect of sunscreen was examined according to the following method. As the result, when the sunscreen ointment obtained in the above was applied, a difference of the color in the applied area was not observed compared with the color in an area which was not irradiated with ultraviolet rays, while the skin in the control area turned red nearly crimson.

(Test method)

Hair of the notal of three guinea pigs was cut, and the drug (ointment) was applied in an amount of about 2 mg per 1 $cm^2$. Then, ultraviolet rays were irradiated for 10 minutes by a fluorescent light (FL-20SE, made by TOSHIBA CORPORATION) from a height of 10 cm from the notal. On the other hand, as a control area, a base prepared without mixing the sunscreening agent was applied, and treatments were carried out in the same manner as above. The judgement was conducted after 24 hours with the naked eyes.

Example 34

In a mortar were put 1.0 g of the polystyrene derivative obtained in Example 22, 0.4 g of 2-amino-2-methyl-1-propanol and 9.6 g of purified water, and thoroughly dissolved with stirring. Then, 38 g of a white ointment given in Japanese pharmacoepia was added thereto and kneaded sufficiently to give a sunscreen ointment. The sunscreen effect of the obtained ointment was examined in the same manner as in Example 33. As the result, when the sunscreen ointment obtained in the above was applied, a difference of the color in the applied area was not observed compared with the color in an area which was not irradiated with ultraviolet rays, while the skin in the control area turned red nearly crimson.

Example 35

On a water bath, 0.1 g of white beeswax was dissolved in 0.7 g of cetyl alcohol and well stirred, and was maintained at about 75°C. Thereto was added a solution prepared by mixing and dissolving 1.0 g of the polystyrene derivative previously obtained in Example 23, 0.09 g of caustic soda, 0.3 g of sodium lauryl sulfate, 2.5 g of glycerol and 45.4 g of purified water and heating to 75°C, and naturally cooled to room temperature with thoroughly stirring to give a sunscreen lotion.

The sunscreen effect of the obtained sunscreen lotion was examined in the same manner as in Example 33. As the result, when the sunscreen lotion obtained in the above was applied, a difference of the color in the applied area was not observed compared with the color in an area which was not irradiated with ultraviolet rays, while the skin in the control area turned red, nearly crimson.

Examples 36 to 43

Sunscreen ointments were obtained in the same manner as in Example 31 except that the polystyrene derivative used in Example 33 was changed into 1.0 g of

the polystyrene derivatives obtained in Examples 24 to 31, respectively. The sunscreen effect of the obtained ointments were examined in the same manner as in Example 33. As the result, in each case, the color in the area on which the ointment was applied was not changed compared with the color in an area which was not irradiated with ultraviolet rays.

Example 44

To a base solution of 0.12 g of sodium hydroxide, 0.08 g of potassium hydroxide and 6.26 g of purified water were added 2.40 g of the polystyrene derivative obtained in Example 3, 0.38 g of 2-amino-2-methyl-1-propanol and 0.76 g of glycerol, which was heated to 50°C with stirring, and naturally cooled to room temperature with sufficiently stirring to give a sunscreen lotion.

The sunscreen effect of the obtained sunscreen lotion was examined in the same manner as in Example 32. As the result, the color in the area on which the sunscreen lotion was applied was not changed compared with the color in an area which was not irradiated with ultraviolet rays.

Example 45

A sunscreen ointment was obtained in the same manner as in Example 33 except that the polystyrene derivative used in Example 33 was changed into 1.0 g of the polystyrene derivative obtained in Example 32. The sunscreen effect of the obtained ointment was examined in the same manner as in Example 33. As the result, the color in the area on which the ointment was applied was not changed compared with the color in an area which was not irradiated with ultraviolet rays.

Example 46

[Preparation of a copolymer of p-vinylbenzaldehyde 2,4-dimethylthiosemicarbazone and maleic acid copolymer]

There were dissolved 12.00 g of p-vinylbenzaldehyde 2,4-dimethylthiosemicarbazone and 5.00 g of maleic anhydride in 100 mℓ of dry dimethylformamide. After heating the solution to 60° to 62°C while passing through dry nitrogen gas and stirring, 5 mℓ of a solution of 0.50 g of α,α'-azobisisobutyronitrile in dimethylformamide previously prepared was added thereto, and the reaction was carried out at 60° to 62°C for 23 hours. After the reaction, the reaction solution was cooled with ice water, poured into 1 ℓ of ethanol to precipitate the polymer, and then the polymer was collected by filteration. The polymer was dissolved again in 80 mℓ of dimethylformamide, poured into 1 ℓ of ethanol to reprecipitate, collected by filteration, and dried under vacuum to give 16.40 g of colorless powder (yield 96 %). The powder was dissolved in 300 mℓ of dimethyl sulfoxide, 150 mℓ of 1N-NaOH was added thereto, and stirred at room temperature for 30 minutes. After the reaction solution was diluted with 1 ℓ of water, the solution was poured into a cellophane tube (made by WAKO PURE CHEMICAL INDUSTRY) to conduct dialysis five times with 20 ℓ of water. After the dialysis, pH of the dialyzed solution in the tube was adjusted to 2 by adding dilute hydrochloric acid. The produced precipitate was collected by filteration, washed with water and then with methanol, and was dried under vacuum to give 15.60 g of colorless powder (yield 95 %).

The physical properties and elementary analysis of the obtained product were measured according to the following methods. The structural formula, the values of elementary analysis, the values of differential thermal analysis and the measurement result of the ultraviolet absorption properties are shown in Table 2. Also, the ultraviolet absorption spectrum is shown in Fig. 37, and the infrared absorption spectrum is shown in Fig. 38.

(Differential thermal analysis)

The change from room temperature to 475°C was measured by using a thermal analysis station (TAS-100

type, made by Rigaku Denki Co., Ltd.).

(Infrared absorption spectrum)

The measurement was carried out by using a diffraction grating infrared spectrophotometer (1R-700 type, made by JAPAN SPECTROSCOPIC CO., LTD.) according to KBr method.

(Elementary analysis)

Carbon, hydrogen and nitrogen were analyzed at the same time by using an elementary analyzer (CHN CORDER MT-3, made by Yanagimoto Co., LTD), and sulfur was analyzed according to oxygen-flask method.

(Ultraviolet absorption spectrum)

The measurement was carried out by using a spectrophotometer (UV-200S, made by SHIMADZU CORPORATION), as a dimethylformamide solution of the sample having a suitable concentration, with a quartz cell having an optical path of 10 mm.

(Avarage molecular weight)

Suitable amount of the sample was dissolved in a 0.01N NaOH aqueous solution, and analyzed by using a highperformance liquid chromatography (made by TOSOH Co., LTD., pump: CCPE, column: TSK gel G 4000SWXL 7.8 x 300 mm + G 3000$SW_{XL}$ 7.8 x 300 mm) and using water-soluble polystyrenes (made by SHOWA DENKO K. K., weight average melocular weight: 690,000 to 1,600) as standard substances.

Next, with respect to the polystyrene derivative obtained in Example 46, acute toxicity and local stimulation were examined. The results are as follows:

[Acute toxicity]

Oral administration was conducted against mice, and $LD_{50}$ was found. Concerning $LD_{50}$, no mouse died even by the administration of 5000 mg/kg, and the mice recovered as good as normal mice. Also, sex difference was not observed.

[Local stimulation]

(a) Into the conjunctival sac of a rabbit, 0.1

mℓ of a 10 % aqueous solution of the polystyrene derivative was dropped, and the stimulation was measured according to the criteria for the judgement of Draize, and Kay and Calandra. As the result, it was judged that there was no stimulation. (b) A scratch was given to the notal skin of a guinea pig with a sterilized injection needle of 18 gauge, and thereon an adhesive tape used for a patch test impregnated with 1 mℓ of a 10 % aqueous solution of the polystyrene derivative was applied. Then, the estimation of the stimulation was conducted according to the criteria of Draize. As the result, it was judged that the stimulation was mild, and in fact, there was no stimulation.

[Percutaneous absorption]

The skin of a quinea pig was peeled, washed with physiological saline and then set in a percutaneous absorption apparatus which was made in accordance with the description in Franz, T. J, J. Invest, Dermatol, 64, 190, 1975, by using a diffusion cell. The sample room was filled with 0.5 mℓ of a 1 % aqueous solution of the polystyrene derivative, and the acceptor was filled with physiological saline. After maintaining at 33°C for 20 hours while stirring with a magnetic stirrer, the physiological saline in the acceptor was taken out, and the ultraviolet absorbency was measured. In the same manner as above, ultraviolet absorbency was measured, using water alone as a negative control substance and a 1 % aqueous solution of p-aminobenzoic acid as a positive control substance. As the result, the sunstance of the polystyrene derivative showed an absorption curve similar to that of the negative control substance. To the contrary, the spectrum of the positive control substance was shown the corresponding to an amount of 93.0 ppm of the compound.

From the results mentioned above, it is confirmed that the polystyrene derivative is not permeated into skin.

From the matter mentioned above, it is

confirmed that the polystyrene derivative of the present invention does not involve stimulation or percutaneous absorption, and is useful not only for an ordinary sunscreen but also for protecting the exposed skin of patients of hypersensitiveness against sunlight.

Example 47

[Preparation of a copolymer of p-vinylbenzaldehyde 2,4-dimethylthiosemicarbazone and maleic acid monoamide]

In the same manner as in Example 46, 6.00 g of p-vinylbenzaldehyde 2,4-dimethylthiosemicarbazone and 2.50 g of maleic anhydride were polymerized, and reprecipitation was conducted to give 8.10 g of colorless powder of a copolymer (yield 94 %). Then, 8.10 g of the obtained powder was dissolved in 160 mℓ of dimethyl sulfoxide, 20 mℓ of 28 % aqueous ammonia was added thereto, and the mixture was stirred at room temperature for 30 minutes. The reaction solution was diluted by pouring into 1 ℓ of water and poured into a cellophane tube (made by WAKO PURE CHEMICAL INDUSTRY) to conduct dialysis 5 times with 10 ℓ of water. After the dialysis, pH of the dialyzed solution in the tube was adjusted to 2 by adding dilute hydrochloric acid. The produced precipitate was collected by filteration, washed with water, and dried under vacuum to give 7.50 g of colorless powder (yield 88 %).

The physical properties and elementary analysis of the obtained product were measured in the same manner as in Example 46. The results are shown in Table 2. Also, the ultraviolet absorption spectrum is shown in Fig. 39, and the infrared absorption spectrum is shown in Fig. 40.

Example 48

[Preparation of a copolymer of p-vinylbenzaldehyde 2,4-dimethylthiosemicarbazone and acrylic acid]

There were dissolved 10.0 g of p-vinylbenzaldehyde 2,4-dimethylthiosemicarbazone and 5.0 g

of acrylic acid in 100 mℓ of dimethylformamide. After heating the solution to 80° to 85°C while passing through nitrogen gas and stirring, 5 mℓ of a solution of 0.50 g of α,α'-azobisisobutyronitrile in dimethylformamide previously prepared was added thereto and the reaction was carried out at 80° to 85°C for 18 hours. After the reaction, the reaction solution was cooled with ice water, poured into 1 ℓ of ethanol to precipitate the polymer and then the polymer was collected by filteration. The polymer was dissolved again in 80 mℓ of dimethylformamide, poured into 1 ℓ of ethanol to reprecipitate, collected by filteration, and dried under vacuum to give 13.4 g of coloress powder (yield 89 %).

The physical properties and elementary analysis of the obtained product were measured in the same manner as in Example 46. The results are shown in Table 2. Also, the ultraviolet absorption spectrum is shown in Fig. 41, and the infrared absorption spectrum is shown in Fig. 42.

Example 49

[Preparation of a copolymer of p-vinylbenzaldehydethiosemicarbazone and maleic acid]

In the same manner as in Example 46, 6.00 g of p-vinylbenzaldehydethiosemicarbazone was copolymerized with 2.86 g of maleic anhydride in 30 mℓ of N,N-dimethylformamide by using 0.18 g of α,α'-azobisisobutyronitrile at 60° to 62°C for 24 hours to give 6.32 g of colorless powder (yield 71 %). The powder was added to 300 mℓ of 1N-NaOH, and the solution was stirred at room temperature for 15 hours to hydrolyze. The reaction solution was poured into a cellophane tube to conduct dialysis, and treated with dilute hydrochloric acid to give 4.90 g of light yellow powder (yield 77 %). The physical properties and elementary analysis of the obtained product were measured in the same manner as in Example 46. As a differential thermal analyzer, TG-DTA200 made by Seiko instruments Inc. was used. The

results are shown in Table 2. Also, the infrared absorption spectrum is shown in Fig. 43, and the ultraviolet absorption spectrum is shown in Fig. 44.

Example 50

[Preparation of a copolymer of p-vinylbenzaldehyde 4-methyl-2-phenylthiosemicarbazone and maleic acid]

In the same manner as in Example 49, 5.70 g of p-vinylbenzaldehyde 4-methyl-2-phenylthiosemicarbazone was copolymerized with 1.68 g of maleic anhydride in 30 mℓ of N,N-dimethylformamide by using 0.14 g of α,α'-azobisisobutyronitrile at 80° to 82°C for 15 hours. Then, 6.00 g (yield 88 %) of the obtained colorless powder was hydrolyzed, dialyzed, and treated with dilute hydrochloric acid to give 5.29 g of colorless powder (yield 88 %).

The physical properties and elementary analysis of the obtained product were measured in the same manner as in Example 50. The results are shown in Table 2. Also, the infrared absorption spectrum is shown in Fig. 45, and the ultraviolet absorption spectrum is shown in Fig. 46.

Example 51

[Preparation of a copolymer of p-vinylbenzaldehyde 2-methyl-4-phenylthiosemicarbazone and maleic acid]

In the same manner as in Example 49, 6.00 g of p-vinylbenzaldehyde 2-methyl-4-phenylthiosemicarbazone was copolymerized with 1.98 g of maleic anhydride in 40 mℓ of tetrahydrofuran by using 0.16 g of α,α'-azobisisobutyronitrile at 60° to 62°C for 24 hours. Then, 4.80 g (yield 60 %) of the obtained colorless powder was hydrolyzed, dialyzed, and treated with dilute hydrochloric acid to give 3.96 g of colorless powder (yield 82 %).

The physical properties and elementary analysis of the obtained product were measured in the same manner as in Example 49. The results are shown in Table 2.

Also, the infrared absorption spectrum is shown in Fig. 47, and the ultraviolet absorption spectrum is shown in Fig. 48.

Example 52

[Preparation of a copolymer of p-vinylbenzaldehyde 2-(β-hydroxymethyl)-4-methylthiosemicarbazone and maleic acid]

In the same manner as in Example 49, 6.00 g of p-vinylbenzaldehyde 2-(β-hydroxymethyl)-4-methylthiosemicarbazone was copolymerized with 6.66 g of maleic anhydride in 40 mℓ of N,N-dimethylformamide by using 0.25 g of α,α'-azobisisobutyronitrile at 60° to 62°C for 24 hours. Then, 4.91 g (yield 39 %) of the obtained colorless powder was hydrolyzed, dialyzed, and treated with dilute hydrochloric acid to give 3.40 g of colorless powder (yield 69 %).

The physical properties and elementary analysis of the obtained product were measured in the same manner as in Example 49. The results are shown in Table 2. Also, the infrared absorption spectrum is shown in Fig. 49, and the ultraviolet absorption spectrum is shown in Fig. 50.

Example 53

[Preparation of a copolymer p-vinylbenzaldehyde 4-tert.-butyl-2-methylthiosemicarbazone and maleic acid]

In the same manner as in Example 49, 3.00 g of p-vinylbenzaldehyde 4-tert.-butyl-2-methylthiosemicarbazone was copolymerized with 3.18 g of maleic anhydride in 40 mℓ of tetrahydrofuran by using 0.12 g of α,α'-azobisisobutyronitrile at 60° to 62°C for 24 hours. Then, 4.32 g (yield 70 %) of the obtained colorless powder was hydrolyzed, dialyzed and treated with dilute hydrochloric acid to give 4.02 g of colorless powder (yield 93 %).

The physical properties and elementary analysis of the obtained product were measured in the same manner as in Example. The results are shown in Table 2. Also,

the infrared absorption spectrum is shown in Fig. 51, and the ultraviolet absorption spectrum is shown in Fig. 52.

Table 2

| Ex. | Structural formula | Weight average molecular weight | b/a |
|---|---|---|---|
| 46 | A random copolymer represented by 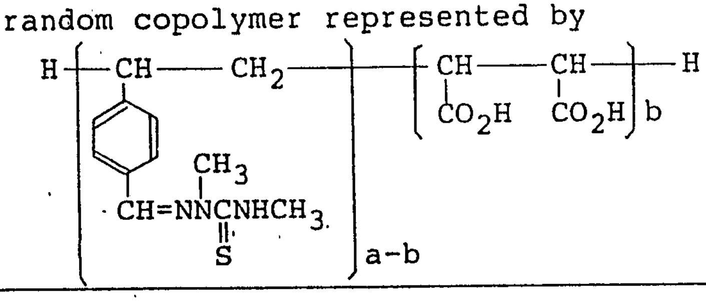 | 9700 | 0.52 |
| 47 | A random copolymer represented by 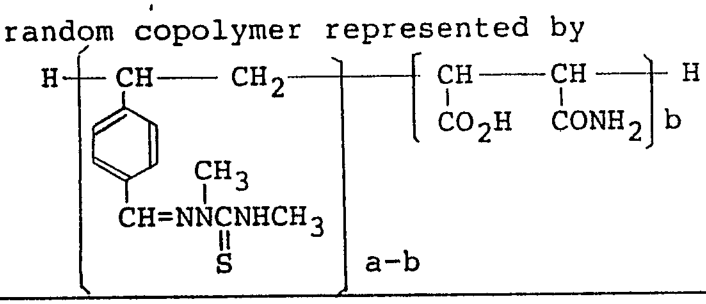 | 7000 | 0.49 |
| 48 | A random copolymer represented by 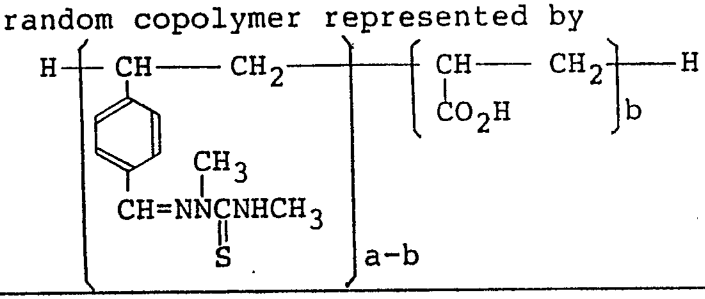 | 118000 | 0.56 |

- continued -

- continued -

| Ex. | Structural formula | Weight average molecular weight | b/a |
|---|---|---|---|
| 49 | A random copolymer represented by 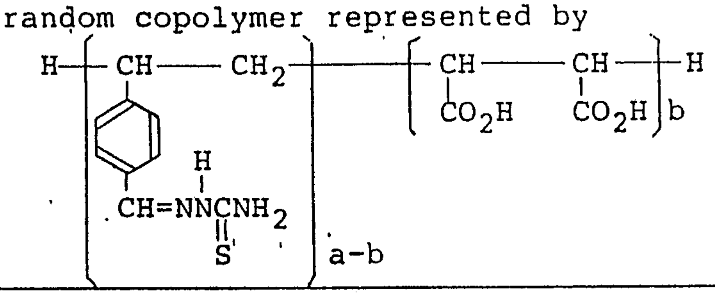 | 7400 | 0.61 |
| 50 | A random copolymer represented by 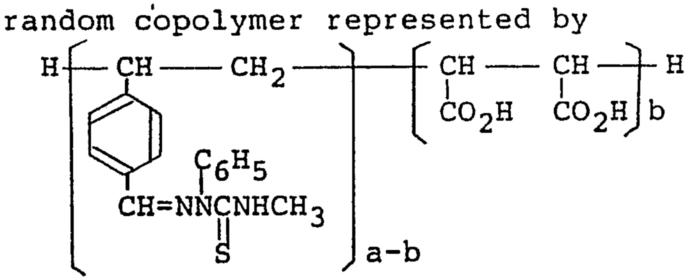 | 1800 | 0.62 |
| 51 | A random copolymer represented by 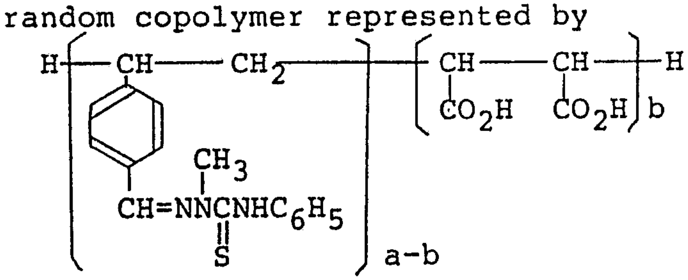 | 13000 | 0.81 |

- continued -

- continued -

| Ex. | Structural formula | Weight average molecular weight | b/a |
|---|---|---|---|
| 52 | A random copolymer represented by 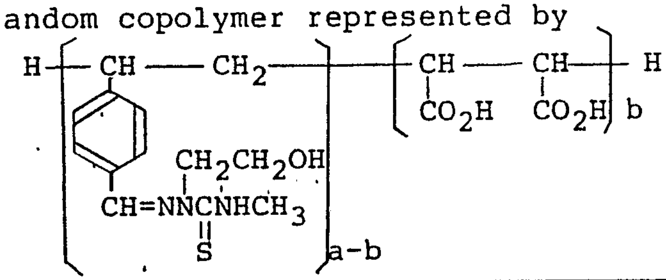 | 9200 | 0.81 |
| 53 | A random copolymer represented by 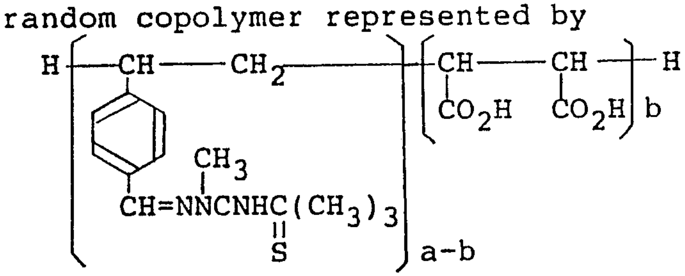 | 12000 | 0.72 |

- continued -

- continued -

| Ex. | Elementary analysis (%) C | H | N | S | Differential thermal analysis | Ultraviolet absorptive properties: Wavelength of ultraviolet at the maximum absorption and the extinction coefficient (a) |
|---|---|---|---|---|---|---|
| 46 | 54.7 | 5.6 | 12.2 | 9.2 | 65.3°C (Endothermic)<br>352.5°C (Exothermic) | 322 nm (DMF)<br>a 103 |
| 47 | 54.9 | 6.0 | 16.1 | 9.0 | 72.5°C (Endothermic)<br>237.3°C (Exothermic)<br>355.3°C (Exothermic) | 322 nm (DMF)<br>a 108 |
| 48 | 58.8 | 6.2 | 13.5 | 10.1 | 55.6°C (Endothermic)<br>217.0°C (Exothermic)<br>352.0°C (Exothermic) | 323 nm (DMF)<br>a 115 |
| 49 | 50.5 | 4.5 | 10.8 | 8.3 | 291.3°C (Exothermic)<br>389.1°C (Exothermic) | 312 nm (DMF)<br>a 77.6 |
| 50 | 58.3 | 4.9 | 8.7 | 6.6 | 239.9°C (Exothermic)<br>388.6°C (Exothermic) | 322 nm (DMF)<br>a 70.4 |

- continued -

- continued -

| Ex. | Elementary analysis (%) C | H | N | S | Differential thermal analysis | Ultraviolet absorptive properties: Wavelength of ultraviolet at the maximum absorption and the extinction coefficient (a) |
|---|---|---|---|---|---|---|
| 51 | 51.9 | 4.4 | 5.4 | 4.1 | 216.1°C (Exothermic)<br>357.4°C (Exothermic) | 325 nm (DMF)<br>a 46.0 |
| 52 | 47.4 | 4.5 | 5.4 | 4.1 | 232.6°C (Exothermic)<br>376.1°C (Exothermic) | 322 nm (DMF)<br>a 47.7 |
| 53 | 52.9 | 5.5 | 7.3 | 5.6 | 246.4°C (Exothermic)<br>357.3°C (Exothermic) | 322 nm (DMF)<br>a 63.6 |

From the results mentioned above, it is confirmed that the polystyrene derivative excellently absorbs ultraviolet rays having a wavelength of from 290 to 350 nm.

Next, the sunscreening agent of the present invention is specifically explained by means of Examples, but it is to be understood that the present invention is not limited to the Examples.

Example 54

On a water bath, 4.8 g of white vaseline and 4.3 g of stearyl alcohol were mixed with heating (75°C). Next, 3.0 g of propylene glycol, 0.4 g of sodium lauryl sulfate, 1.5 g of the polystyrene derivative obtained in Example 46, 0.8 g of triethanolamine and 11.0 g of purified water were mixed, and the mixture was heated (75°C) to give a uniform solution. Then, the solution was added to the mixture of white vaseline and stearyl alcohol, thoroughly mixed with stirring, and cooled to room temperature to give a sunscreen ointment.

The obtained sunscreen ointment had properties like those of a hydrophilic ointment given in Japanese pharmacopoeia. Then, the effect of sunscreen was examined according to the following method. As the result, when the sunscreen ointment obtained in the above was applied, a difference of the color in the applied area was not observed compared with the color in an area which was not irradiated with ultraviolet rays, while the skin in the control area turned red nearly crimson.

(Testing method)

Hair of the notal of three guinea pigs was cut, and the drug (ointment) was applied in an amount of about 2 mg per 1 $cm^2$. Then, ultraviolet rays were irradiated for 10 minutes by a fluorescent light (FL-20SE, made by TOSHIBA CORPORATION) from a height of 10 cm from the notal. On the other hand, as a control area, a base prepared without mixing the sunscreening agent was applied, and treatments were carried out in the same

manner as above. The judgement was conducted after 24 hours with the naked eyes.

Example 55

A sunscreen lotion was prepared by dissolving by well stirring 1.0 g of the polystyrene derivative obtained in Example 47, 0.4 g of 2-amino-2-methyl-1-propanol, 2.0 g of glycerol, 2.0 g of macrogol ointment given in Japanese Pharmacopoeia and 10.0 g purified water. The sunscreen effect of the obtained lotion was examined in the same manner as in Example 54. As the result, when the sunscreen ointment obtained in the above was applied, a difference of the color in the applied area was not observed compared with the color in an area which was not irradiated with ultraviolet rays, while the skin in the control area turned red nearly crimson.

Example 56

A sunscreen ointment was prepared by thoroughly mixing 1.0 g of the polystyrene derivative obtained in Example 48, 0.5 g of triethanolamine and 1.0 g of purified water to obtain a muddy solution, mixing 7.5 g of macrogol ointment given in Japanese Pharmacopoeia therewith, and kneading them.

The sunscreen effect of the obtained ointment was examined in the same manner as in Example 54. As the result, when the sunscreen ointment obtained in the above was applied, a difference of the color in the applied area was not observed compared with the color in an area which was not irradiated with ultraviolet rays, while the skin in the control plot turned red, nearly crimson.

Examples 57 to 61

Sunscreen ointments were prepared in the same manner as in Example 54 except that the polystyrene derevative obtained in Example 54 was changed into the polystyrene derivatives obtained in Examples 49 to 53. The sunscreen effect of the obtained ointments were

examined in the same manner as in Example 54. As the result, in each case, the color in the area on which the sunscreen ointment was applied was not different compared with the color in an area which was not irradiated with ultraviolet rays.

## INDUSTRIAL APPLICABILITY

It can be seen that the polystyrene derivative of the present invention can be suitably used as a sunscreening agent since the polystyrene derivative can be easily prepared to have water solubility, and has high safety to human body, and the range of its absorbing ultraviolet rays wavelength is the same as that of the wavelength when skin is sunburnt.

It can be also seen that in accordance with the method for preparing of the present invention, the polystyrene derivative having high quality and stability can be obtained since the polystyrene derivative can be purified at the stage of monomers.

Further, the styrene derivative of the present invention can be suitably used as a sunscreening agent for plastics since the styrene derivative of the present invention is excellent in ultraviolet absorptive property within a range of the wavelength of from 290 nm, which is the shortest wavelength contained in the sunlight reaching the surfaces of the earth, to 360 nm which affects the plastics in change.

CLAIMS

1. A styrene derivative represented by the general formula (I):

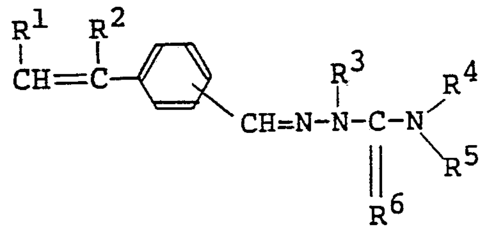

(I)

wherein at least one of $R^1$, $R^2$, $R^3$, $R^4$ and $R^5$ is carboxyl group, an alkyl group having 1 to 5 carbon atoms, a hydroxyalkyl group having 1 to 5 carbon atoms, a carboxyalkyl group having 2 to 6 carbon atoms, an alkoxycarbonyl group having 2 to 6 carbon atoms, an alkoxycarbonylalkyl group having 2 to 6 carbon atoms or an aryl group, the residual groups of above $R^1$, $R^2$, $R^3$, $R^4$ and $R^5$ are hydrogen atom or an alkyl group having 1 to 5 carbon atoms, $R^6$ is sulfur atom or oxygen atom.

2. A sunscreening agent of which active ingredient is a styrene derivative represented by the general formula (I):

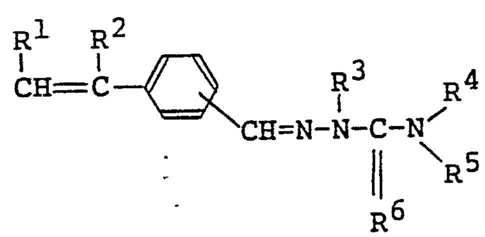

(I)

wherein at least one of $R^1$, $R^2$, $R^3$, $R^4$ and $R^5$ is carboxyl group, an alkyl group having 1 to 5 carbon atoms, a hydroxyalkyl group having 1 to 5 carbon atoms, a carboxyalkyl group having 2 to 6 carbon atoms, an alkoxycarbonyl group having 2 to 6 carbon atoms, an alkoxycarbonylalkyl group having 2 to 6 carbon atoms or an aryl group, the residual groups of above $R^1$, $R^2$, $R^3$,

$R^4$ and $R^5$ are hydrogen atom or an alkyl group having 1 to 5 carbon atoms, $R^6$ is sulfur atom or oxygen atom.

3. A polystyrene derivative represented by the general formula (II):

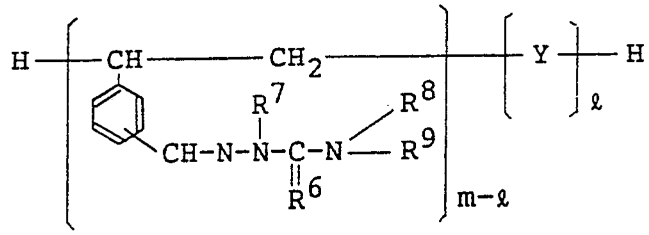

(II)

wherein at least one of $R^7$, $R^8$ and $R^9$ is carboxyl group, a carboxyalkyl group having 2 to 6 carbon atoms, an alkoxycarbonyl group having 2 to 6 carbon atoms or an alkoxycarbonylalkyl group having 2 to 6 carbon atoms, the residual groups of above $R^7$, $R^8$ and $R^9$ are hydrogen atom, a lower alkyl group having 1 to 5 carbon atoms, or an aryl group, $R^6$ is sulfur atom or oxygen atom, Y is

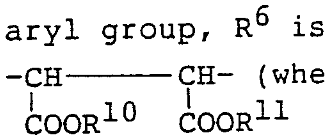

(wherein $R^{10}$ and $R^{11}$ are hydrogen atom or an alkyl group having 1 to 5 carbon atoms respectively),

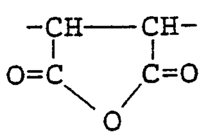

,

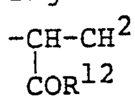

(wherein $R^{12}$ is -OH, $-NH_2$ or $-OC_pH_{2p+1}$ wherein p is an integer of 1 to 4),

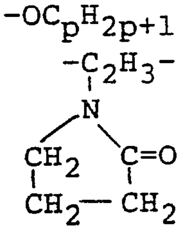

or

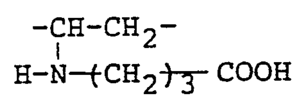

, m is an integer of 20 to 1500, ℓ is an integer of 0 to 1100 which satisfies m > ℓ.

4. The polystyrene derivative of Claim 3, wherein the partial constitutional formula in the general formula (II):

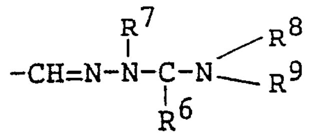 is

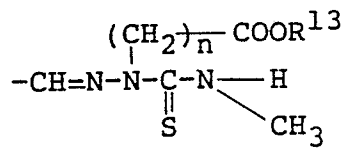 ,

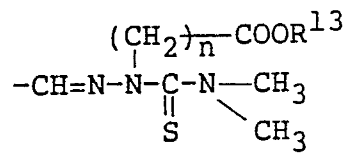 ,

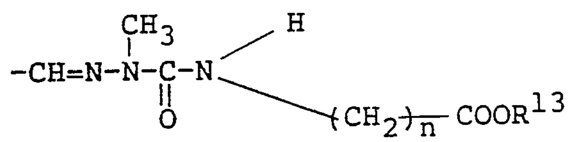 ,

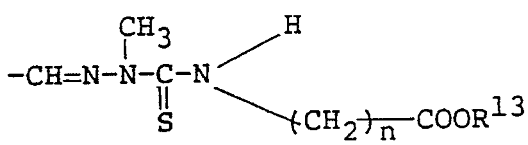 or

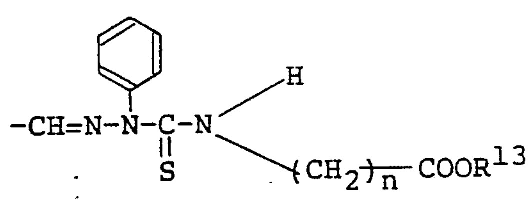

wherein $R^{13}$ is hydrogen atom, a lower alkyl group having 1 to 5 carbon atoms, ammonium group or an alkaline metal, n is an integer of 0 to 3.

5. A method for preparing a polystyrene derivative represented by the general formula (II):

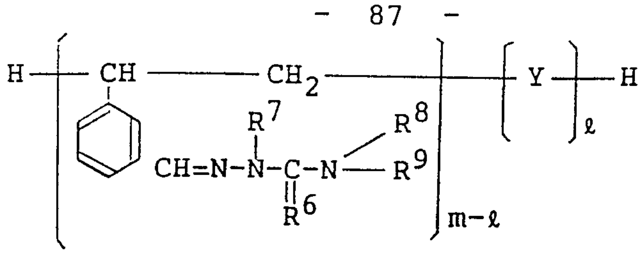

(II)

wherein at least one of $R^7$, $R^8$ and $R^9$ is carboxyl group, a carboxyalkyl group having 2 to 5 carbon atoms, an alkoxycarbonyl group or an alkoxycarbonylalkyl group having 2 to 6 carbon atoms, the residual groups of above $R^7$, $R^8$ and $R^9$ are hydrogen atom, a lower alkyl group having 1 to 5 carbon atoms or an aryl group, $R^6$ is sulfur atom or oxygen atom, Y is 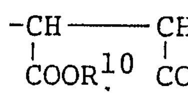 (wherein $R^{10}$ and $R^{11}$ are hydrogen atom or an alkyl group having 1 to 5 carbon atoms respectively), 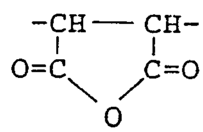, 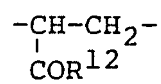 (wherein $R^{12}$ is $-OH$, $-NH_2$ or $-OC_pH_{2p+1}$ wherein p is an integer of 1 to 4), 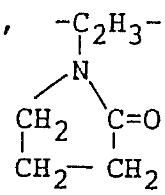 or 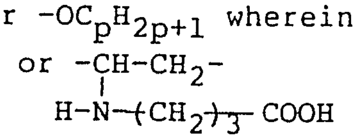, m is an integer of 10 to 1500, ℓ is an integer of 0 to 1100 which satisfies m > ℓ, characterized by using an azo-compound or a peroxide as a polymerization initiator and homopolymerizing in a solution a styrene derivative represented by the general formula (III):

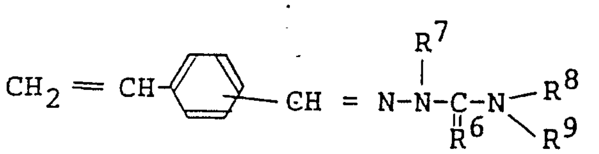

(III)

wherein at least one of $R^7$, $R^8$ and $R^9$ is carboxyl group, a carboxyalkyl group having 2 to 5 carbon atoms, an alkoxycarbonyl group or an alkoxycarbonylalkyl group having 2 to 6 carbon atoms, the residual groups of above $R^7$, $R^8$ and $R^9$ are hydrogen atom, a lower alkyl group having 1 to 5 carbon atoms or an aryl group, $R^6$ is sulfur

atom or oxygen atom, or copolymerizing at least two kinds of said styrene derivatives, or copolymerizing the said styrene derivative and a compound containing unsaturated aliphatic groups having at least one double bond between carbon atoms, at least one of said unsaturated aliphatic groups being terminated by carboxyl group or a salt thereof, anhydride, ester, amide or imide.

6. The method for preparing the polystyrene derivative of Claim 5, wherein said compound containing unsaturated aliphatic groups having at least one double bond between carbon atoms, at least one of said unsaturated aliphatic groups being terminated by carboxyl group or a salt thereof, anhydride, ester, amide or imide, is maleic acid, maleic anhydride, fumaric acid or acrylic acid, or a lower alkyl ester having 1 to 5 carbon atoms of maleic acid, fumaric acid or acrylic acid.

7. A sunscreening agent containing as an active ingredient, a polystyrene derivative represented by the general formula (II):

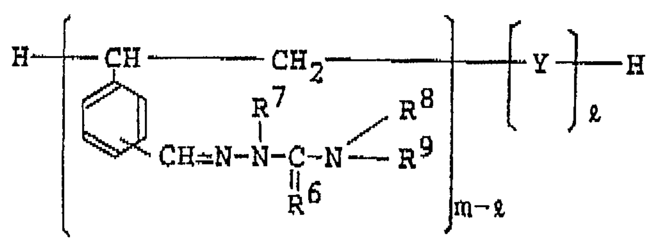

(II)

wherein at least one of $R^7$, $R^8$ and $R^9$ is carboxyl group, a carboxyalkyl group having 2 to 5 carbon atoms, an alkoxycarbonyl group or an alkoxycarbonylalkyl group having 2 to 6 carbon atoms, the residual groups of above $R^7$, $R^8$ and $R^9$ are hydrogen atom, a lower alkyl group having 1 to 5 carbon atoms or an aryl group, $R^6$ is sulfur atom or oxygen atom, Y is 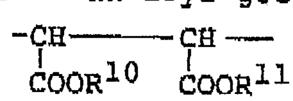 (wherein $R^{10}$ and $R^{11}$ are hydrogen atom or an alkyl group having 1

to 5 carbon atoms, respectively), 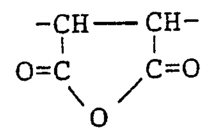 , 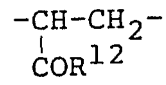 (wherein $R^{12}$ is -OH, $-NH_2$ or $-OC_pH_{2p+1}$ wherein p is an integer of 1 to 4), 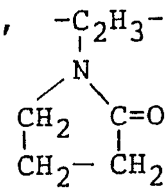 or 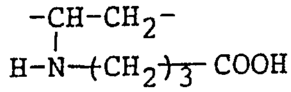 , m is an integer of 10 to 1500, ℓ is an integer of 0 to 1100 which satisfies m > ℓ.

8. The sunscreening agent of Claim 7, wherein the content of the polystyrene derivative is 0.1 to 50 % by weight.

9. The sunscreening agent of Claim 7, wherein the partial constitutional formula in the general formula (II):

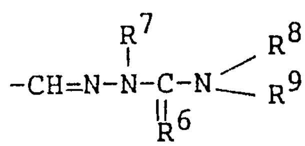 is

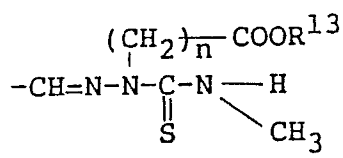 ,

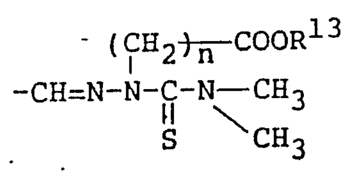 ,

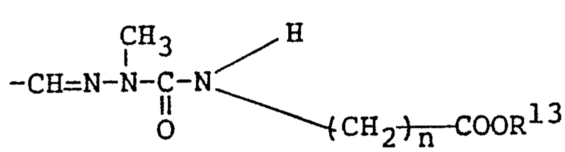 ,

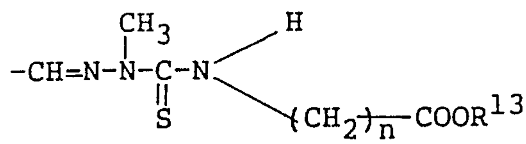 or

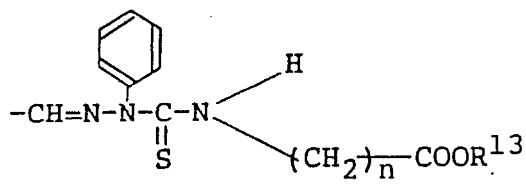

wherein $R^{13}$ is hydrogen atom, a lower alkyl group having 1 to 5 carbon atoms, ammonium group or an alkaline metal, n is an integer of 0 to 3.

10. A polystyrene derivative represented by the general formula (IV):

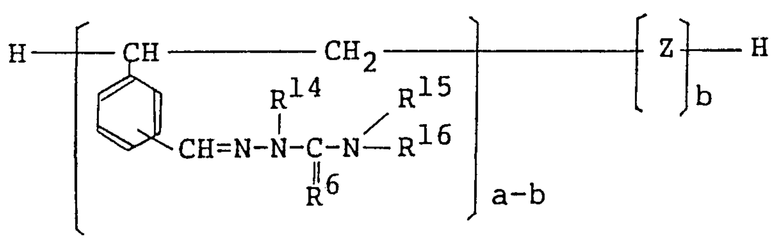 (IV)

wherein $R^{14}$, $R^{15}$ and $R^{16}$ is hydrogen atom, an alkyl group having 1 to 5 carbon atoms, a hydroxyalkyl group having 1 to 5 carbon atoms or an aryl group, respectively, $R^6$ is sulfur atom or oxygen atom, Z is 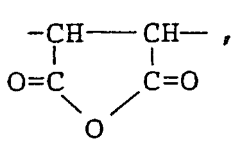, 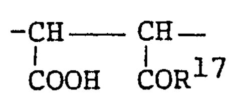 (wherein $R^{17}$ is -OH, $-NH_2$ or $-OC_pH_{2p+1}$ wherein p is an integer of 1 to 4) or 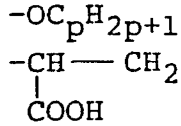, a is an integer of 10 to 1500, b is an integer which satisfies that b/a is 0.2 to 0.9.

11. A method for preparing a polystyrene derivative represented by the general formula (IV):

bond between carbon atoms, at least one of said unsaturated aliphatic groups being terminated by carboxyl group or a salt thereof, anhydride, ester, amide or imide, is maleic acid, maleic anhydride, fumaric acid or acrylic acid, or a lower alkyl ester having 1 to 5 carbon atoms of maleic acid, fumaric acid or acrylic acid.

13. A sunscreening agent containing as an active ingredient a polystyrene derivative represented by the general formula (IV):

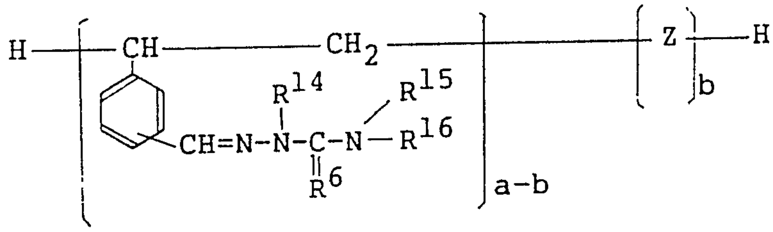

(IV)

wherein $R^{14}$, $R^{15}$ and $R^{16}$ are hydrogen atom, an alkyl group, a hydroxyalkyl group having 1 to 5 carbon atoms or an aryl group, respectively, $R^6$ is sulfur atom or oxygen atom, Z is

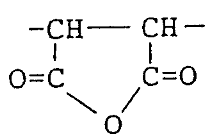 , 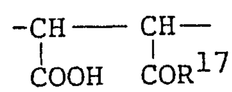 (wherein $R^{17}$ is -OH, $-NH_2$ or $-OC_pH_{2p+1}$ wherein p is an integer of 1 to 4) or 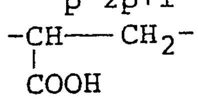, a is an integer of 10 to 1500, b is an integer which satisfies that b/a is 0.2 to 0.9.

14. The sunscreening agent of Claim 13 wherein the content of the polystyrene derivative is 0.1 to 50 % by weight.

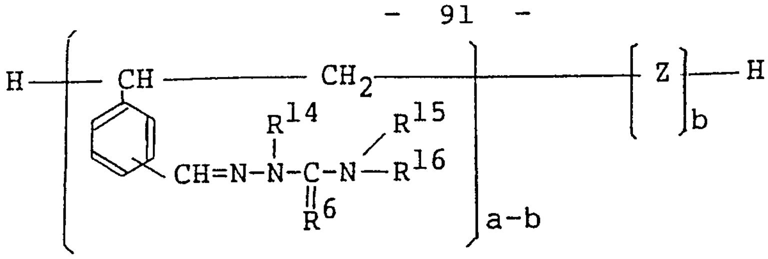

(IV)

wherein $R^{14}$, $R^{15}$ and $R^{16}$ are hydrogen atom, an alkyl group, a hydroxyalkyl group having 1 to 5 carbon atoms or an aryl group, respectively, $R^6$ is sulfur atom or oxygen atom, Z is

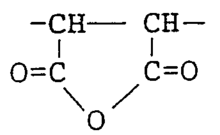 , 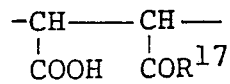 (wherein $R^{17}$ is -OH, $-NH_2$ or $-OC_pH_{2p+1}$ wherein p is an integer of 1 to 4) or 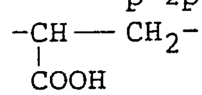, a is an integer of 10 to 1500, b is an integer which satisfies that b/a is 0.2 to 0.9, characterized by

using an azo-compound or a peroxide as a polymerization initiator and

copolymerizing at least one kind of styrene derivatives represented by the general formula (V):

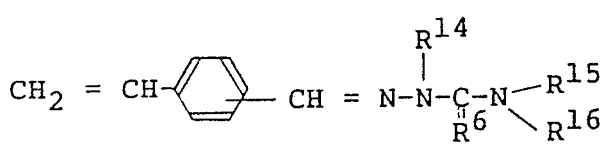

(V)

wherein $R^{14}$, $R^{15}$ and $R^{16}$ are hydrogen atom, an alkyl group, a hydroxyalkyl group having 1 to 5 carbon atoms or an aryl group, $R^6$ is sulfur atom or oxygen atom, and a compound containing unsaturated aliphatic groups having at least one double bond between carbon atoms, at least one of said unsaturated aliphatic groups being terminated by carboxyl group or a salt thereof, anhydride, ester, amide or imide.

12. The method for preparing the polystyrene derivative of Claim 11, wherein said compound containing unsaturated aliphatic groups having at least one double

FIG. 1

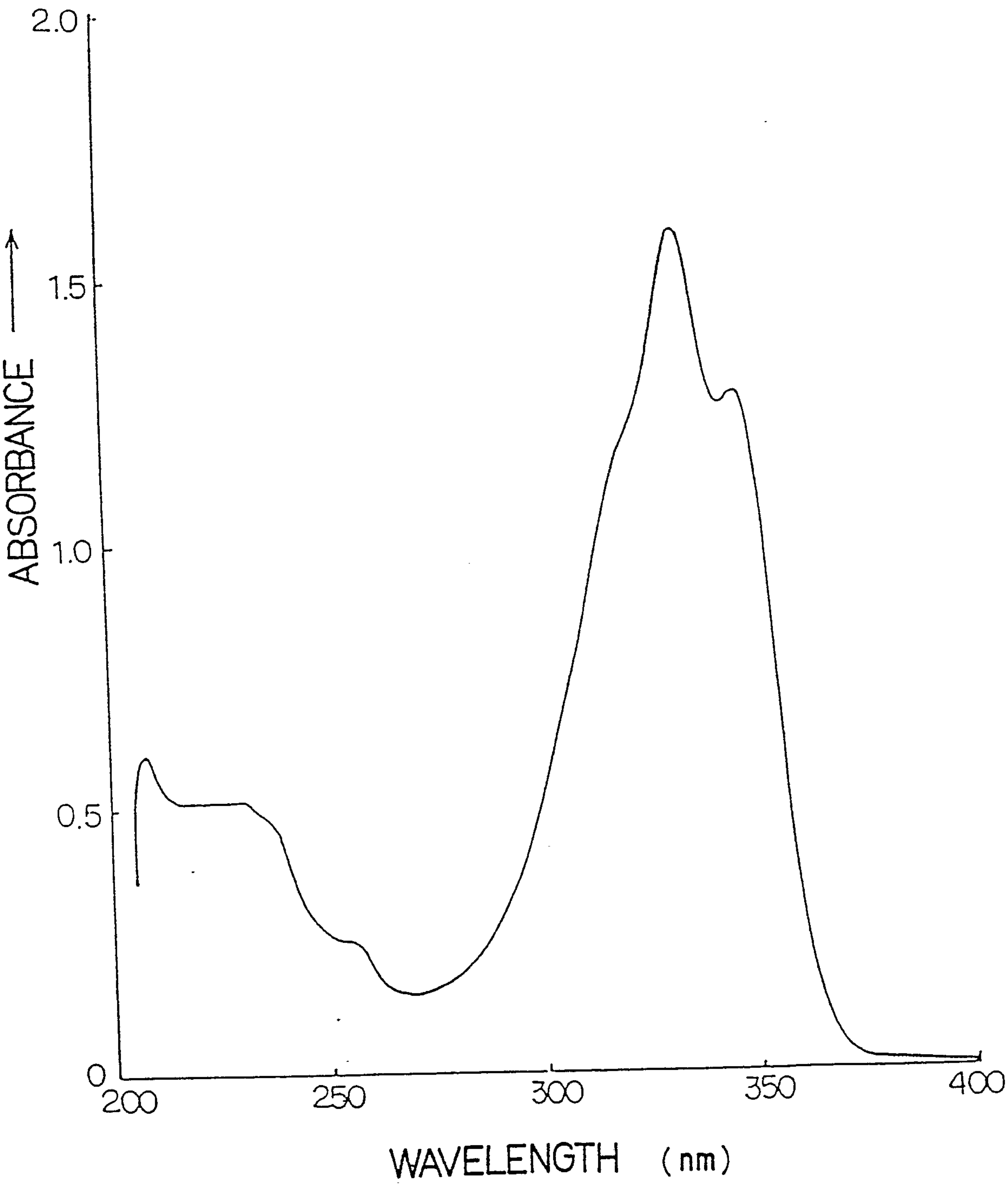

FIG. 2

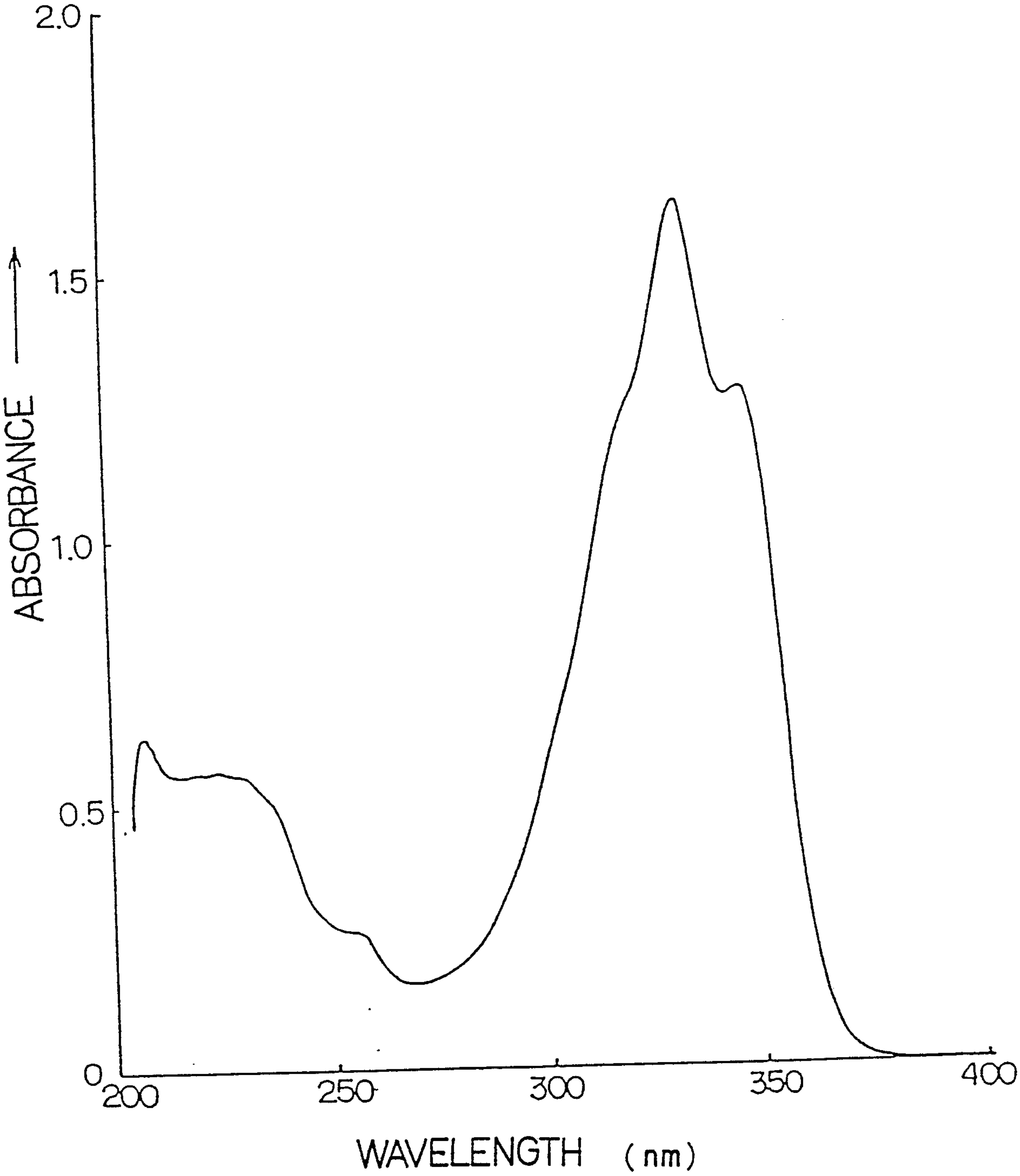

FIG. 3

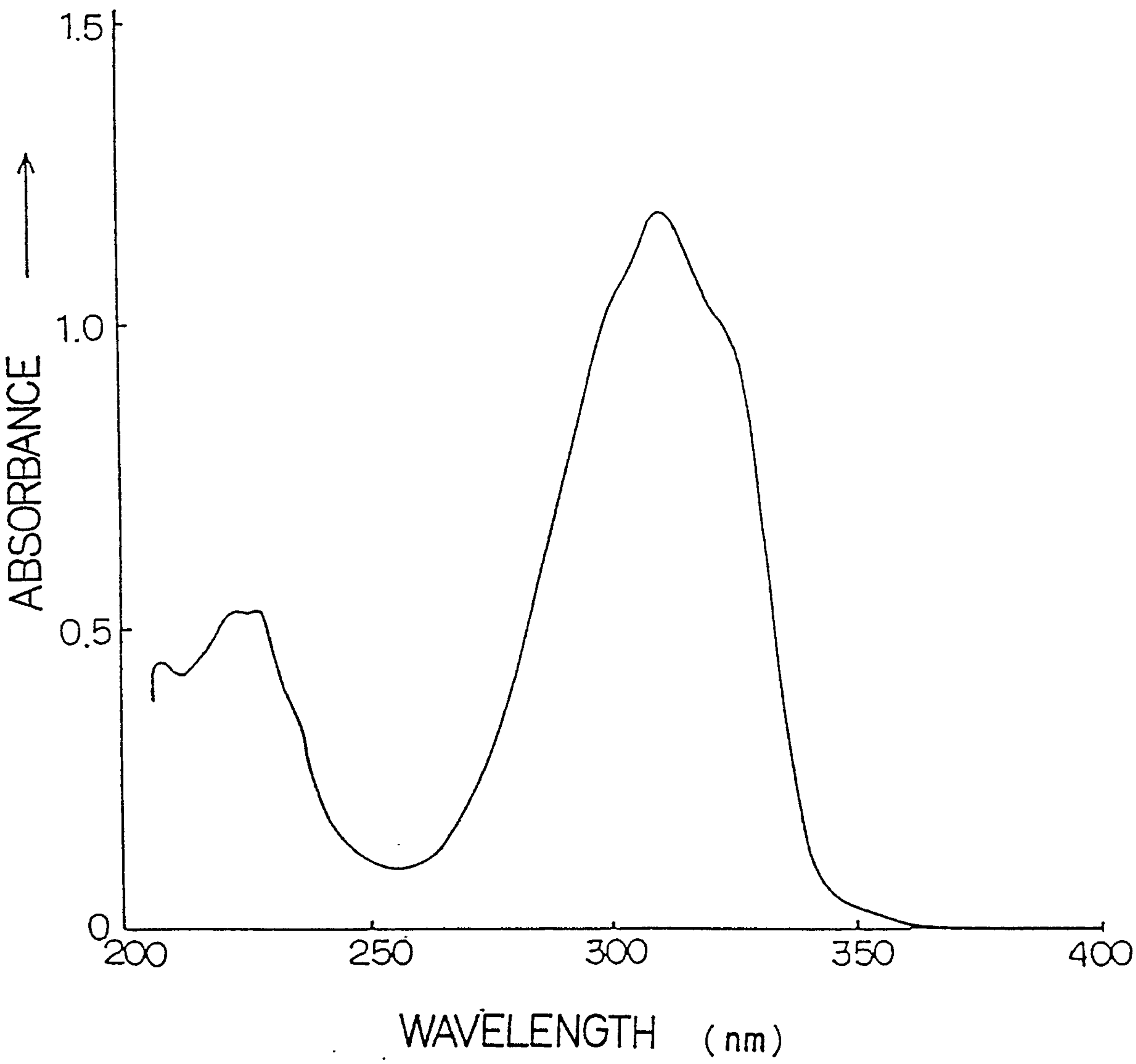

FIG. 4

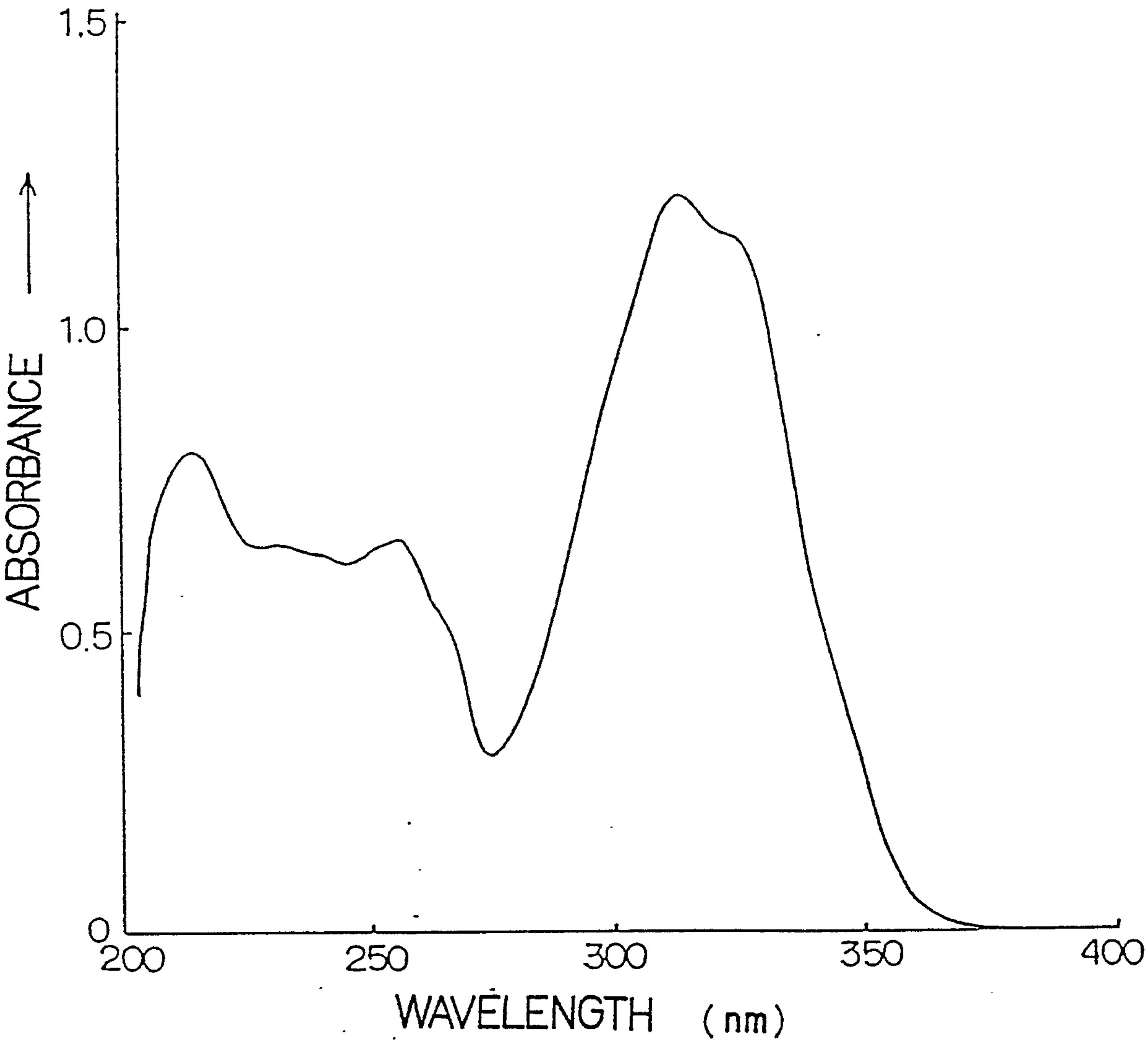

FIG. 5

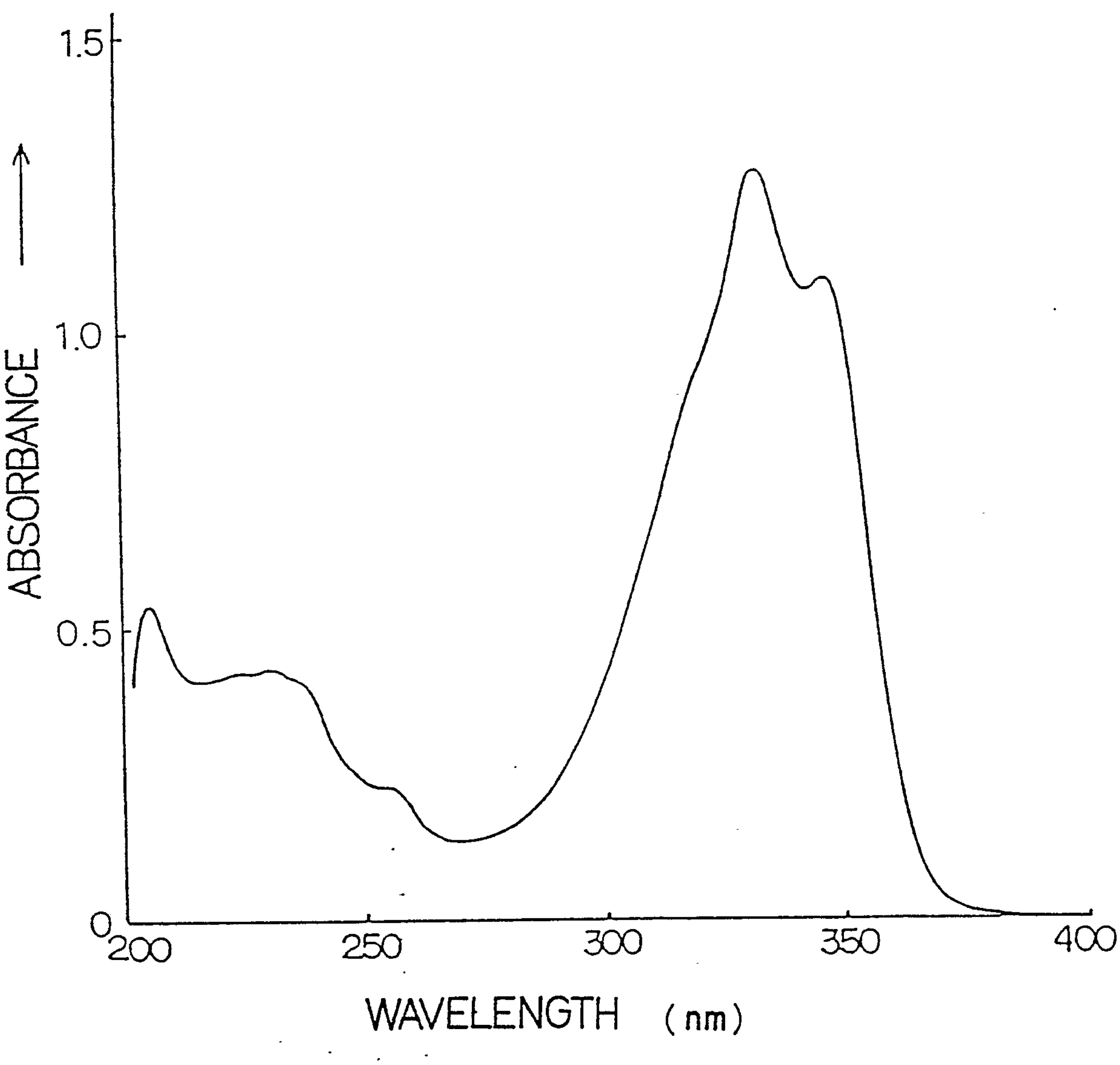

FIG. 6

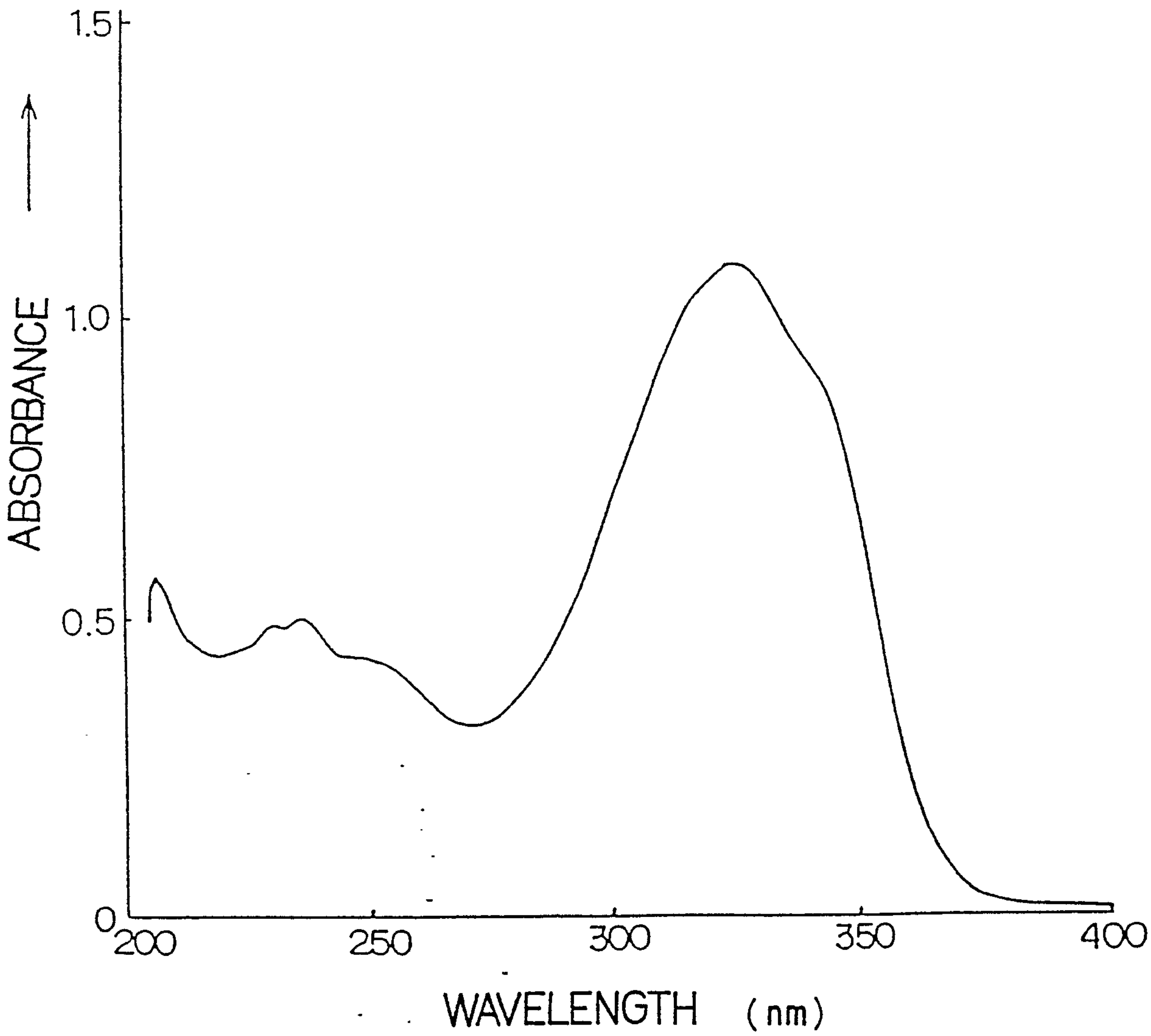

FIG. 7

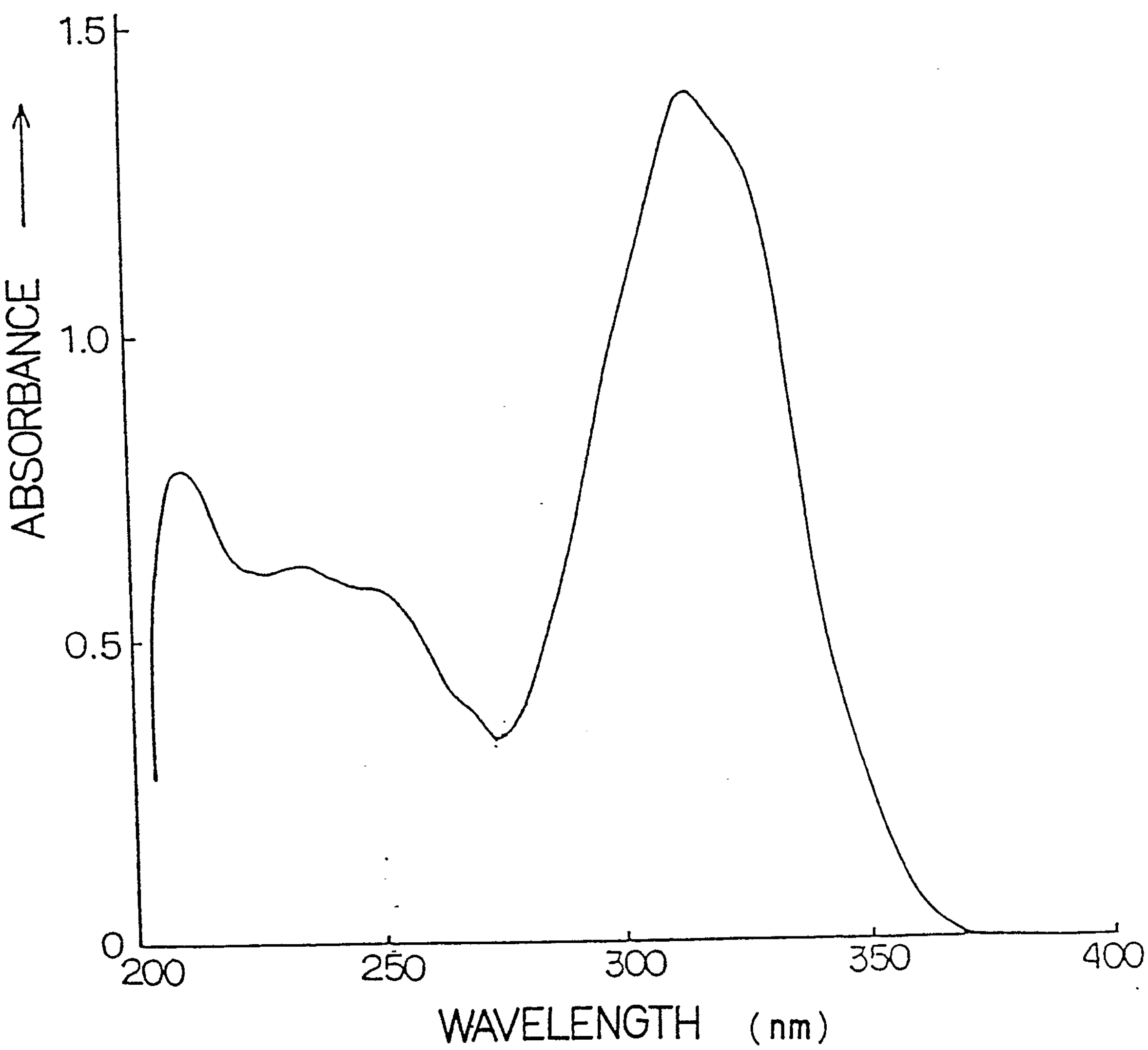

FIG. 8

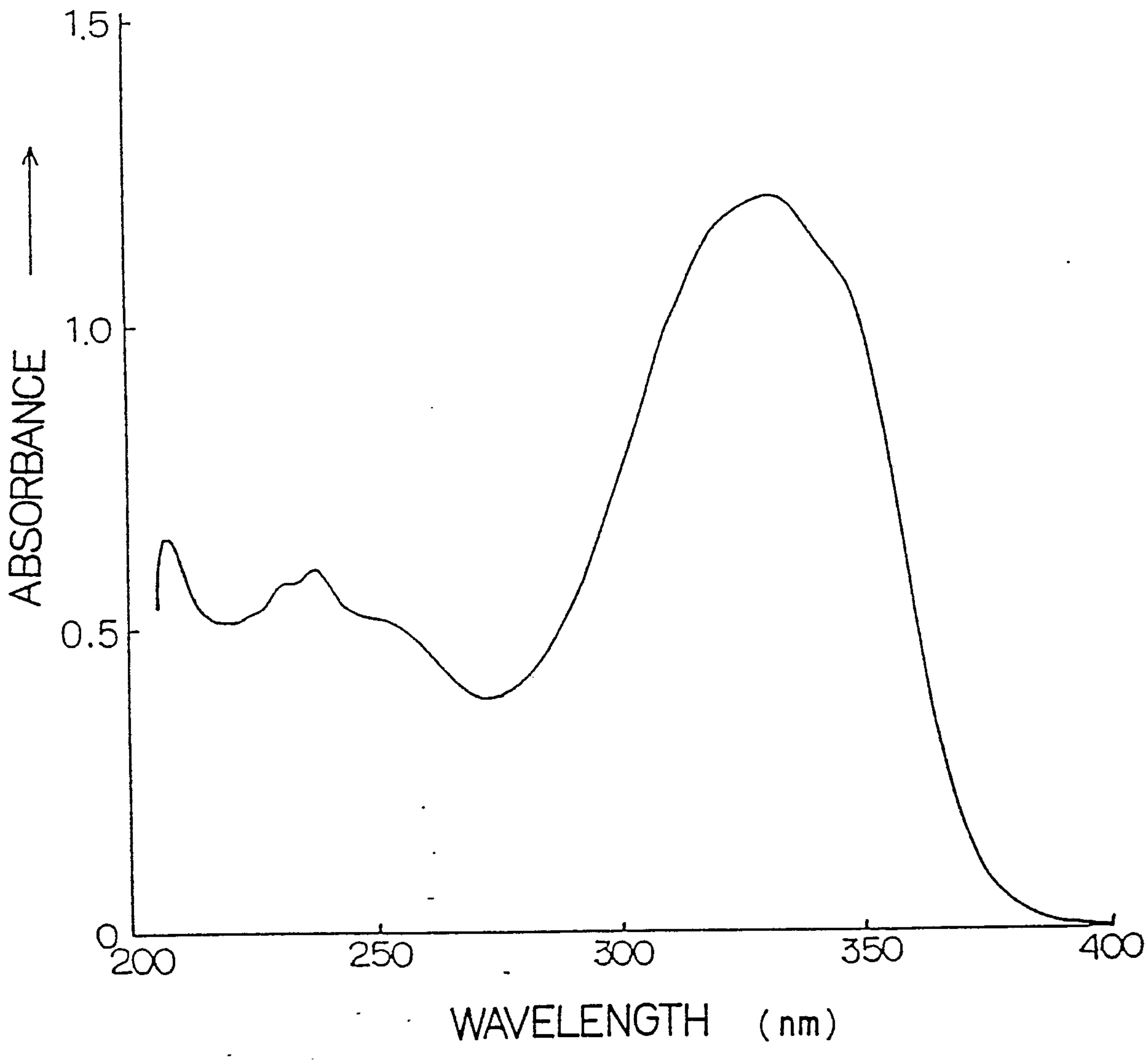

FIG. 9

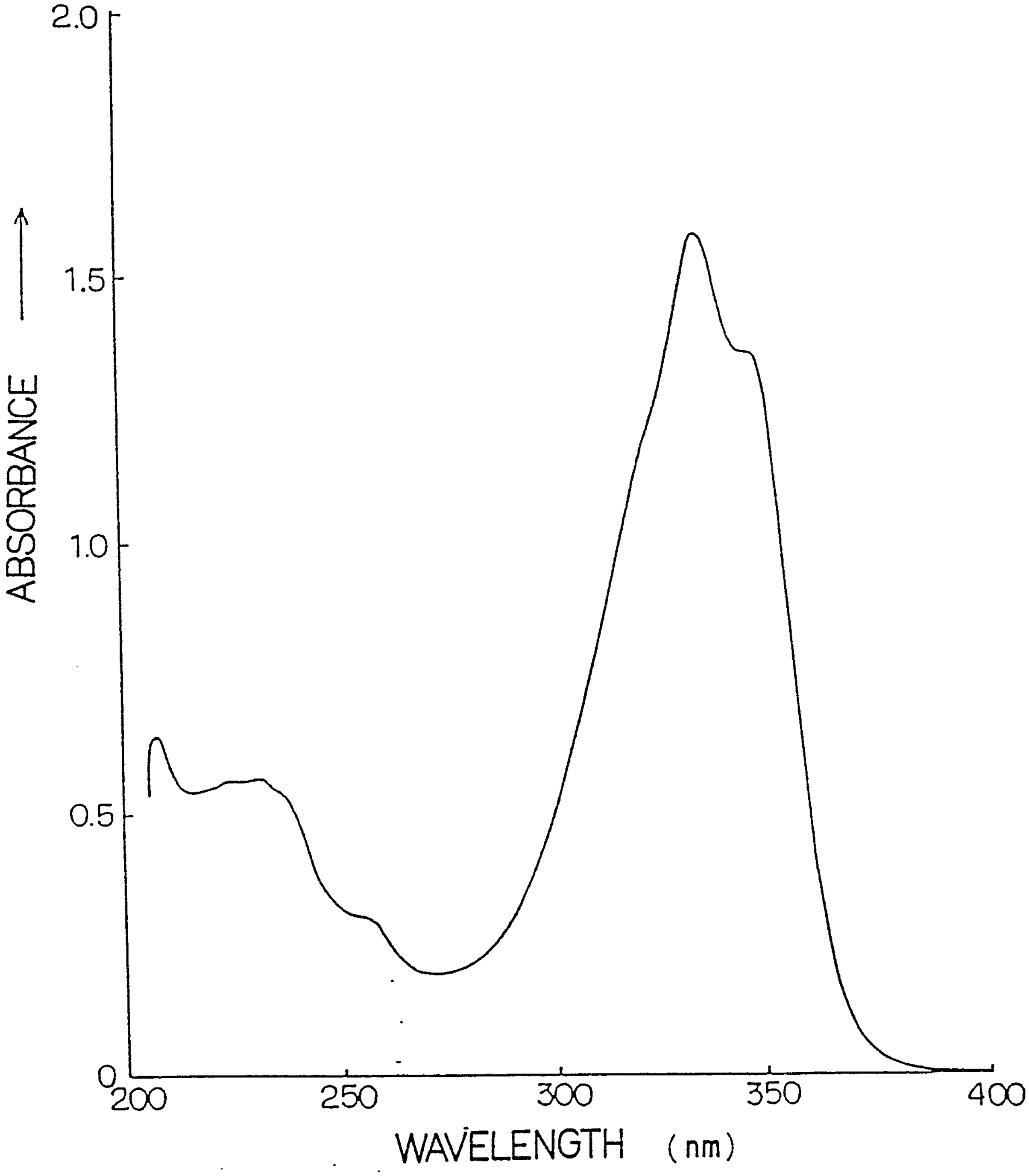

FIG. 10

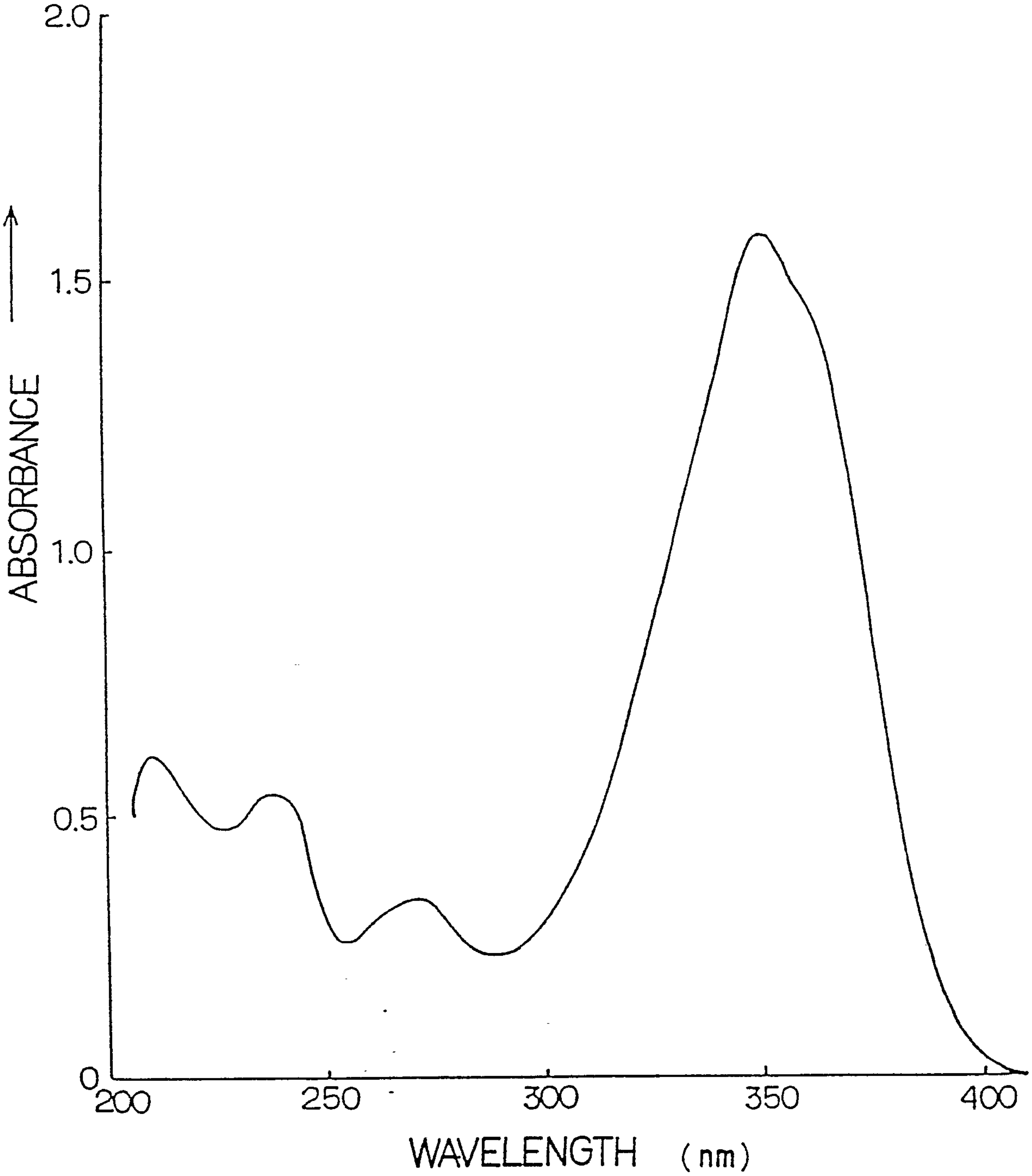

FIG. 11

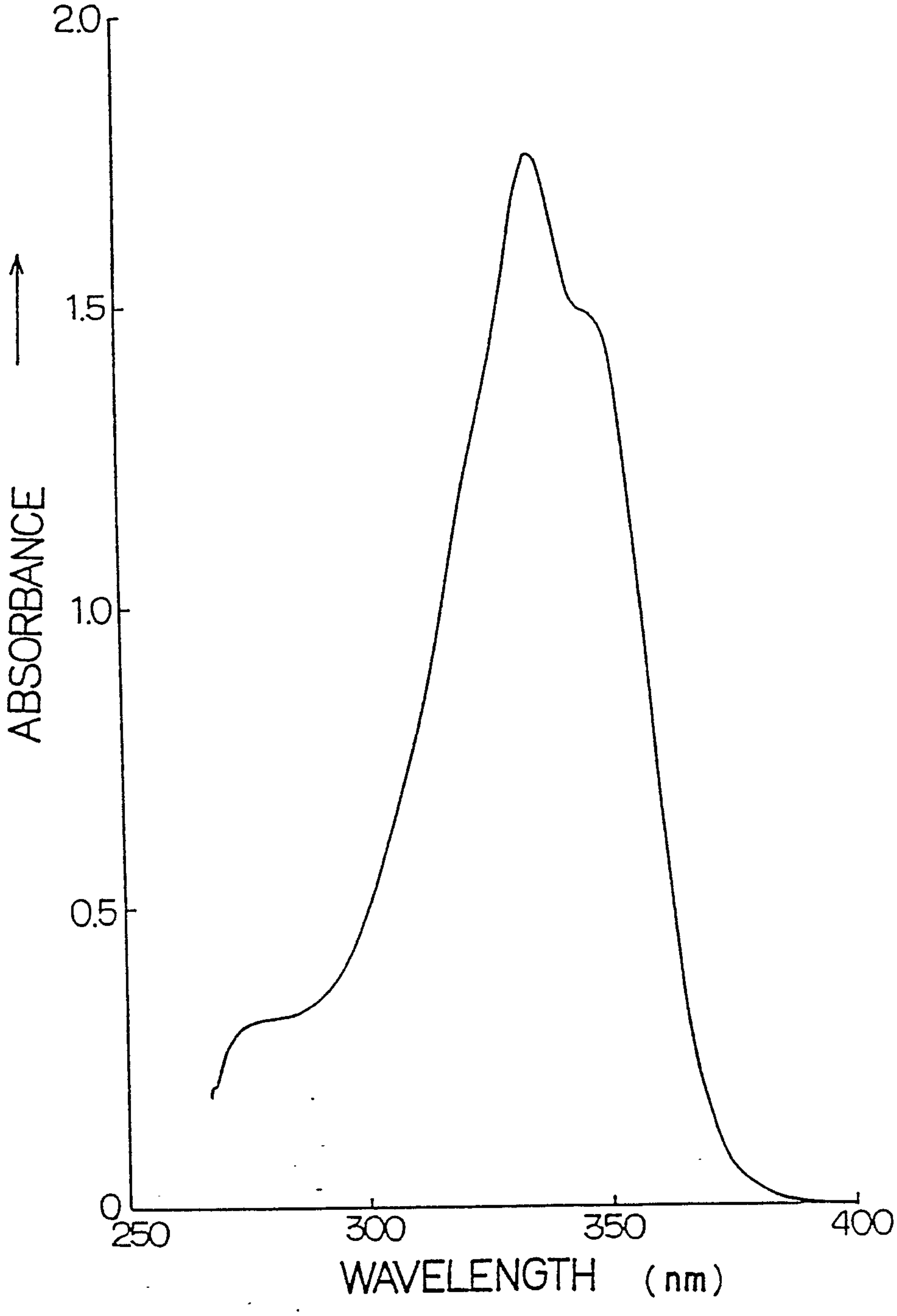

FIG. 12

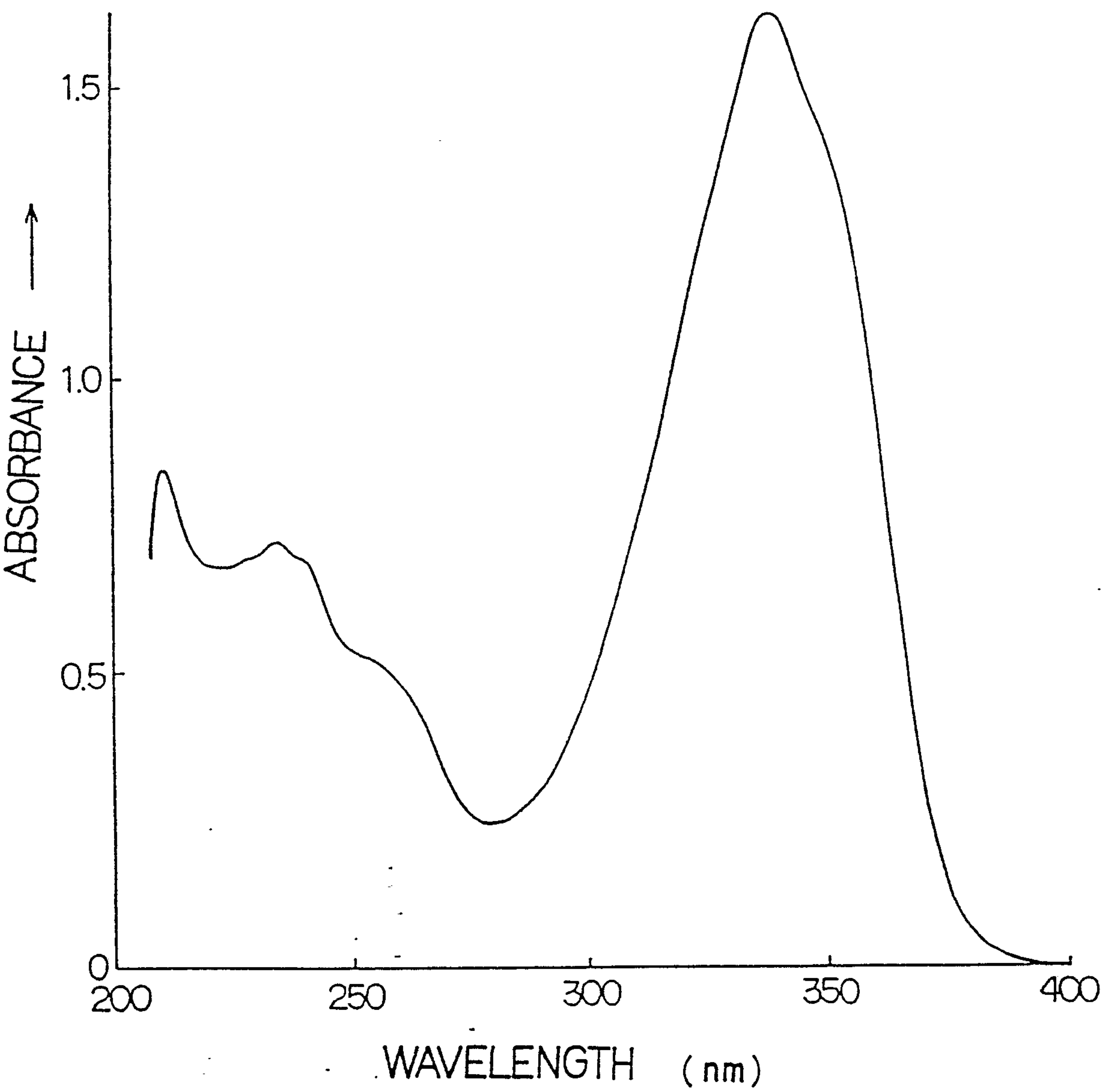

FIG. 13

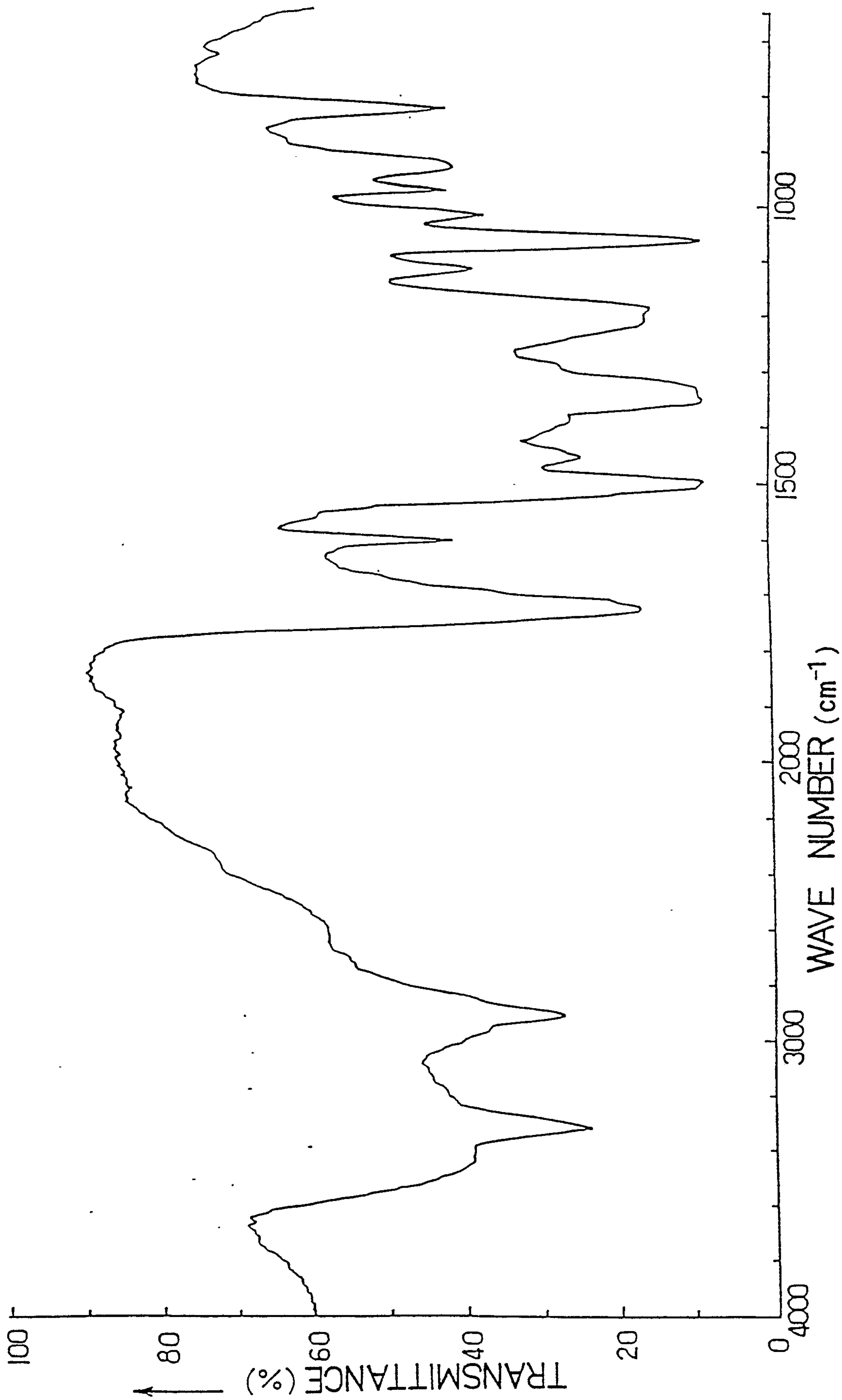

TRANSMITTANCE (%)
0
20
40
60
80
100
4000
3000
2000
1500
1000
WAVE NUMBER ($cm^{-1}$)

FIG. 14

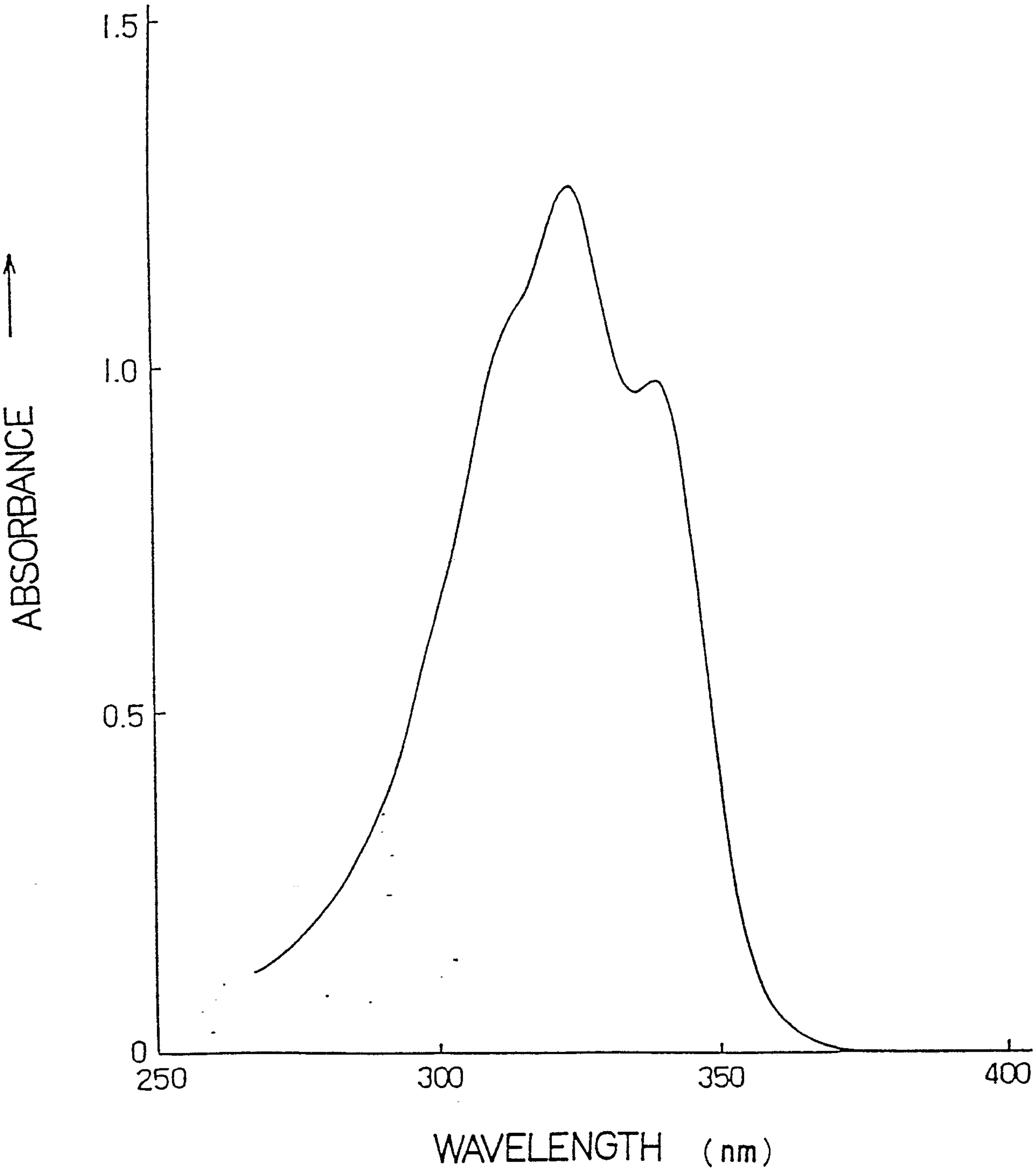

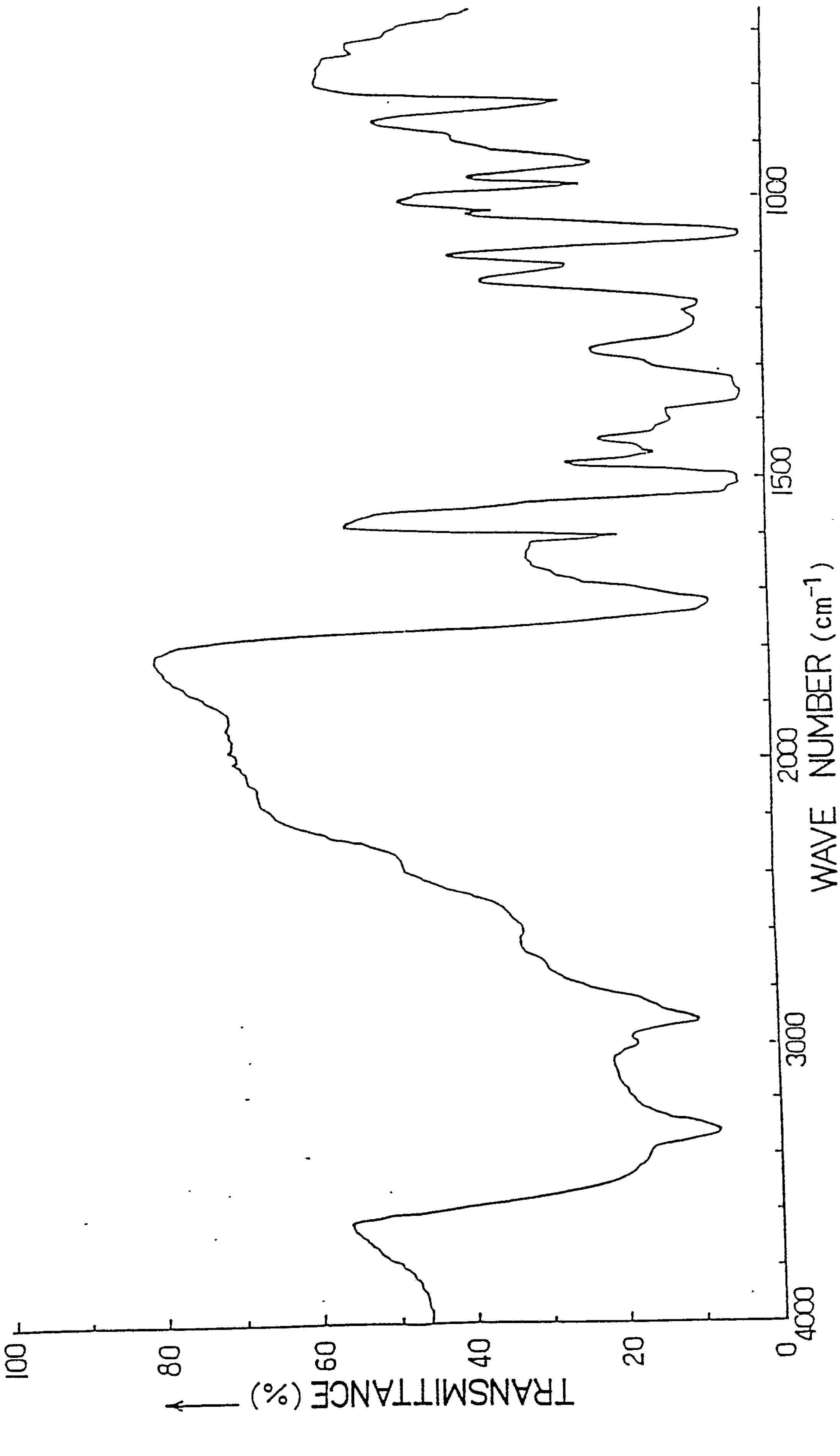
TRANSMITTANCE (%)
100
80
60
40
20
0
4000
3000
2000
1500
1000
WAVE NUMBER (cm$^{-1}$)

FIG. 15

F I G . 16

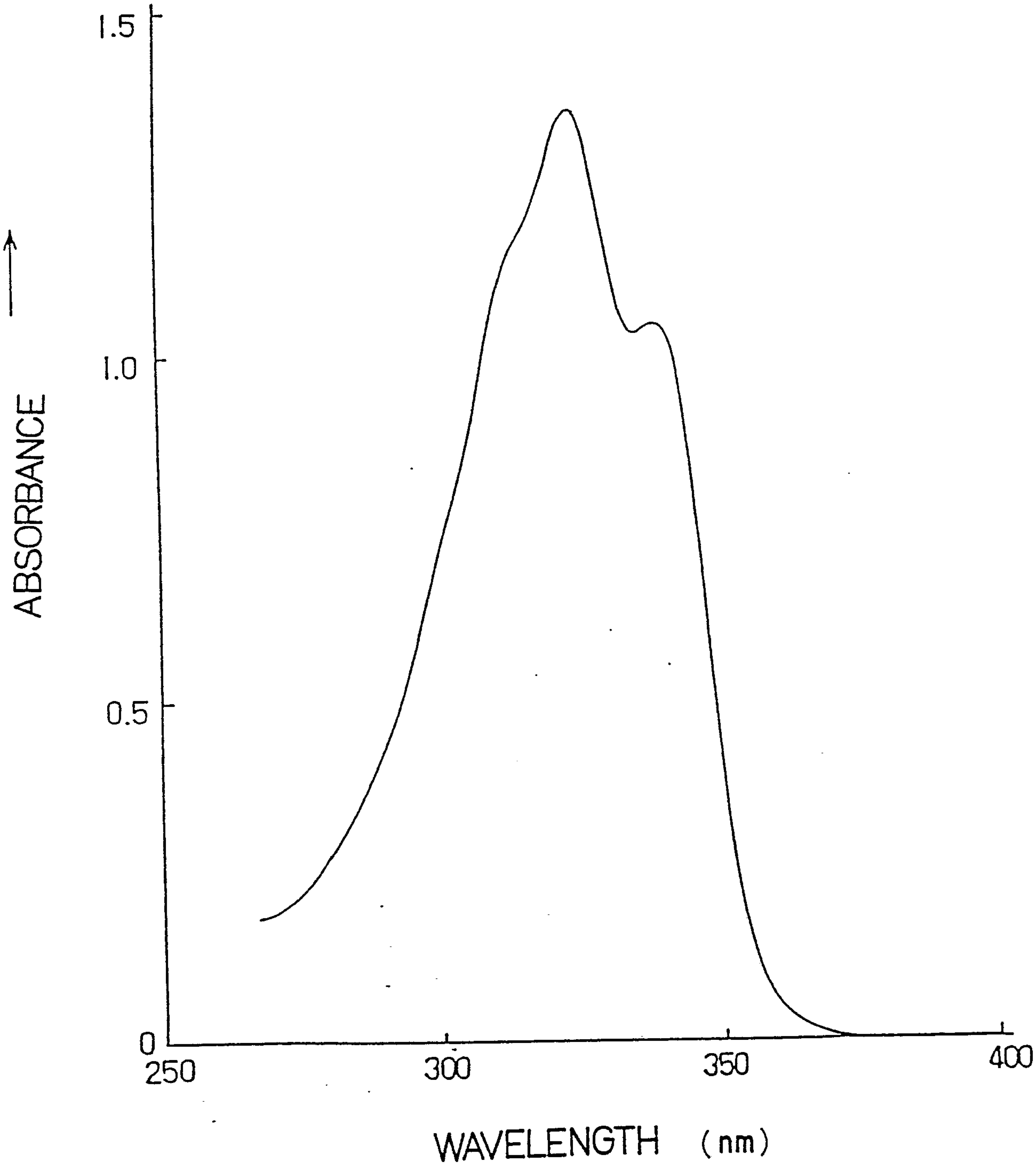

FIG. 17

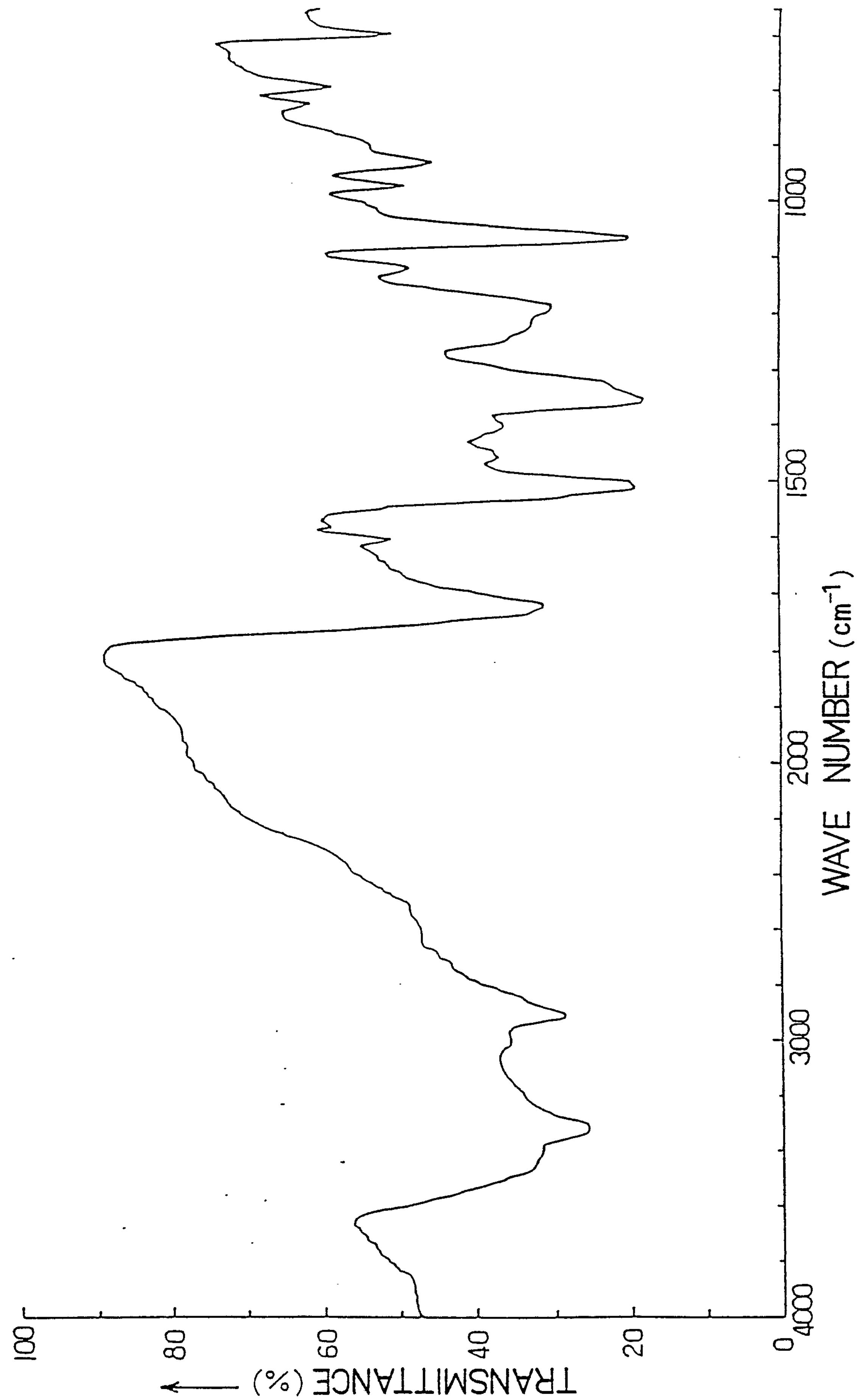
TRANSMITTANCE (%)
100
80
60
40
20
0
4000
3000
2000
1500
1000
WAVE NUMBER (cm$^{-1}$)

FIG. 18

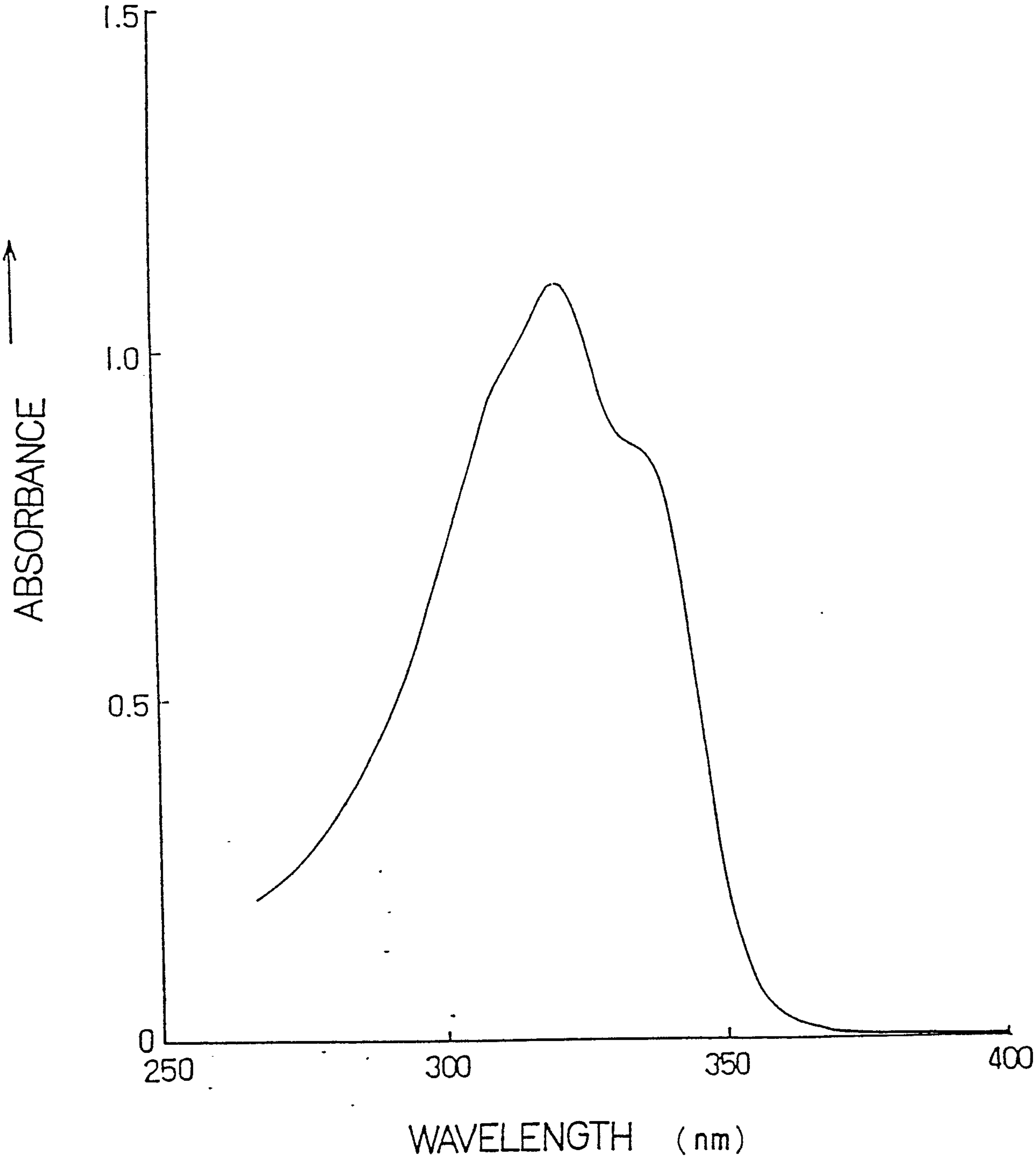

FIG. 19

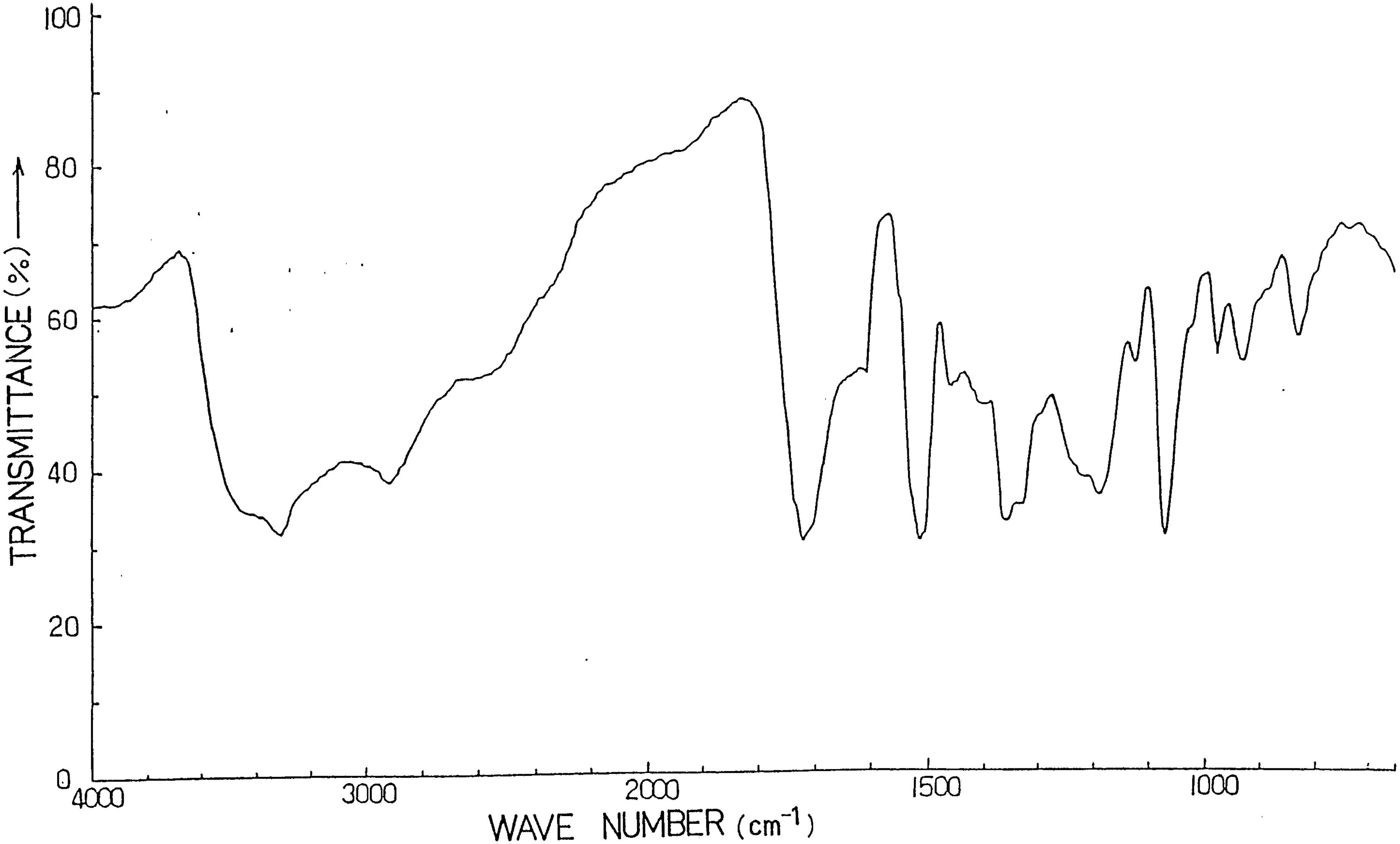
100
80
60
40
20
0
TRANSMITTANCE (%)
4000
3000
2000
1500
1000
WAVE NUMBER (cm⁻¹)

FIG. 20

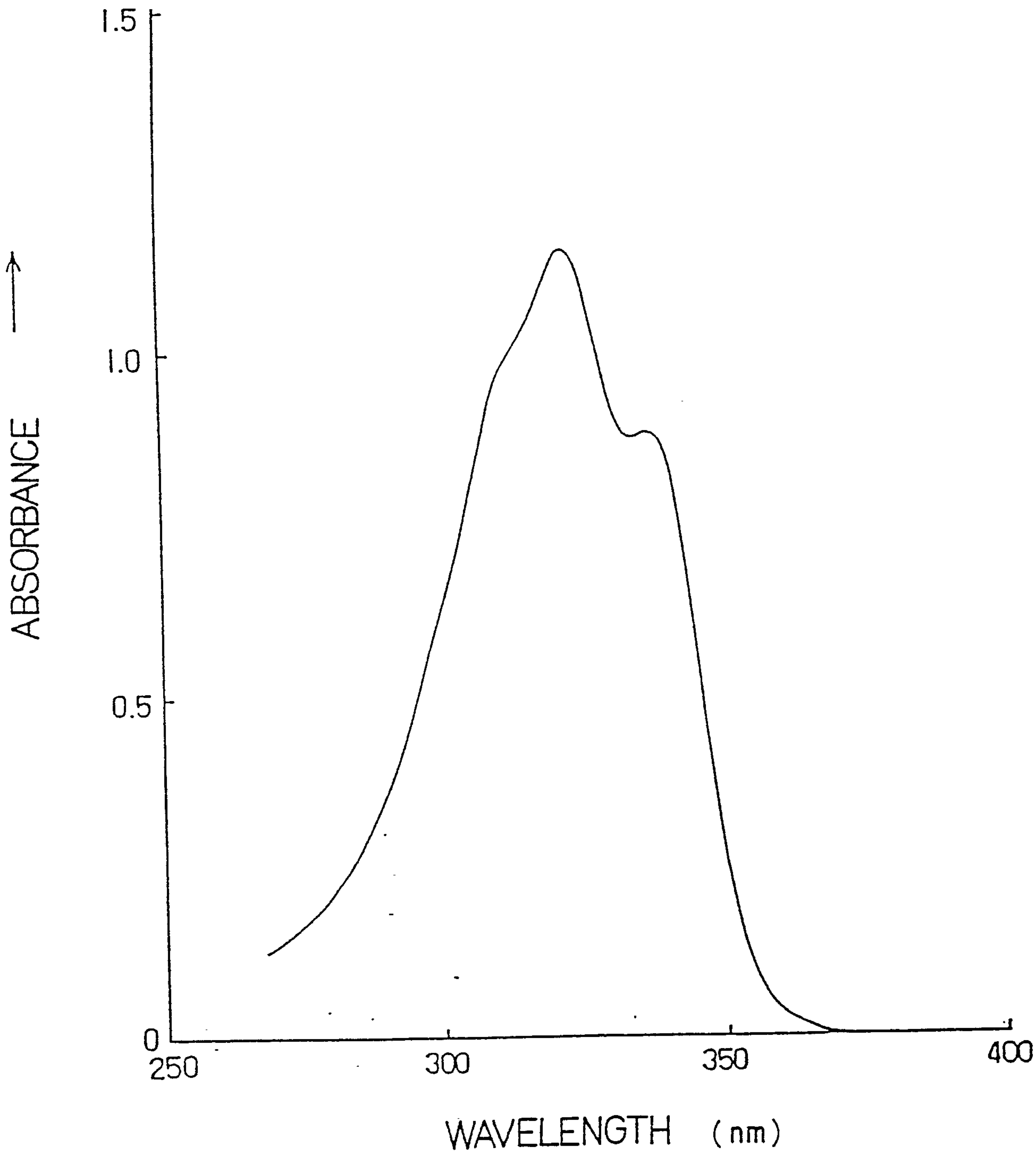

FIG.21

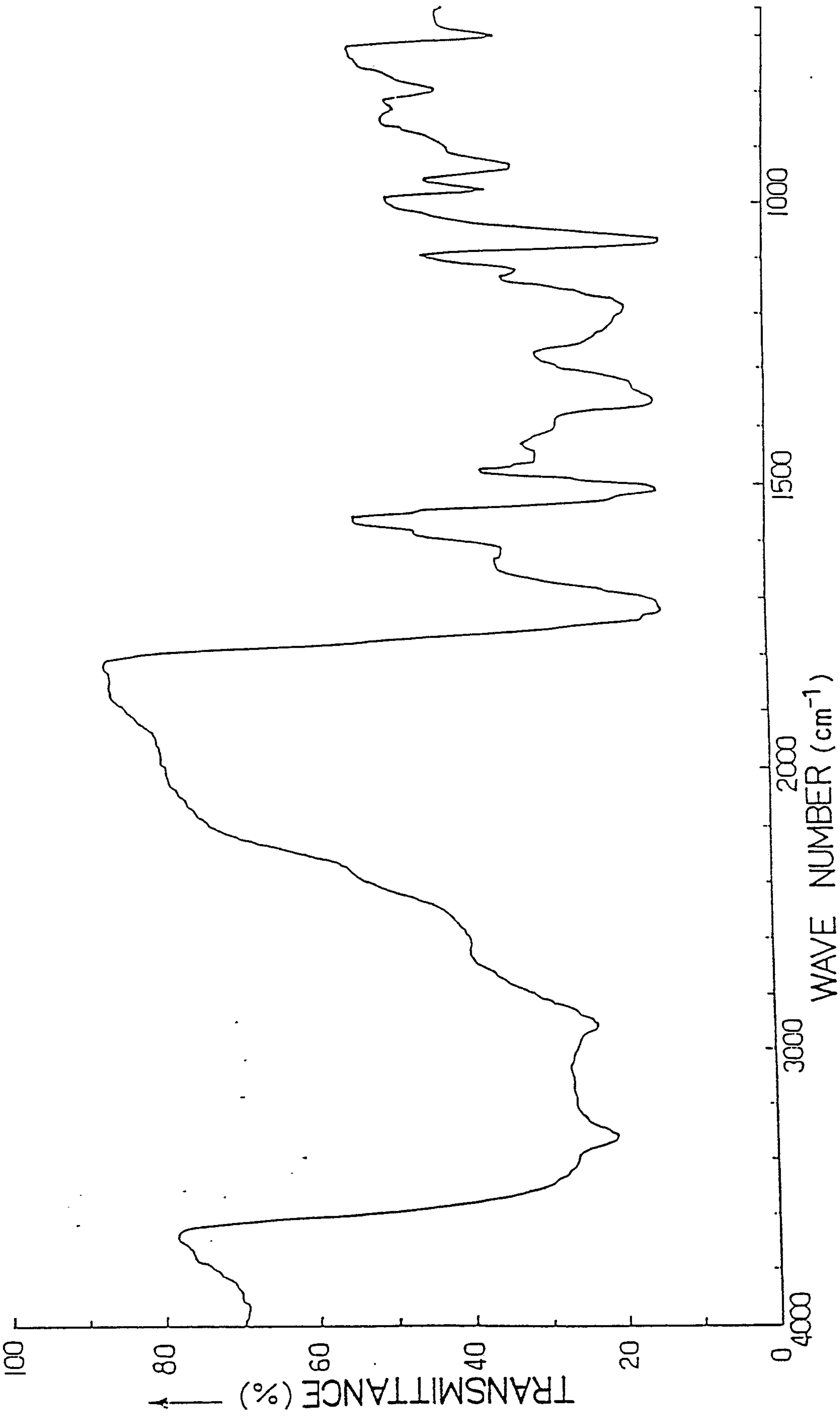
TRANSMITTANCE (%)
100
80
60
40
20
0
4000
3000
2000
1500
1000
WAVE NUMBER (cm$^{-1}$)

FIG. 22

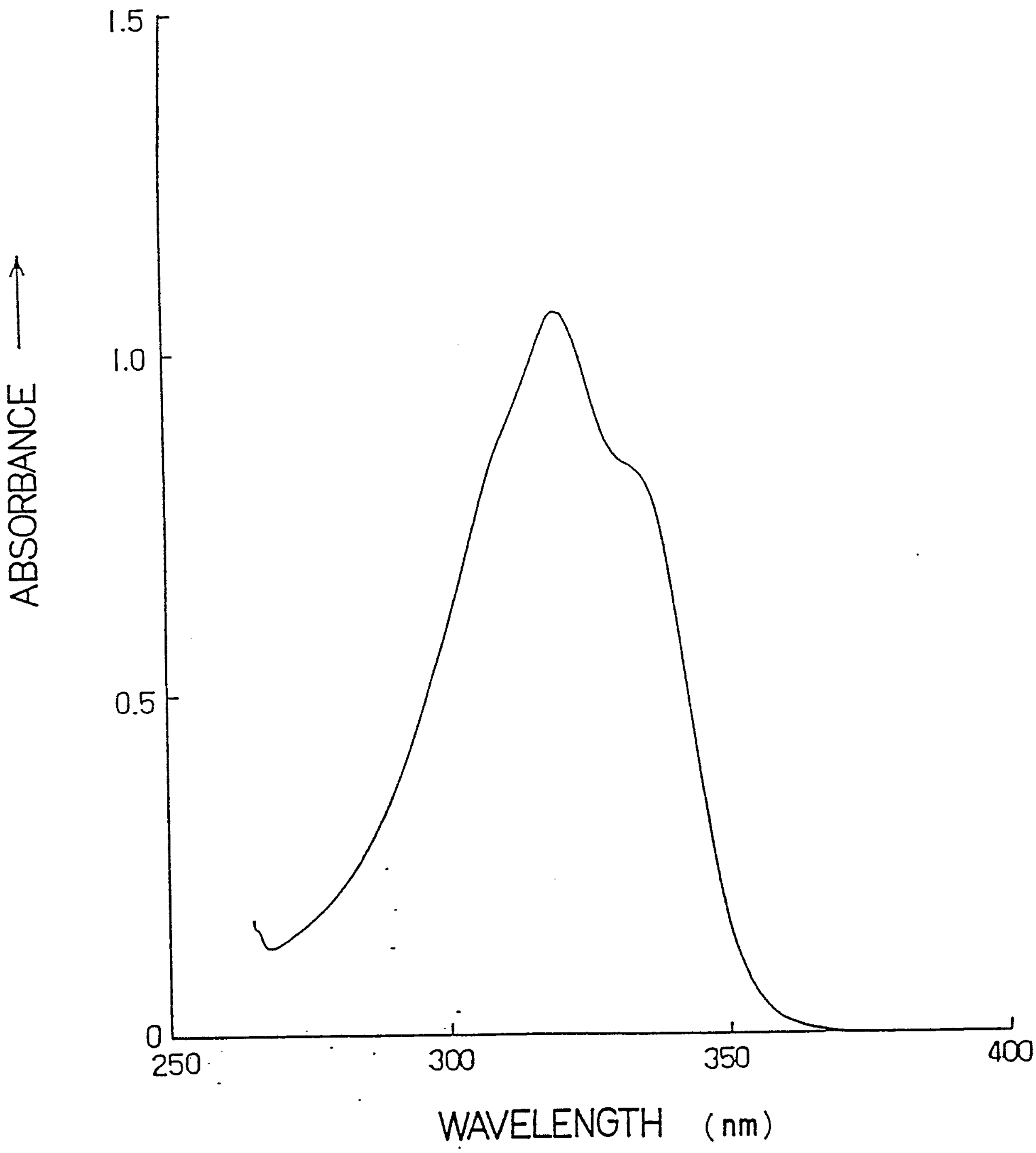

FIG.23

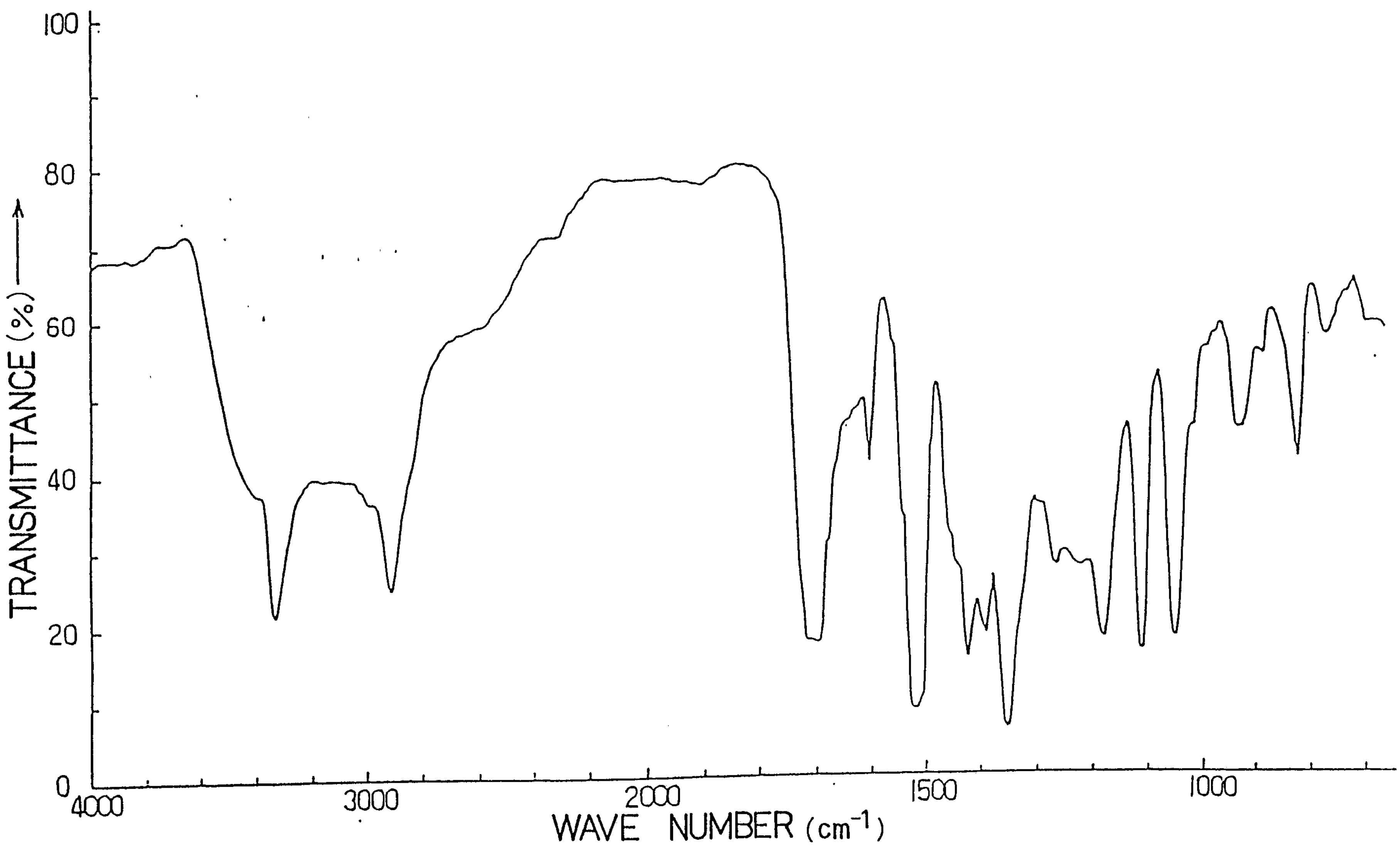
TRANSMITTANCE (%)
100
80
60
40
20
0
4000
3000
2000
1500
1000
WAVE NUMBER (cm$^{-1}$)

FIG.24

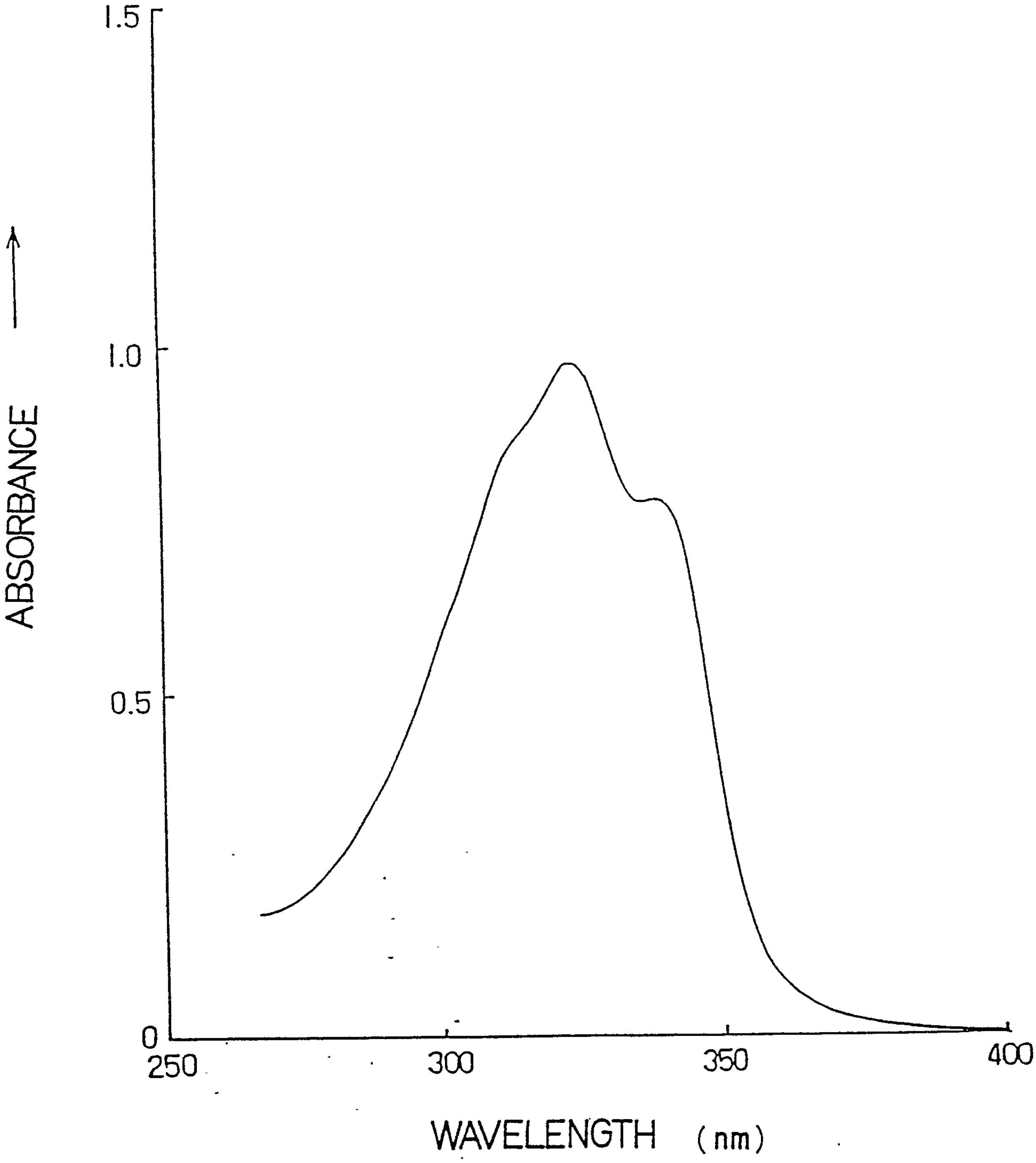

FIG.25

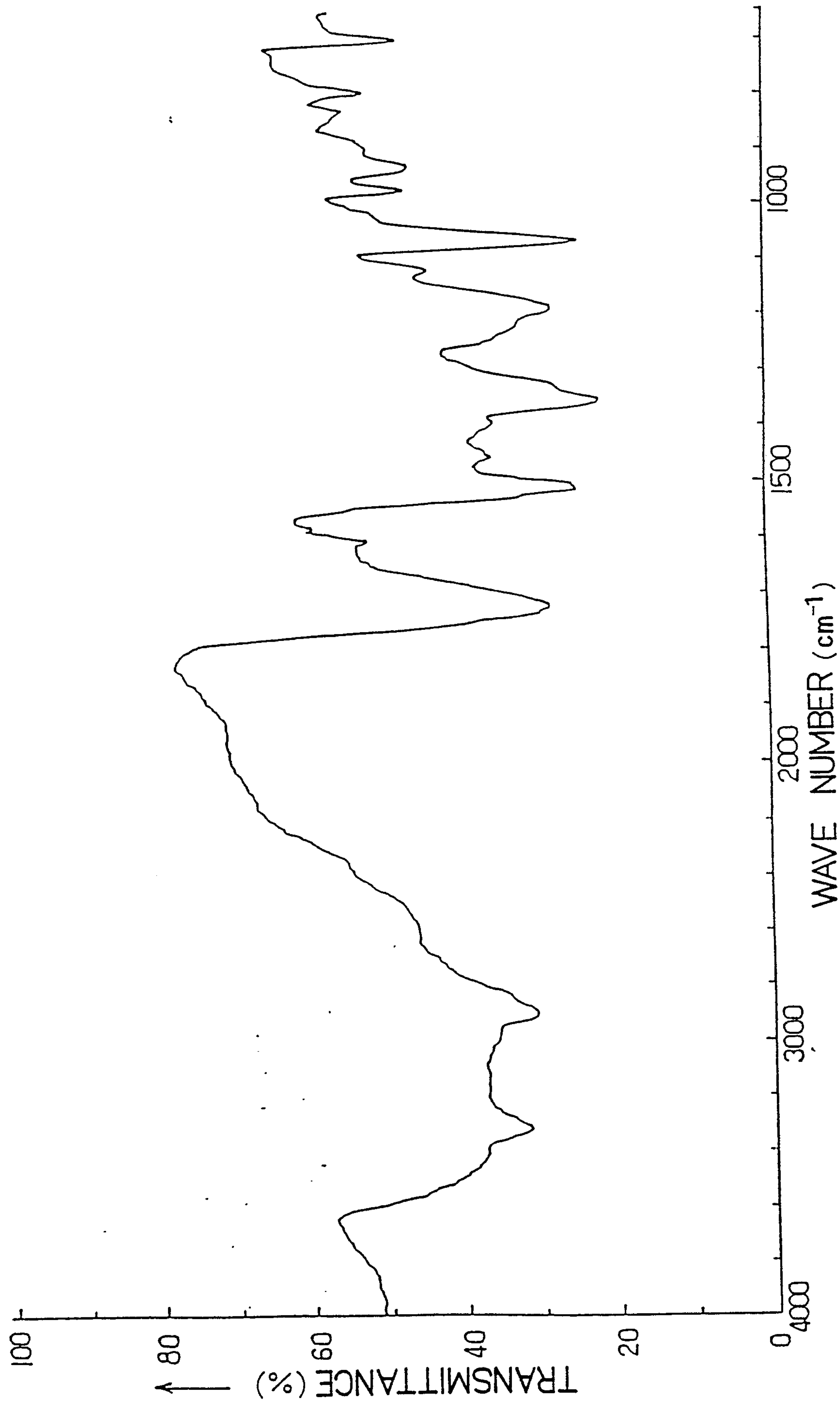
TRANSMITTANCE (%)
100
80
60
40
20
0
4000
3000
2000
1500
1000
WAVE NUMBER (cm$^{-1}$)

FIG.26

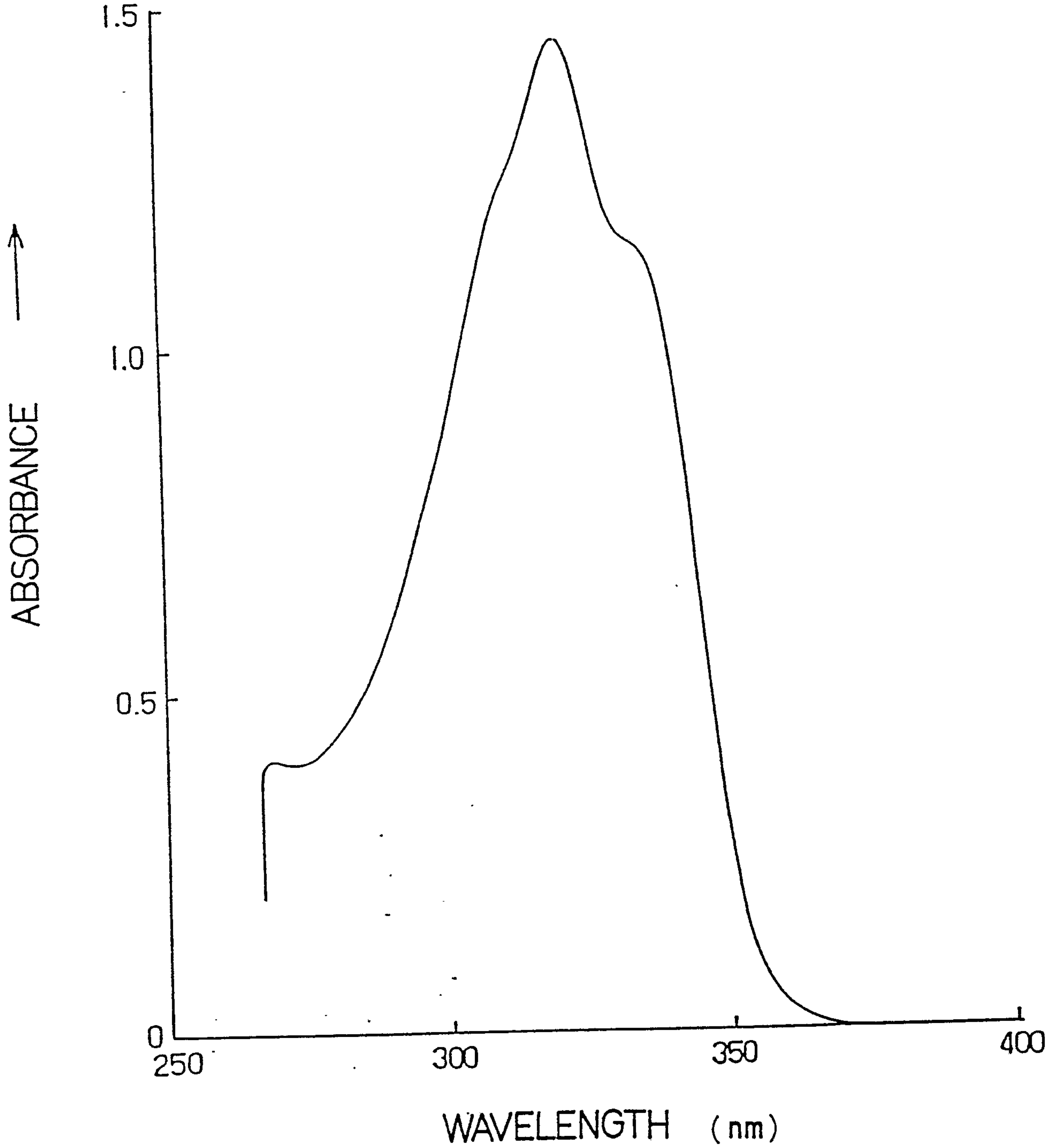

FIG. 27

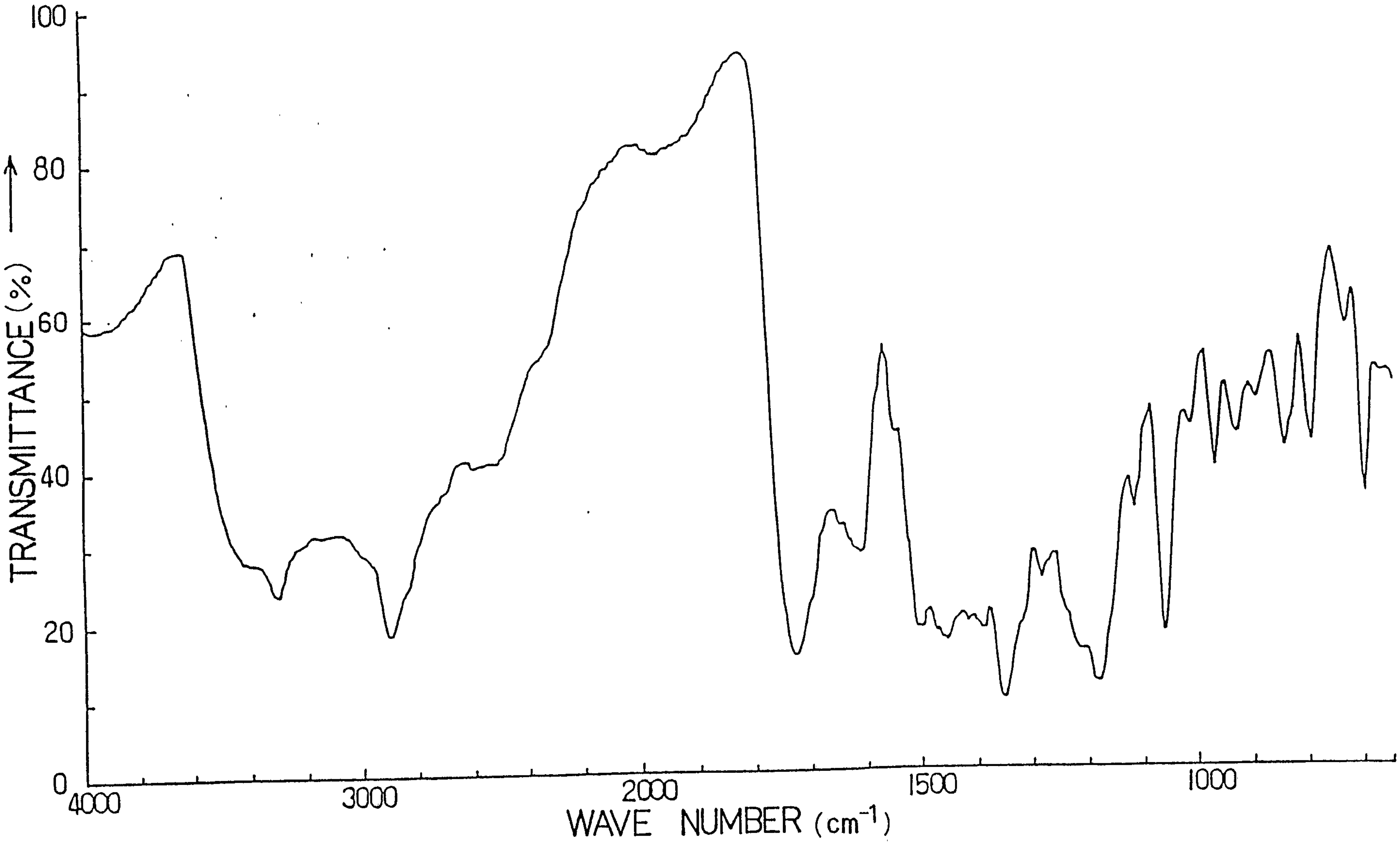
TRANSMITTANCE (%)
100
80
60
40
20
0
4000
3000
2000
1500
1000
WAVE NUMBER ($cm^{-1}$)

FIG. 28

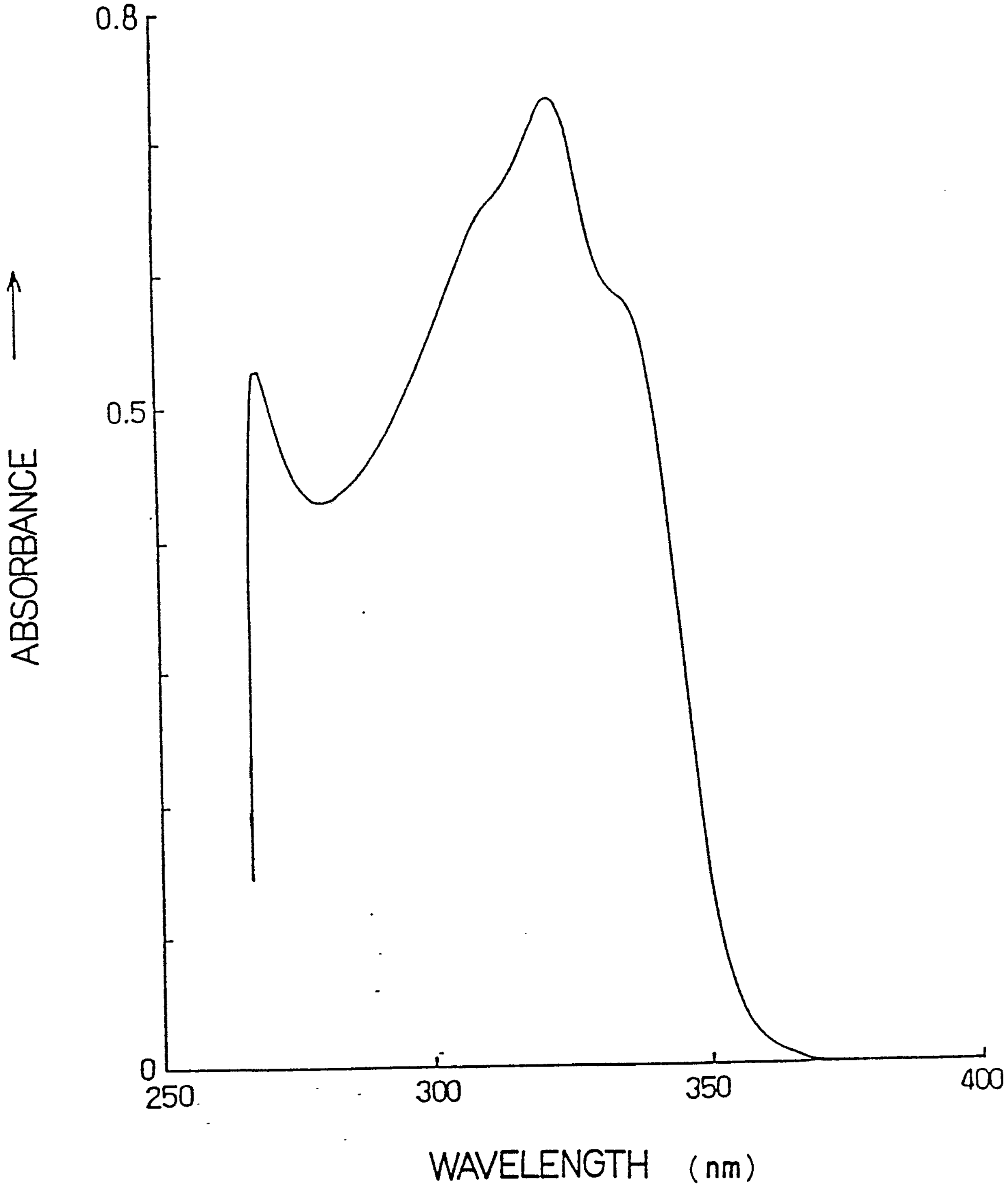

FIG.29

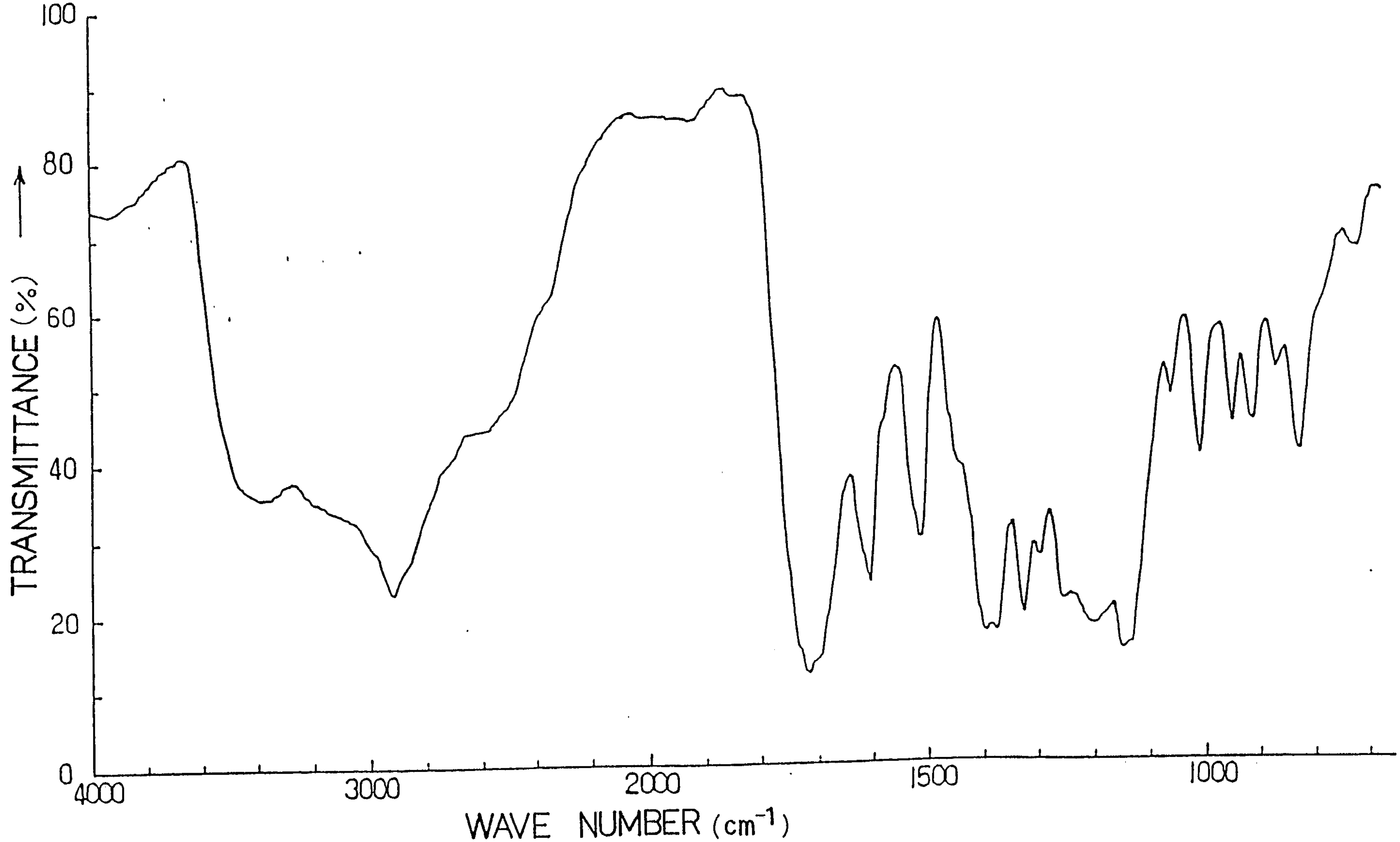
TRANSMITTANCE (%)
100
80
60
40
20
0
4000
3000
2000
1500
1000
WAVE NUMBER (cm$^{-1}$)

FIG. 30

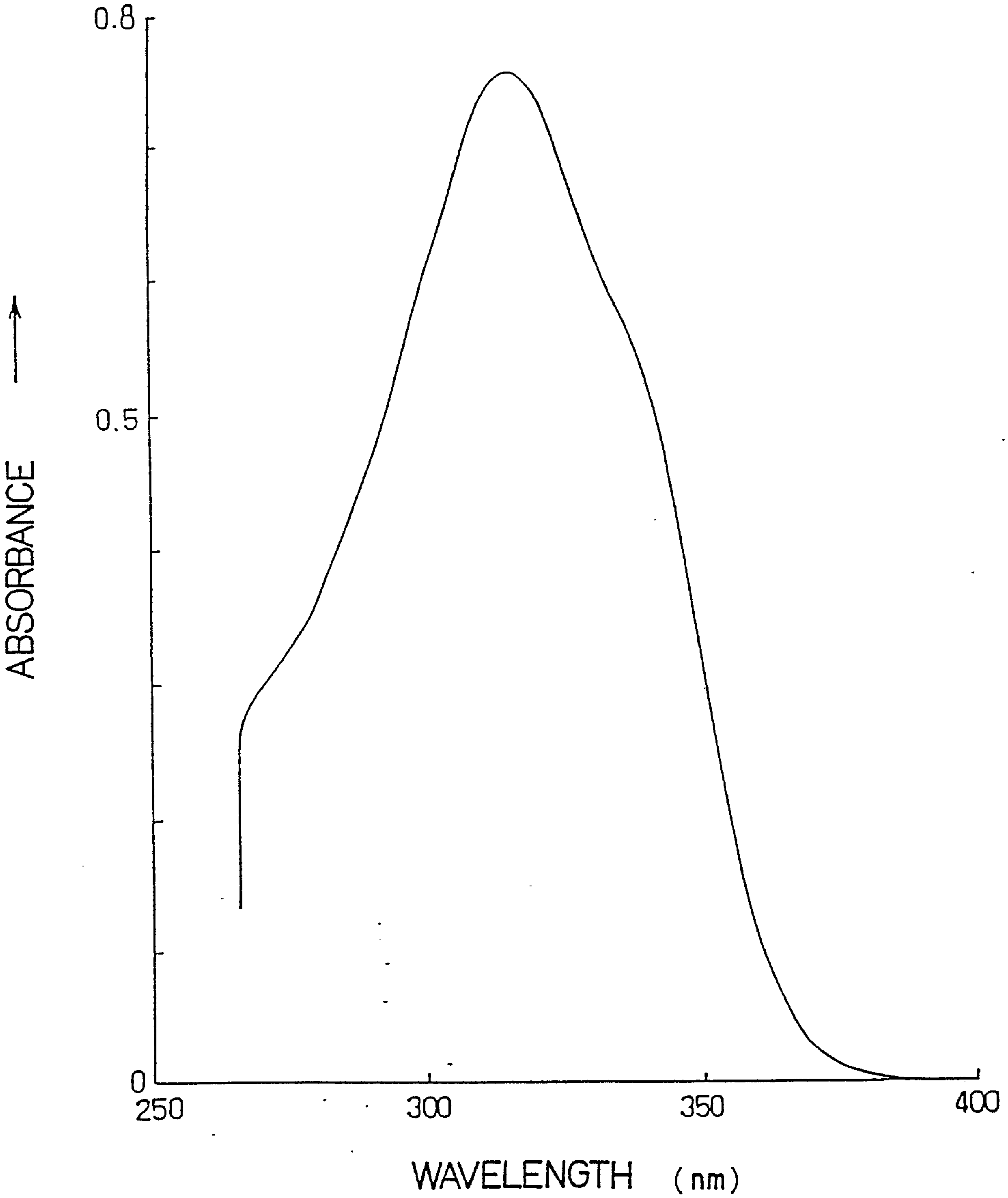

FIG. 31

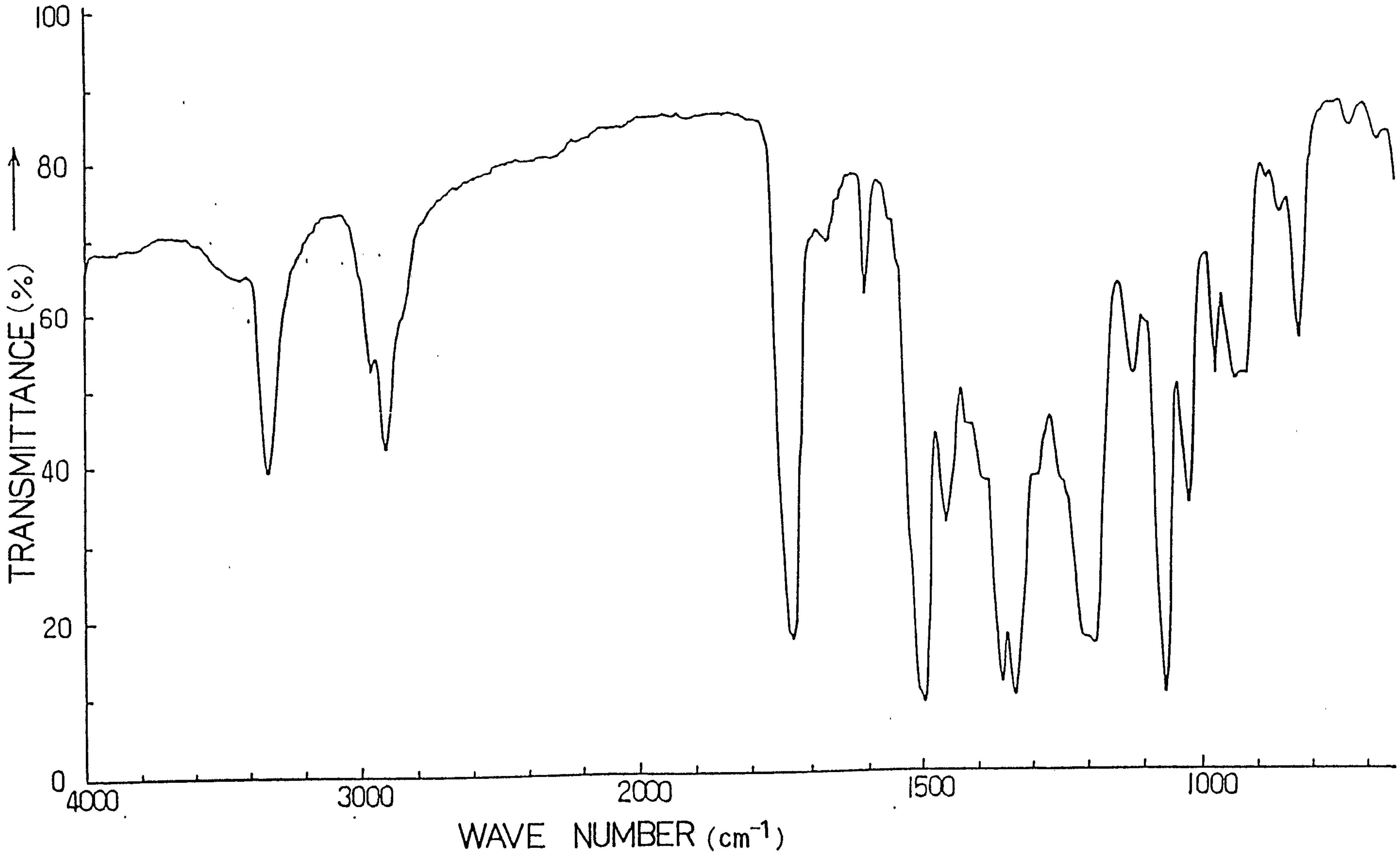
TRANSMITTANCE (%)
100
80
60
40
20
0
4000
3000
2000
1500
1000
WAVE NUMBER (cm$^{-1}$)

FIG. 32

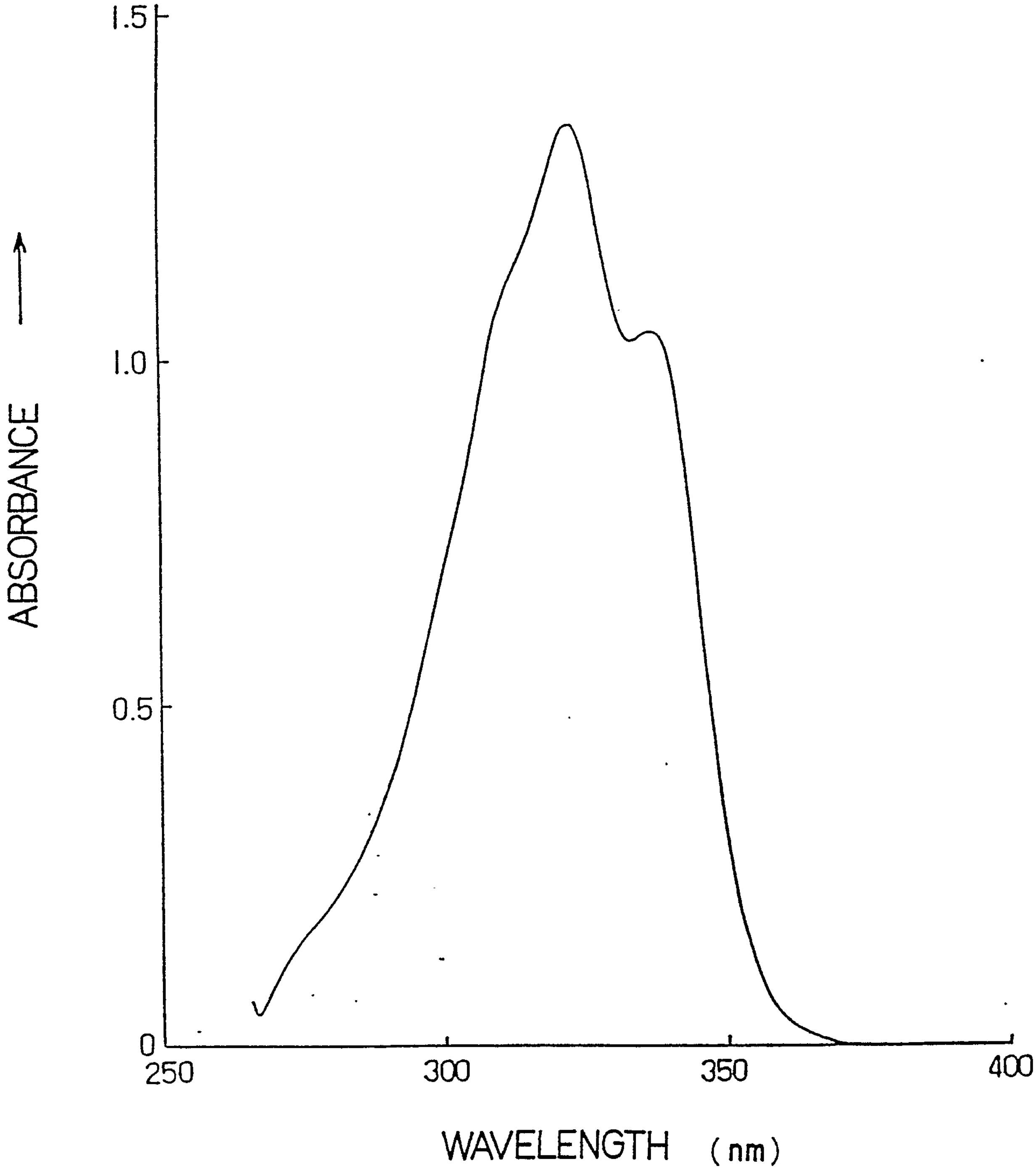

FIG. 33

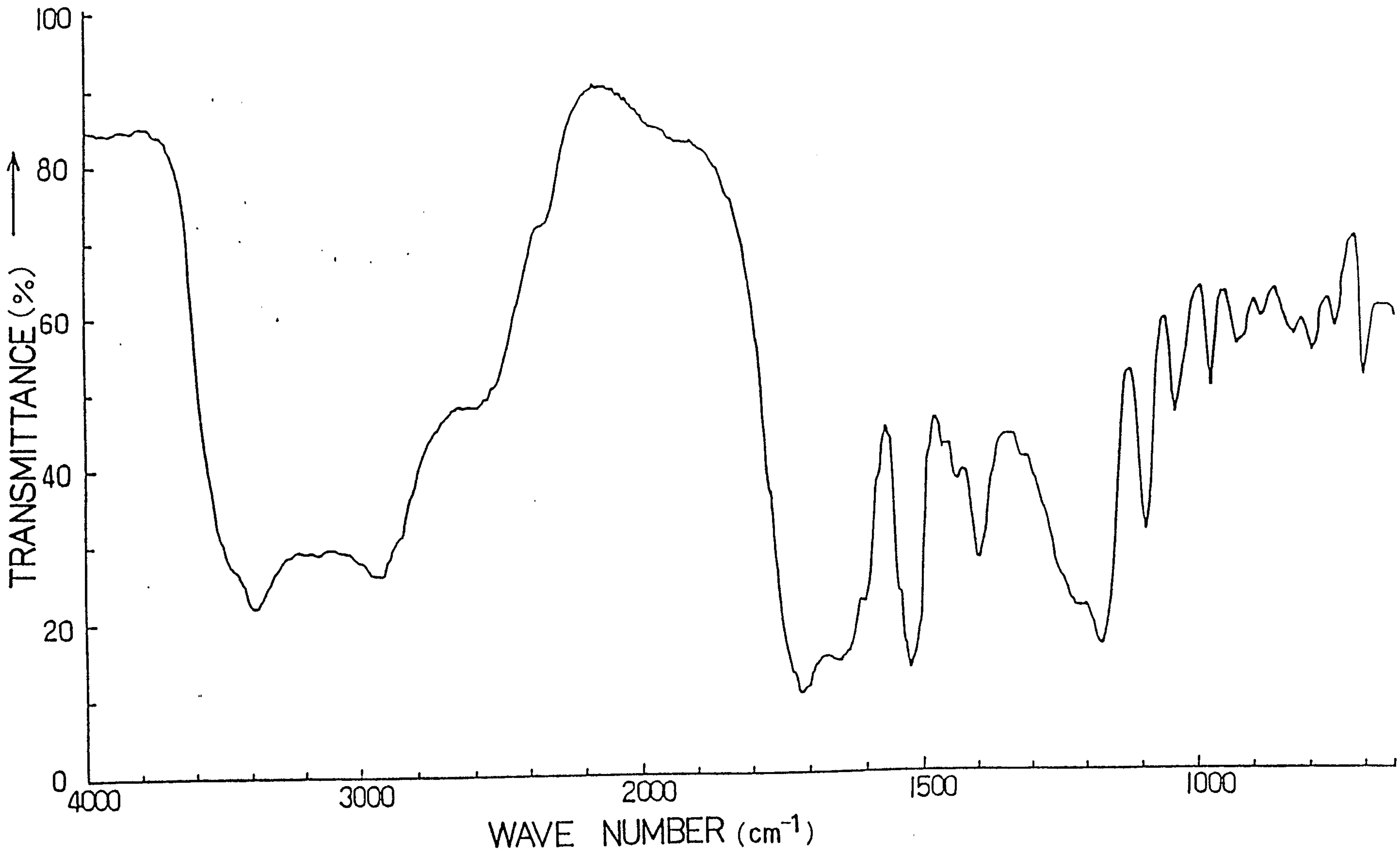
100
80
60
40
20
0
TRANSMITTANCE (%)
4000
3000
2000
1500
1000
WAVE NUMBER ($cm^{-1}$)

FIG. 34

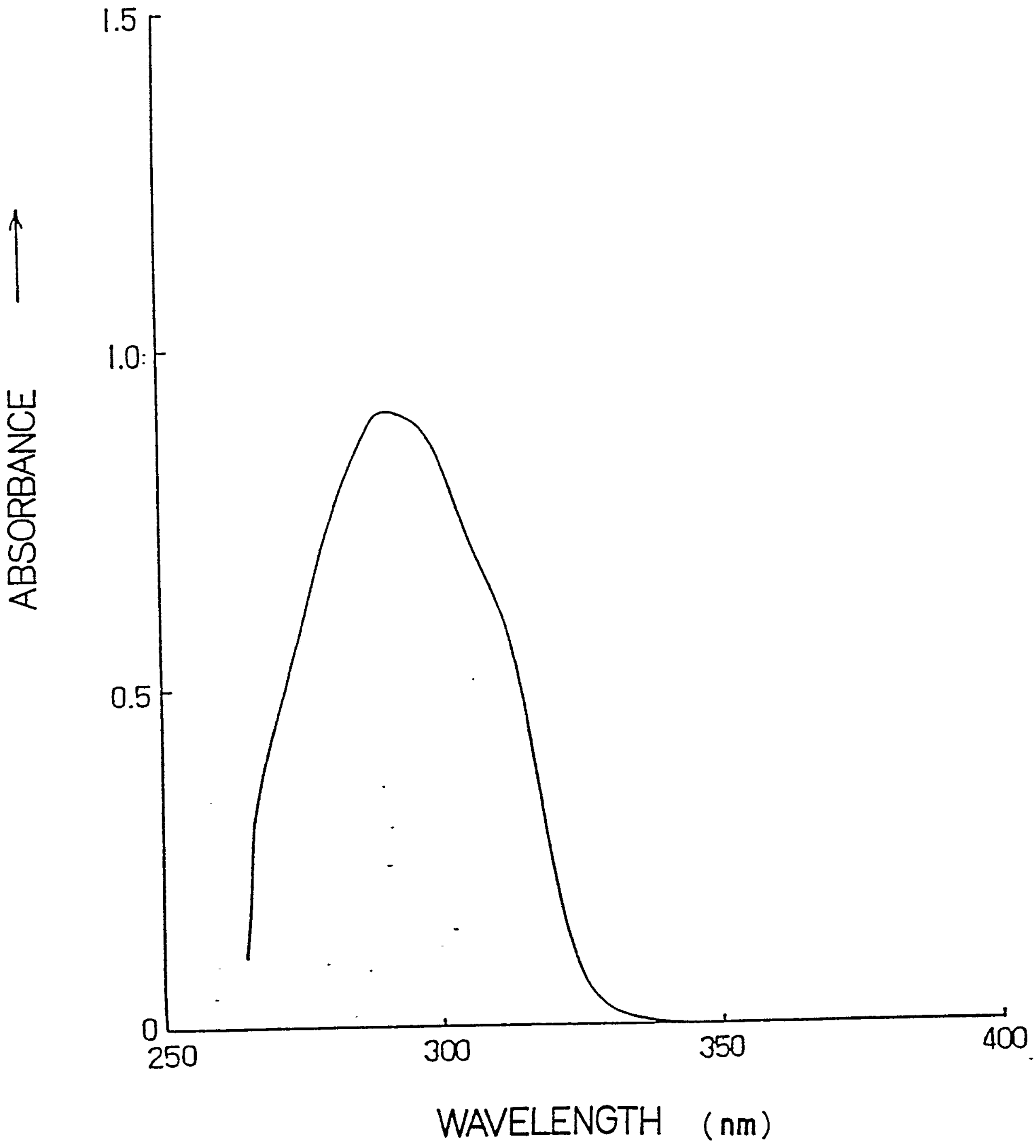

FIG. 35

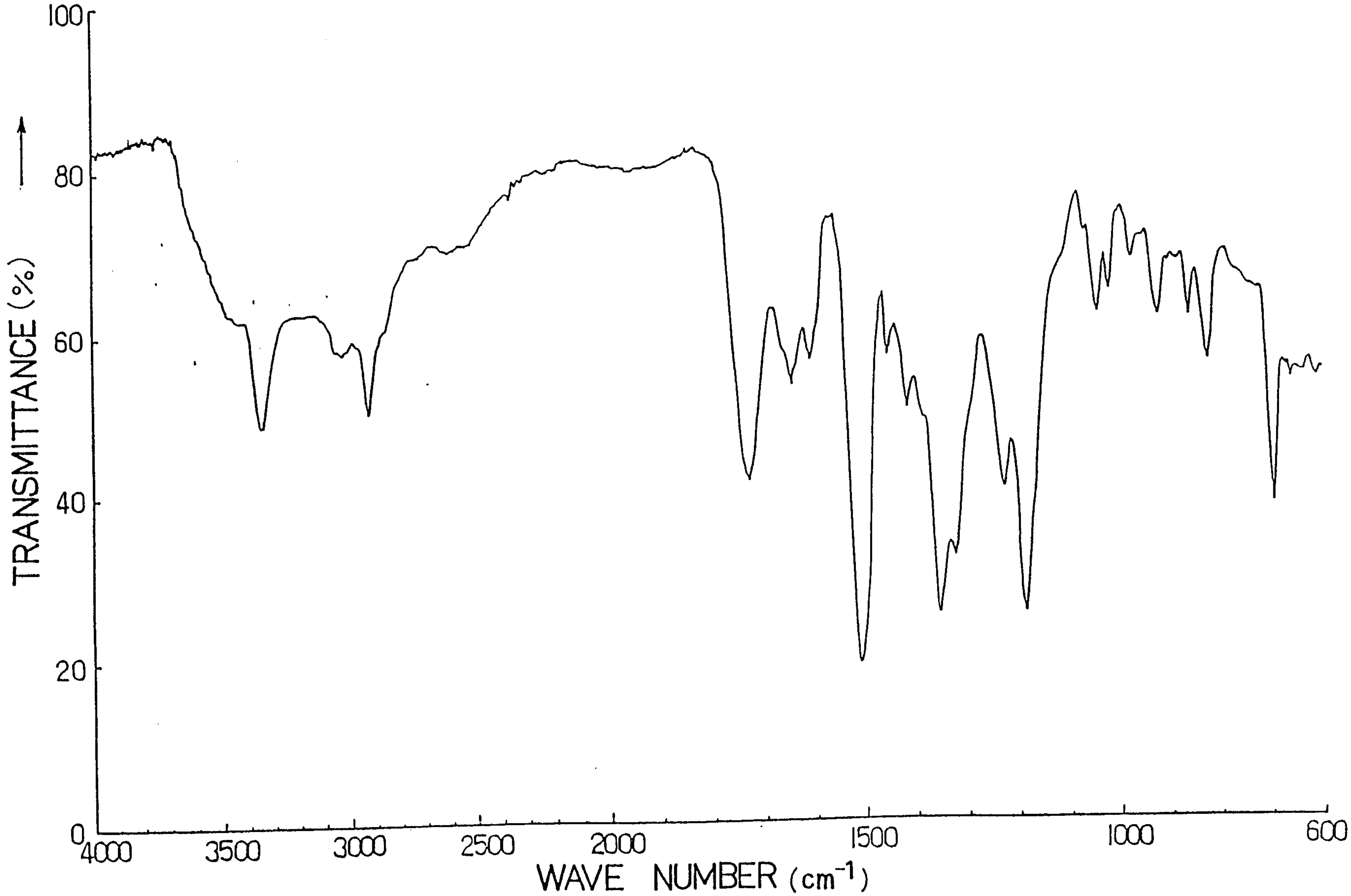
100
80
60
40
20
0
TRANSMITTANCE (%)
4000
3500
3000
2500
2000
1500
1000
600
WAVE NUMBER ($cm^{-1}$)

FIG. 36

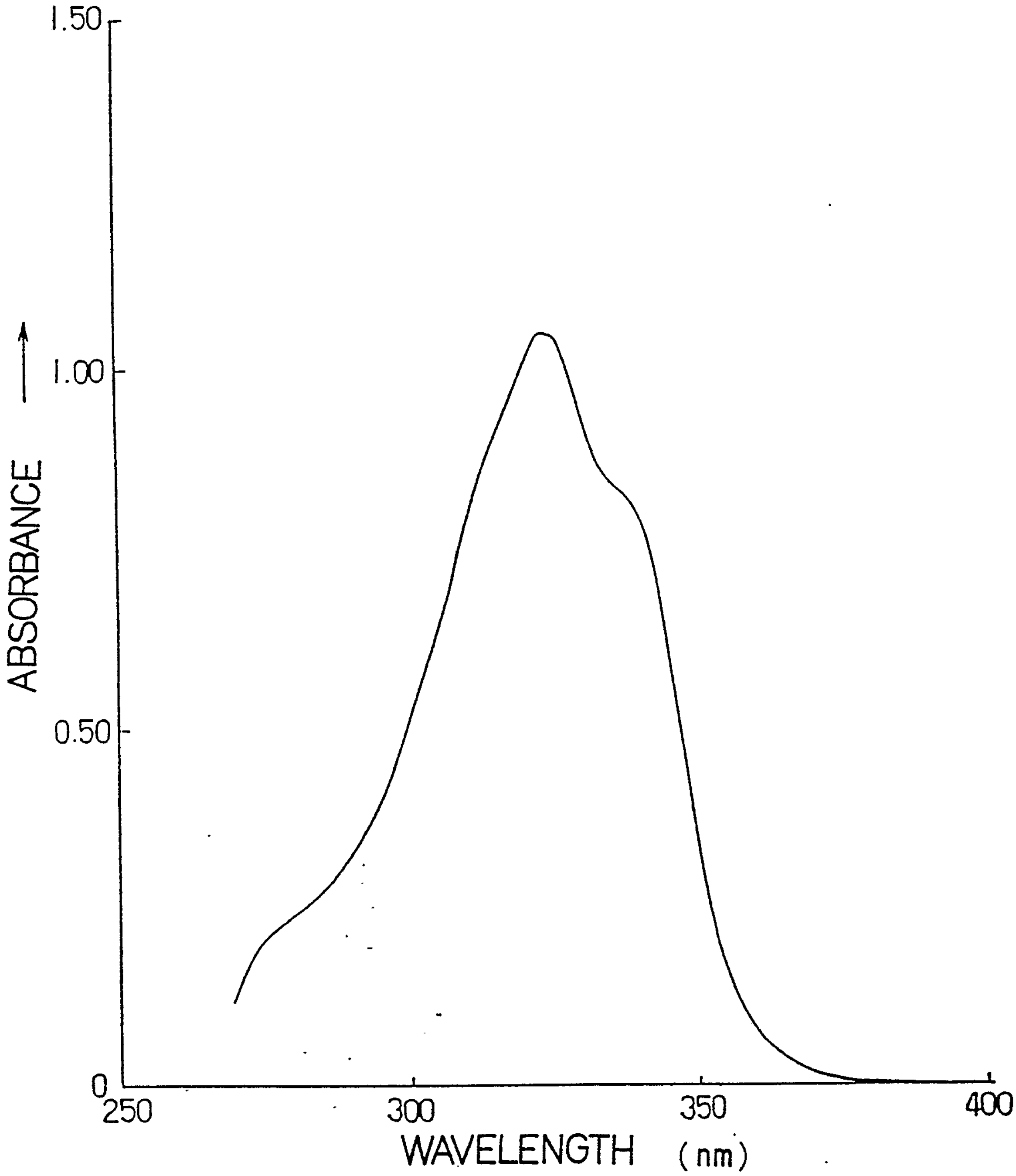

FIG. 37

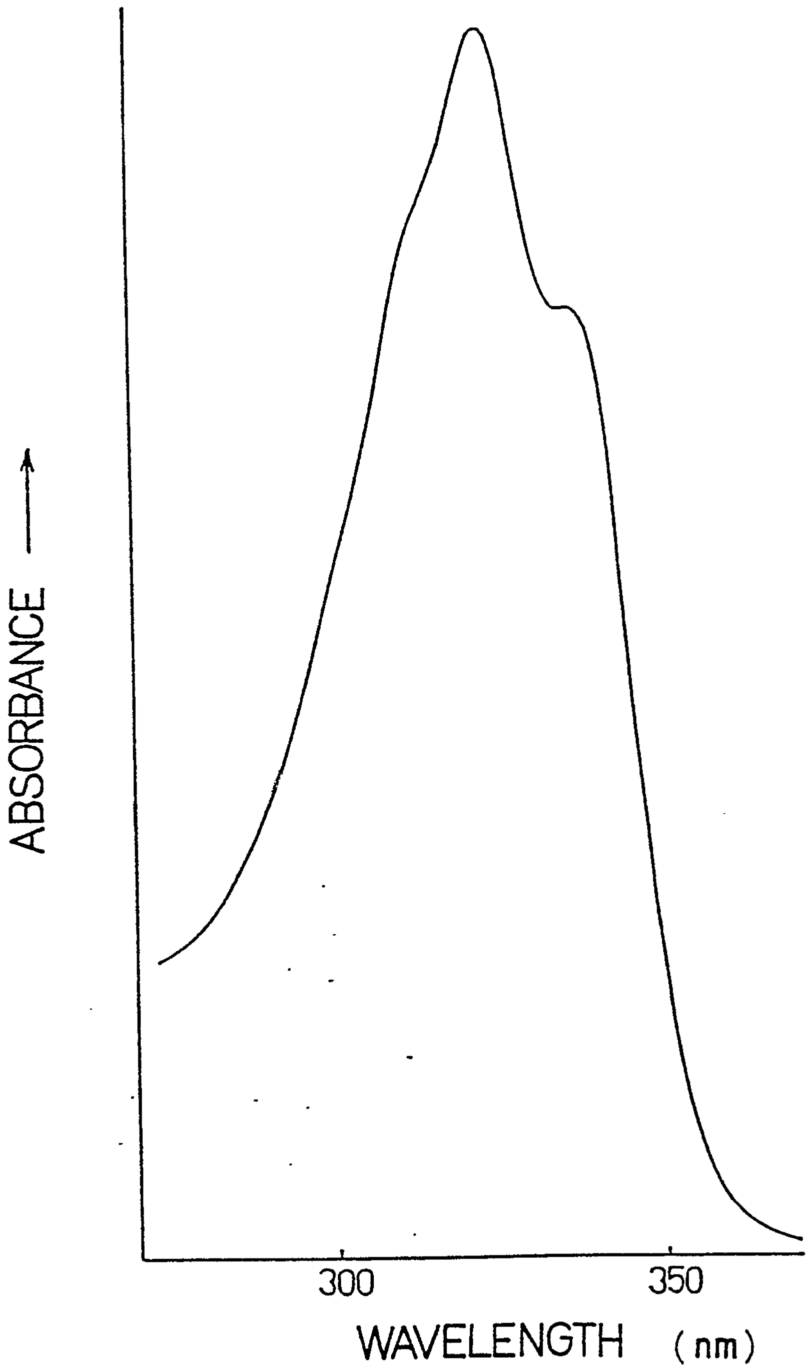

FIG. 38

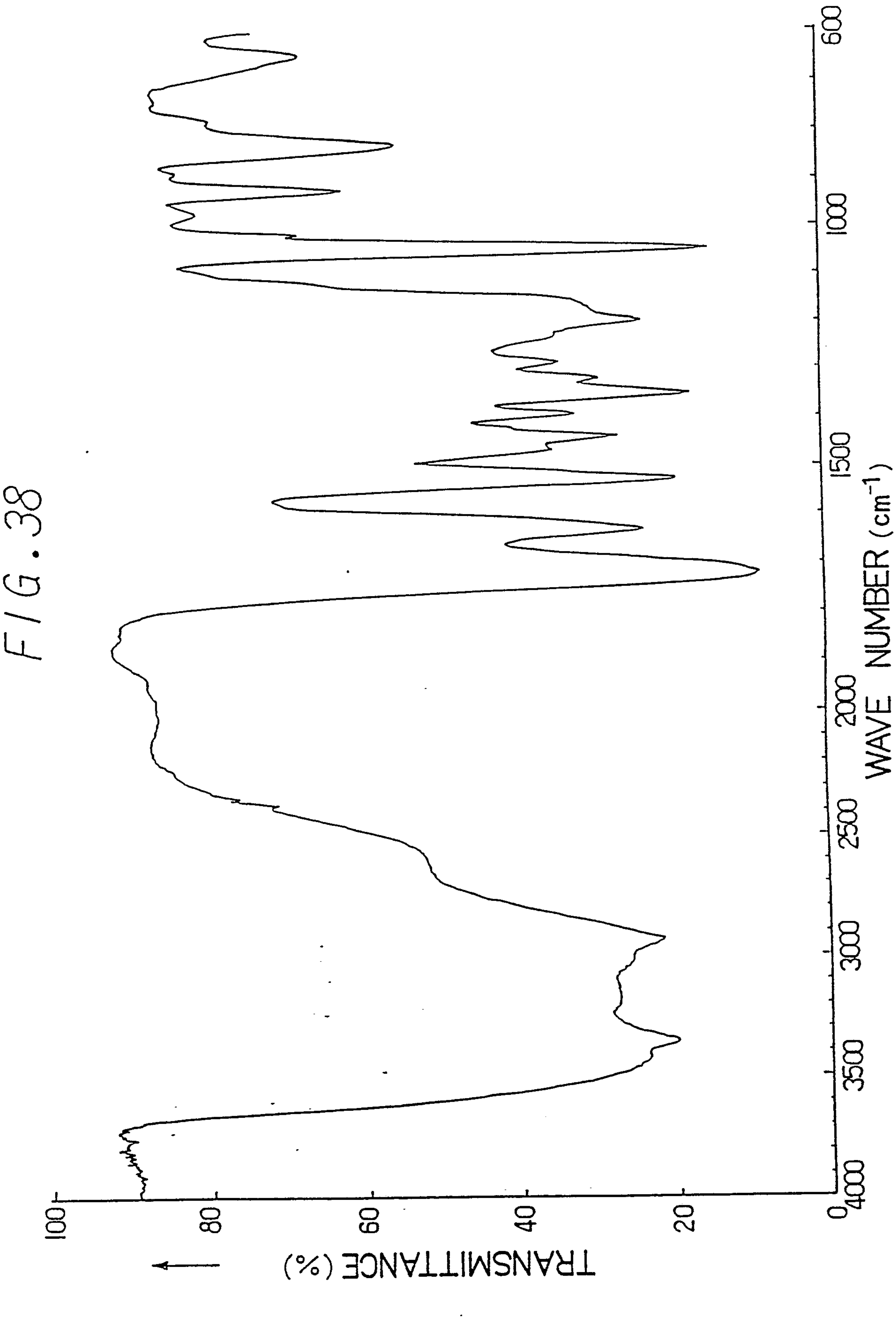
TRANSMITTANCE (%)
100
80
60
40
20
0
4000
3500
3000
2500
2000
1500
1000
600
WAVE NUMBER (cm$^{-1}$)

FIG.39

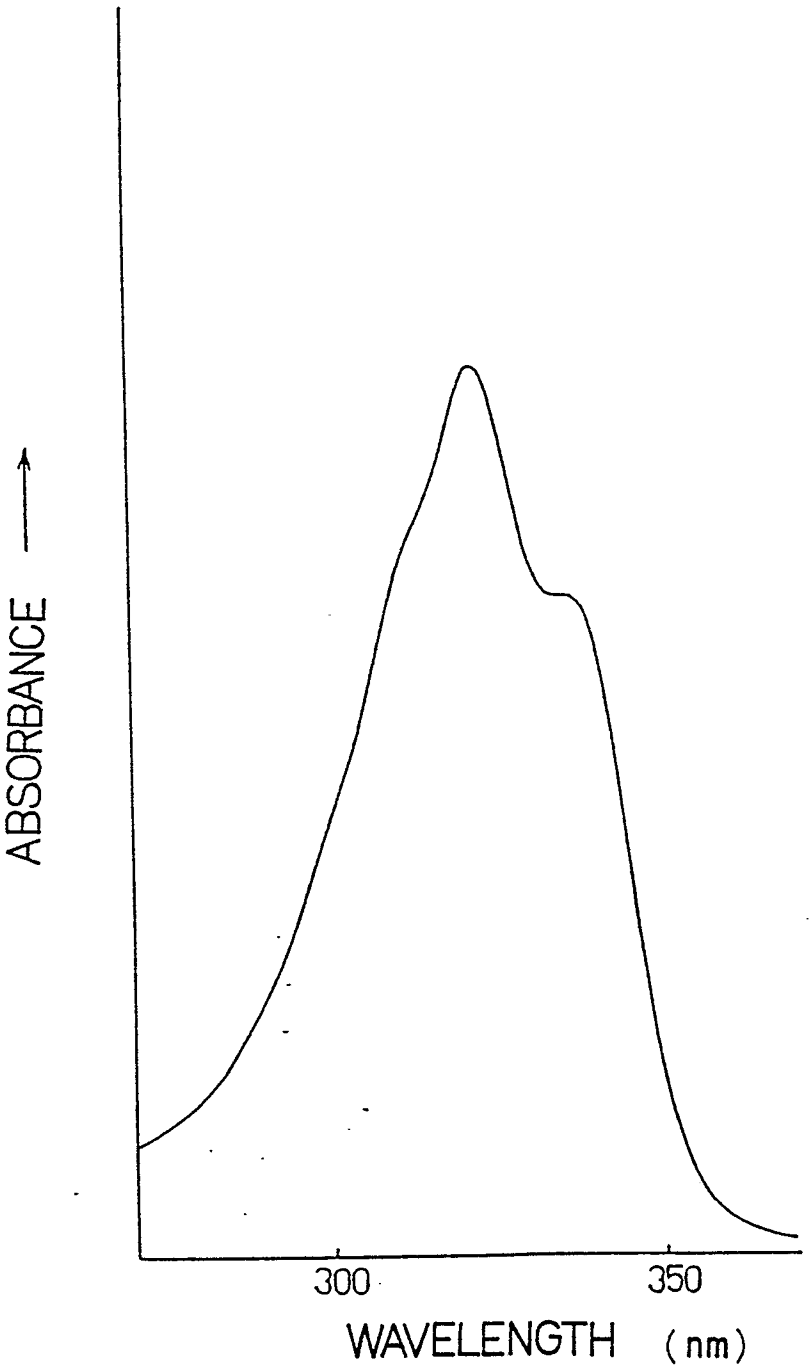

FIG. 40

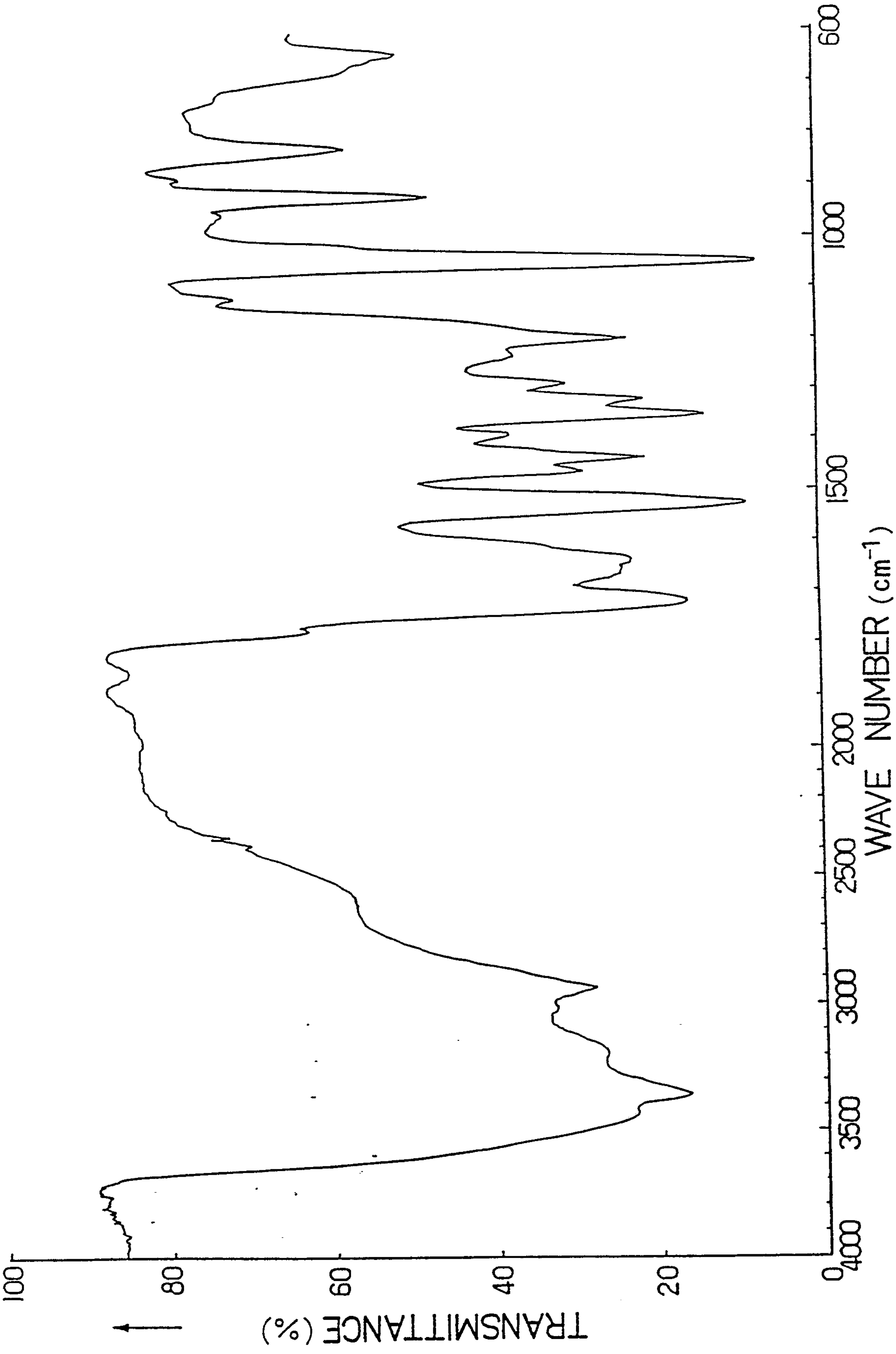
TRANSMITTANCE (%)
100
80
60
40
20
0
4000
3500
3000
2500
2000
1500
1000
600
WAVE NUMBER (cm$^{-1}$)

0 348 513

FIG.41

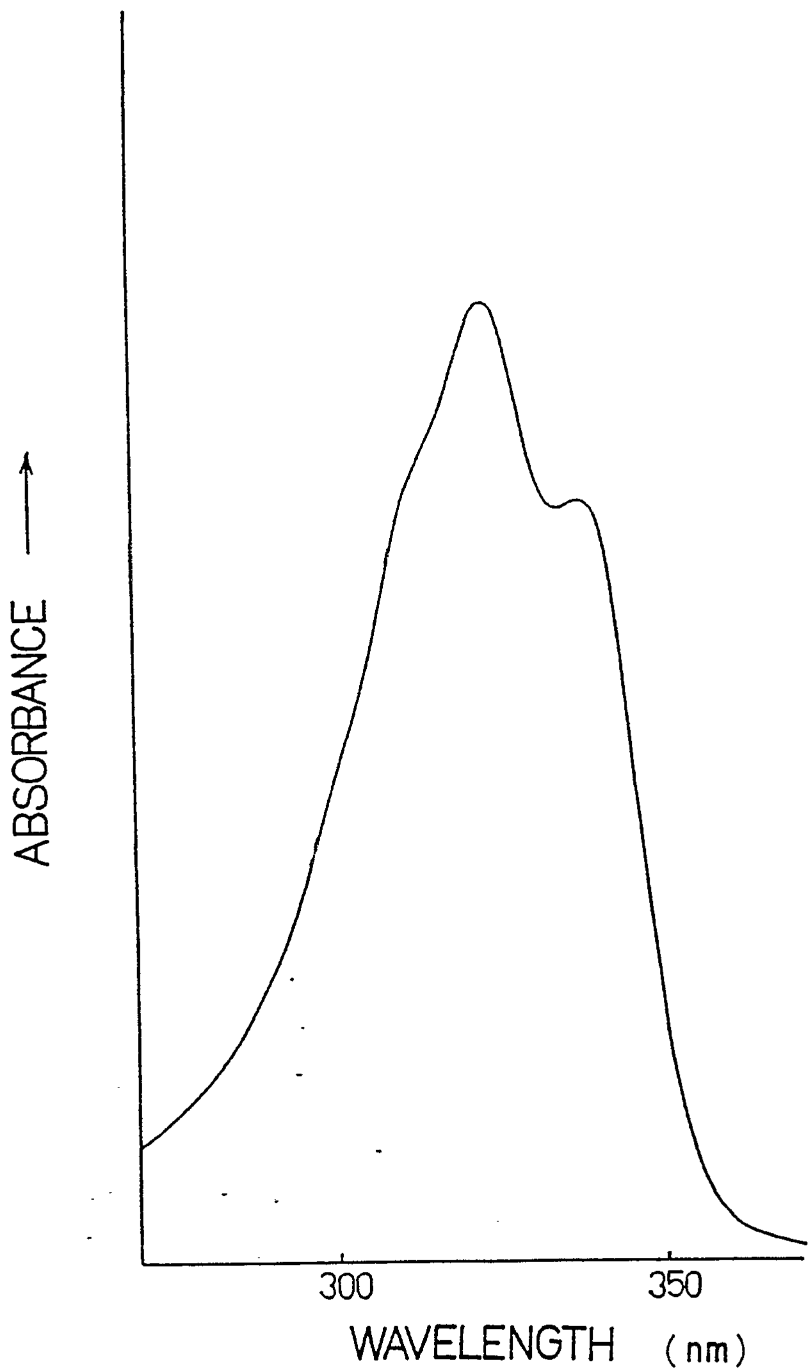

F I G . 42

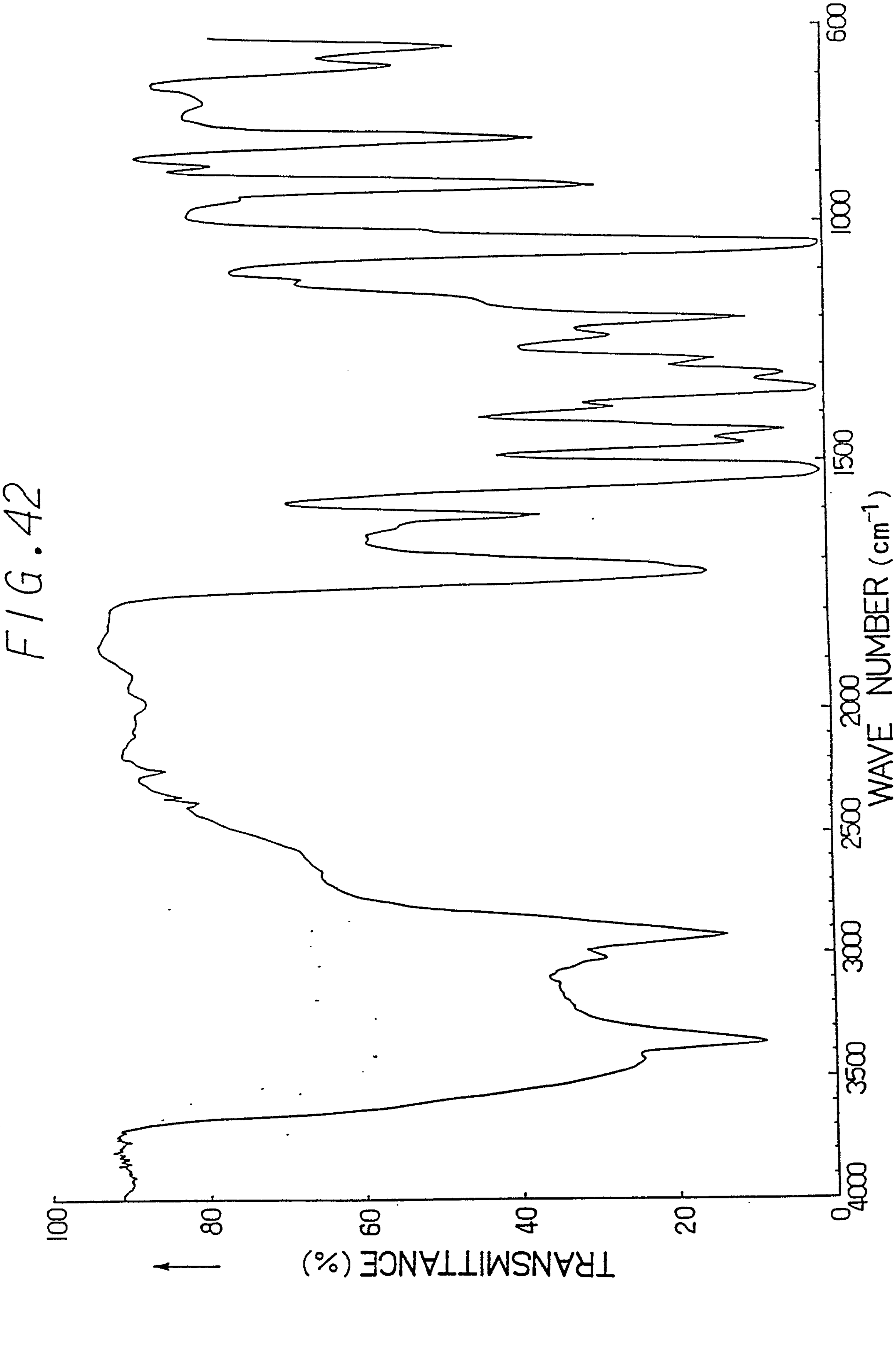
TRANSMITTANCE (%)
100
80
60
40
20
0
4000
3500
3000
2500
2000
1500
1000
600
WAVE NUMBER (cm$^{-1}$)

FIG. 43

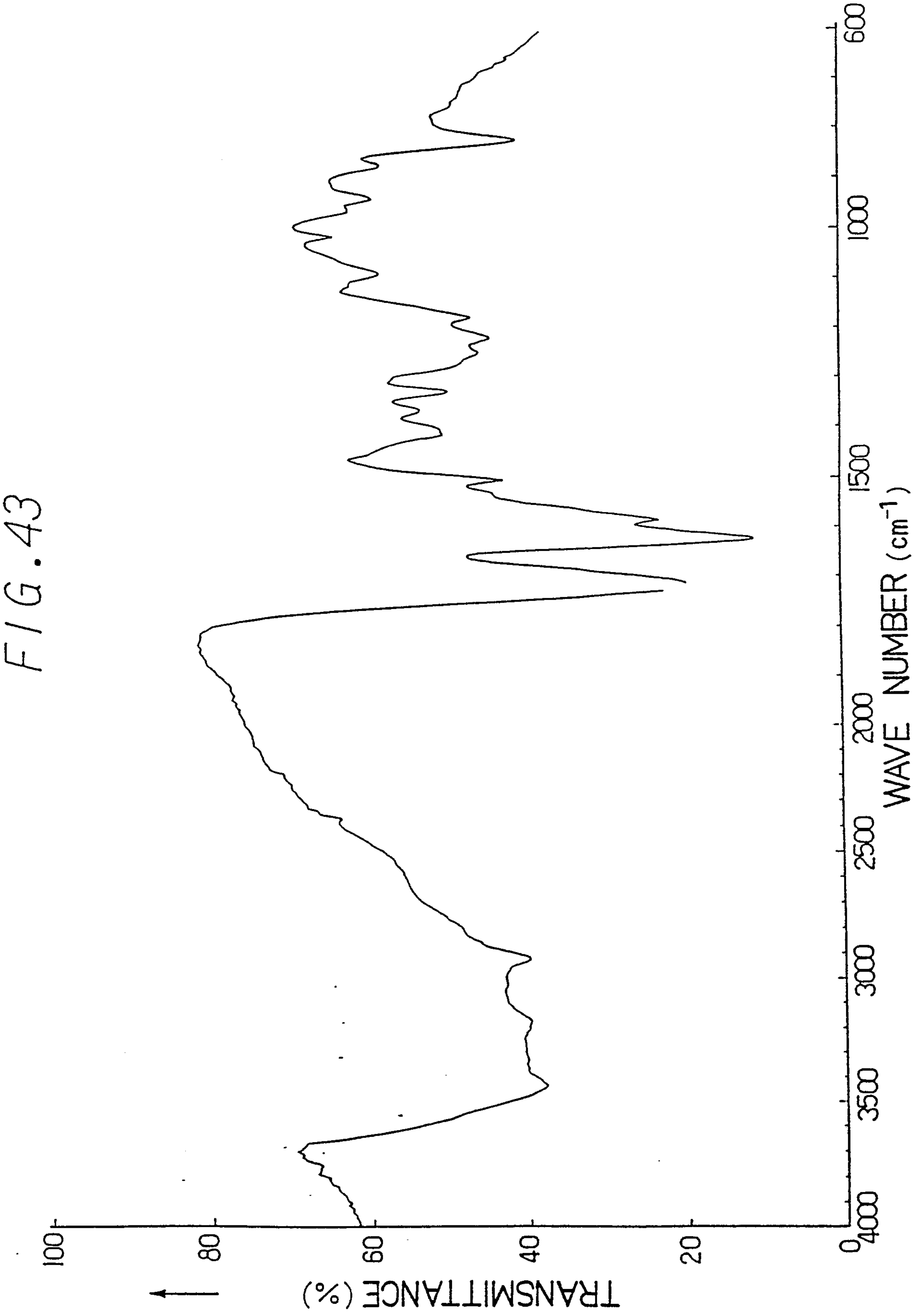
TRANSMITTANCE (%)
100
80
60
40
20
0
4000
3500
3000
2500
2000
1500
1000
600
WAVE NUMBER (cm-1)

FIG.44

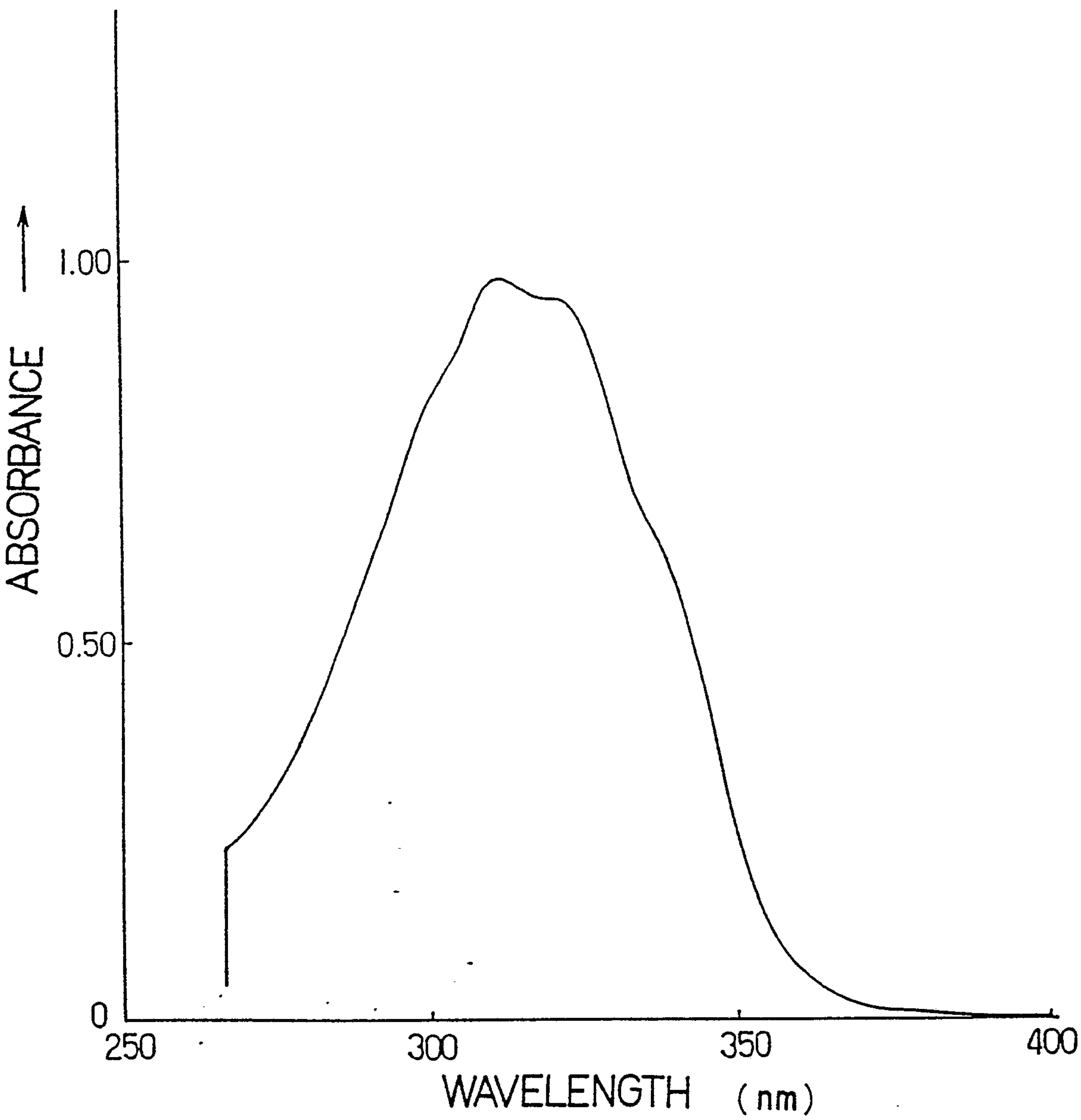

FIG. 45

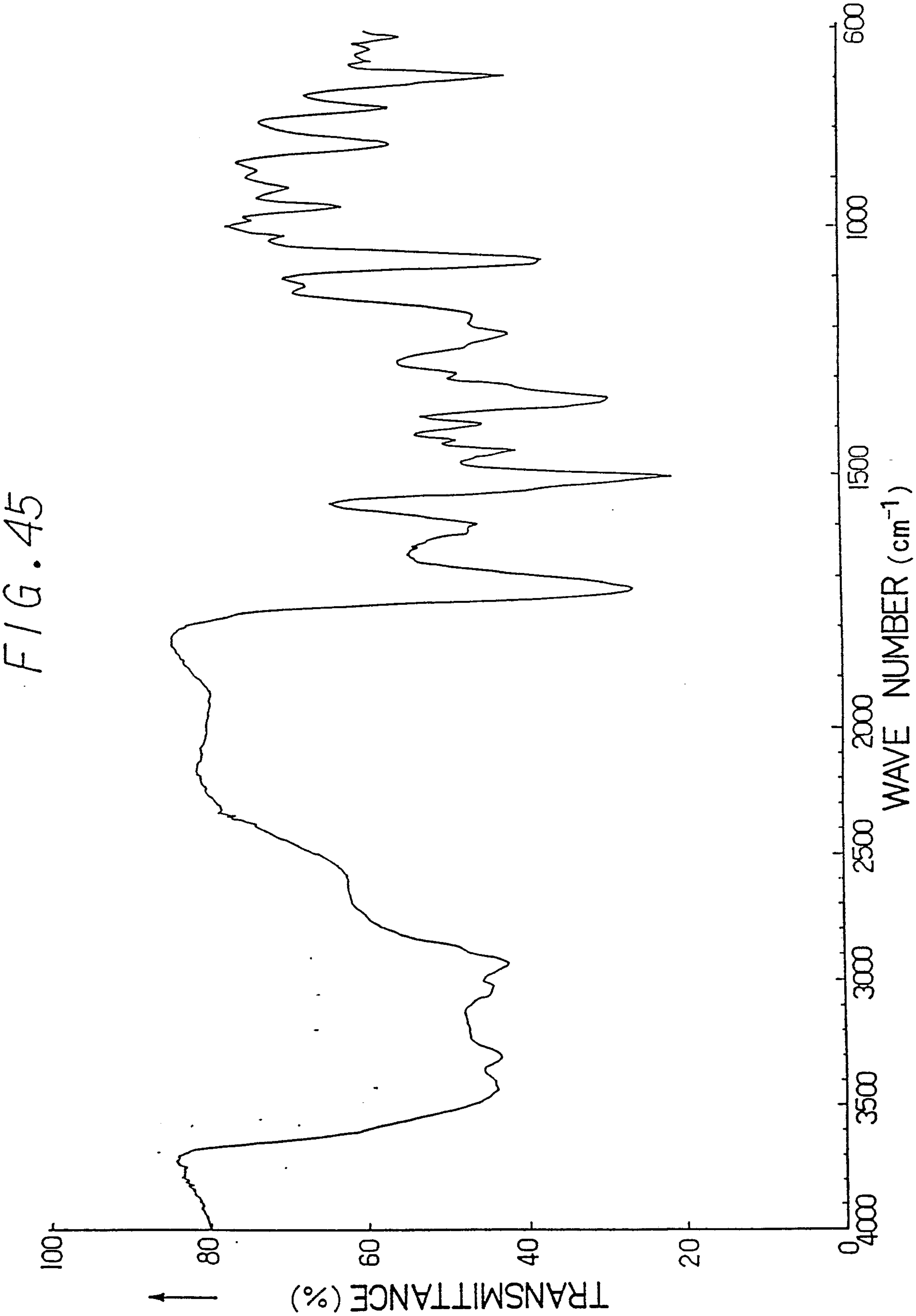
TRANSMITTANCE (%)
100
80
60
40
20
0
4000
3500
3000
2500
2000
1500
1000
600
WAVE NUMBER ($cm^{-1}$)

FIG.46

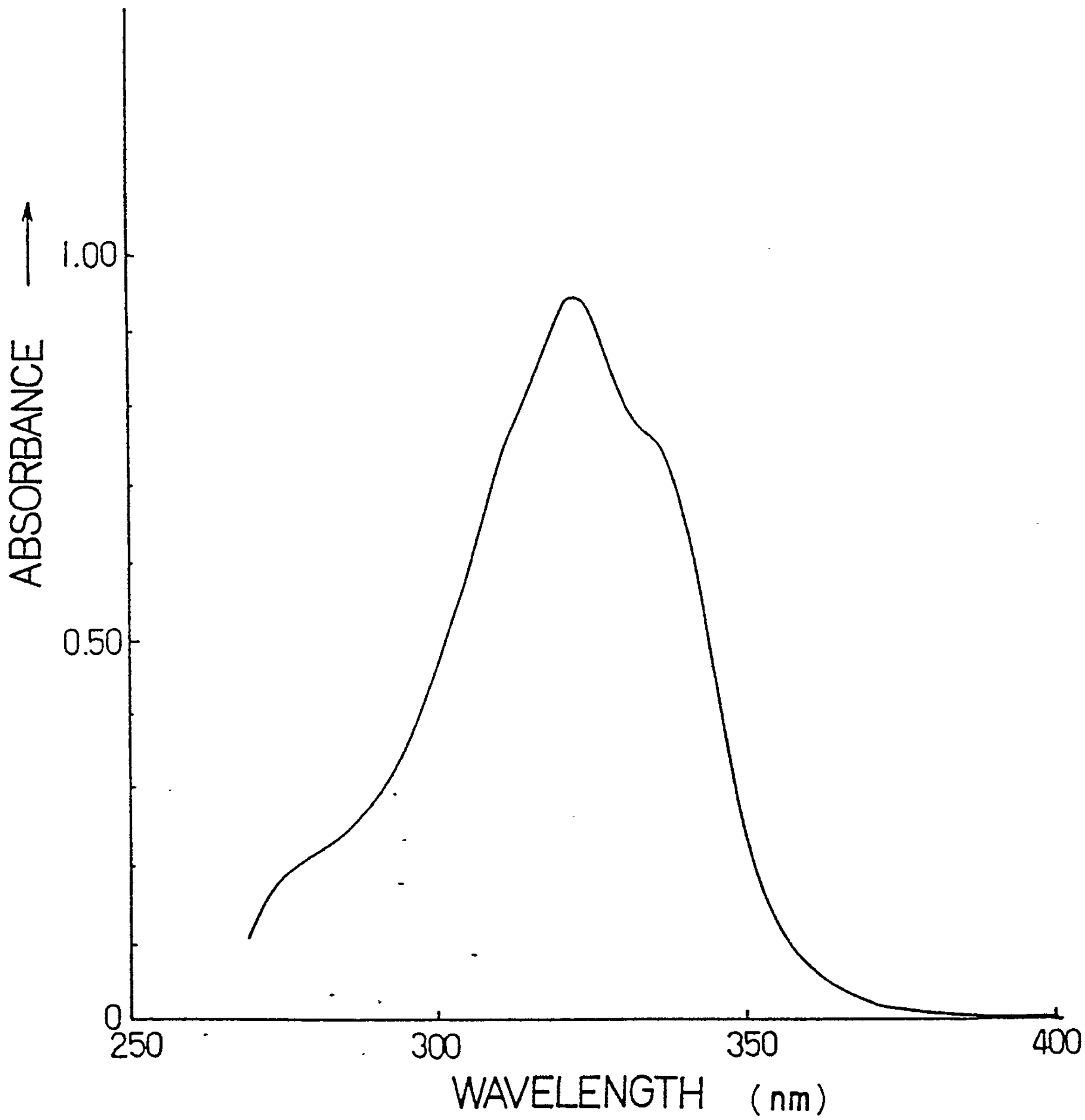

FIG. 47

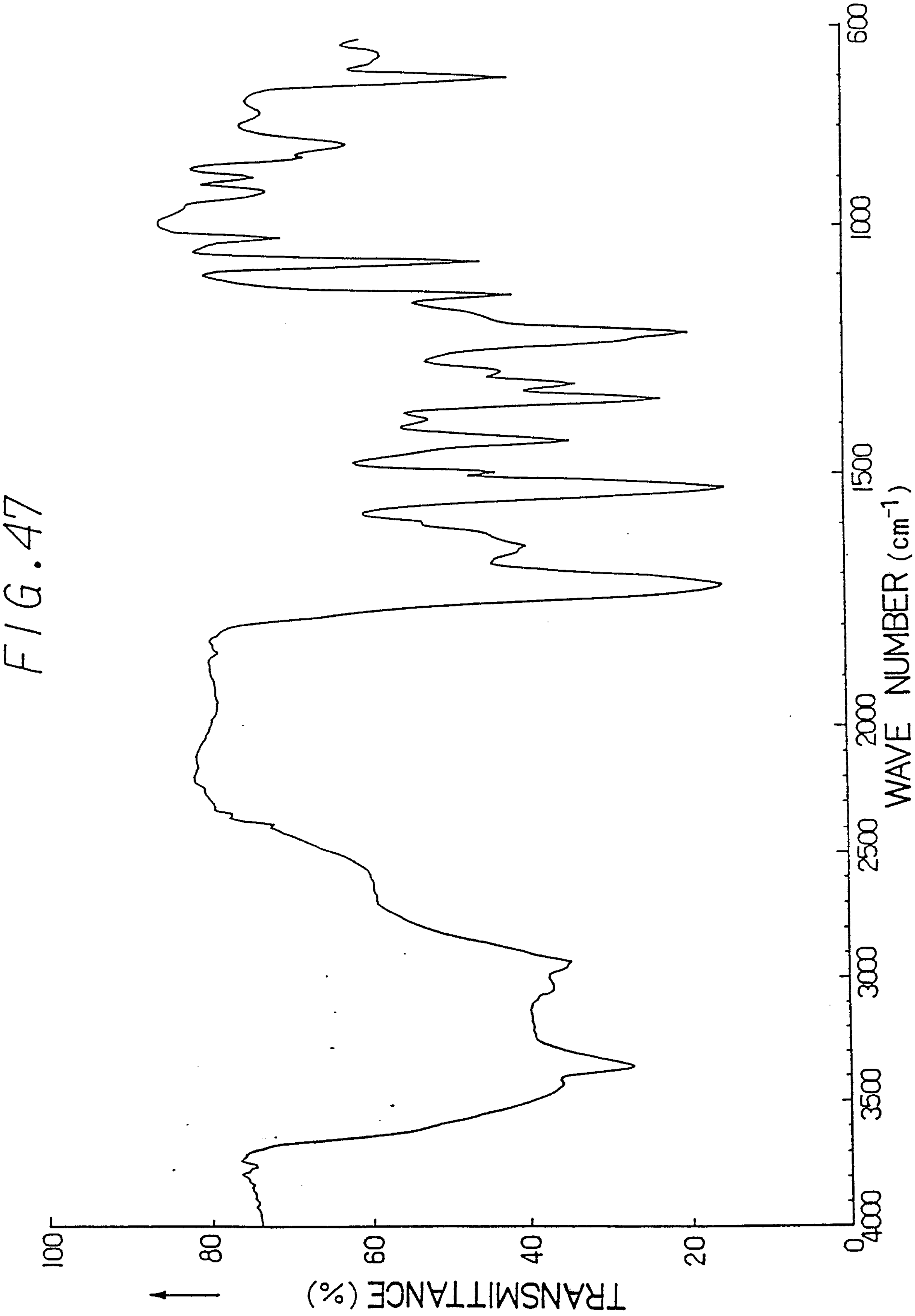
TRANSMITTANCE (%)
100
80
60
40
20
0
4000
3500
3000
2500
2000
1500
1000
600
WAVE NUMBER (cm$^{-1}$)

FIG. 48

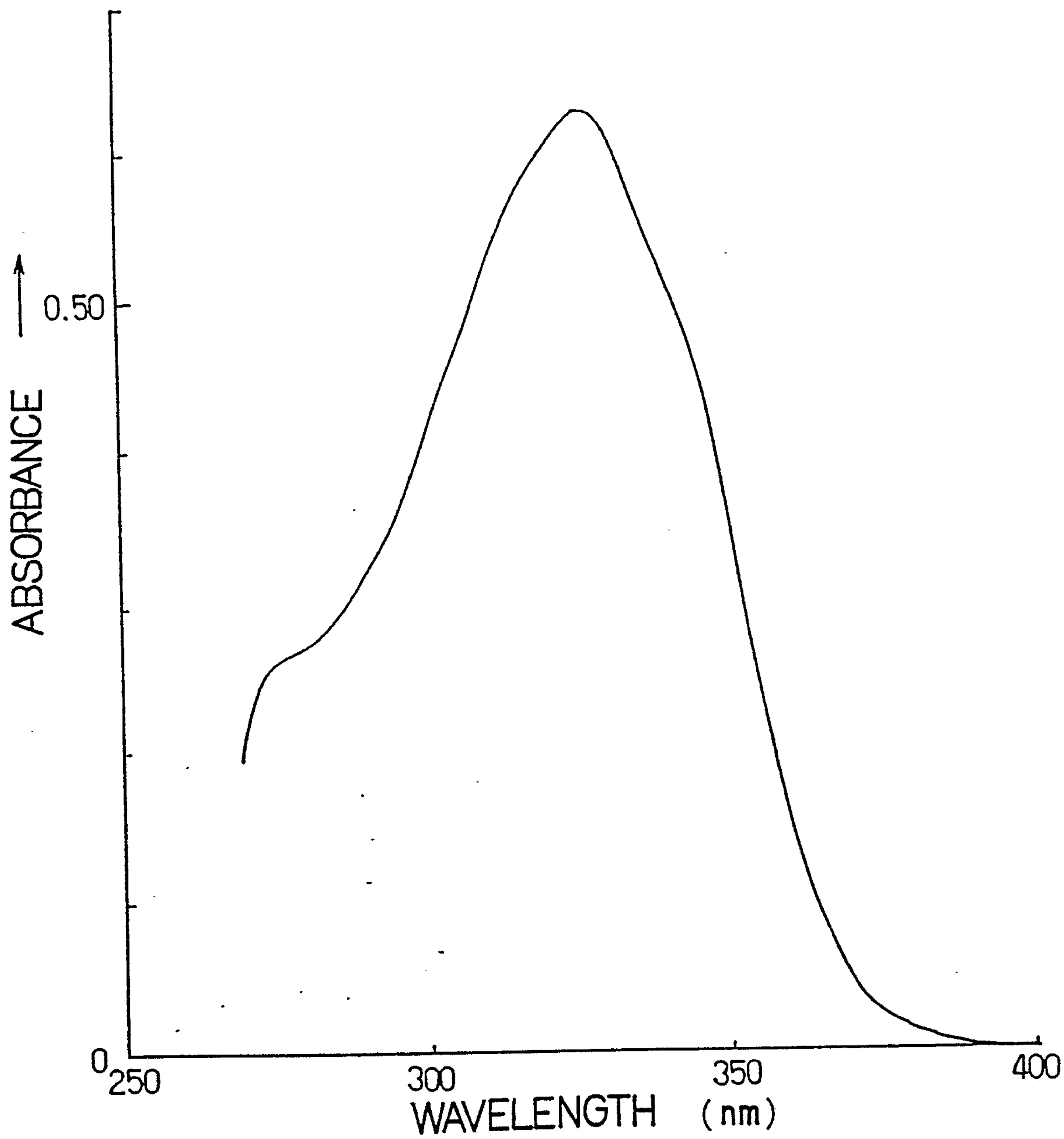

FIG. 49

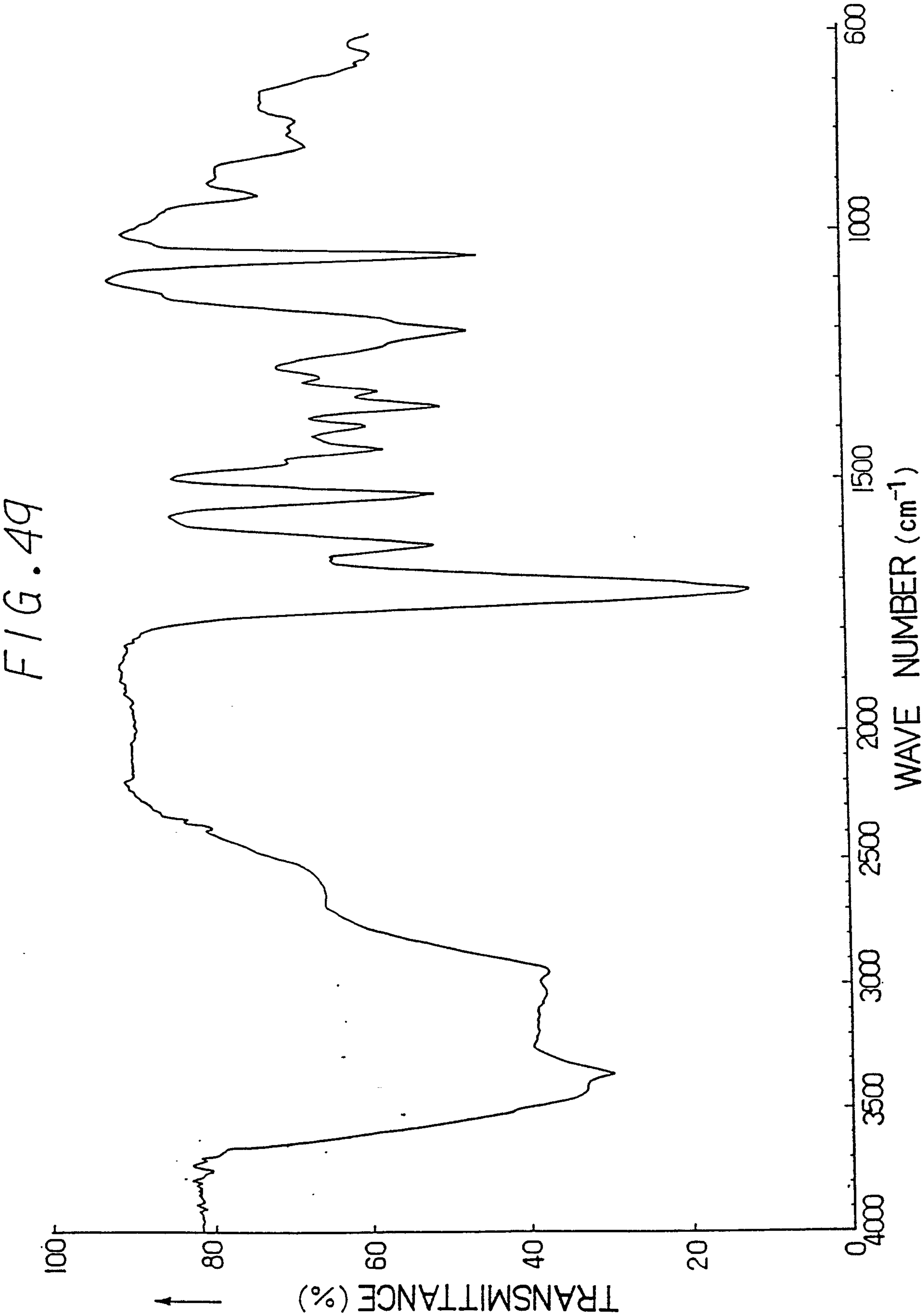
TRANSMITTANCE (%)
100
80
60
40
20
0
4000
3500
3000
2500
2000
1500
1000
600
WAVE NUMBER ($cm^{-1}$)

FIG. 50

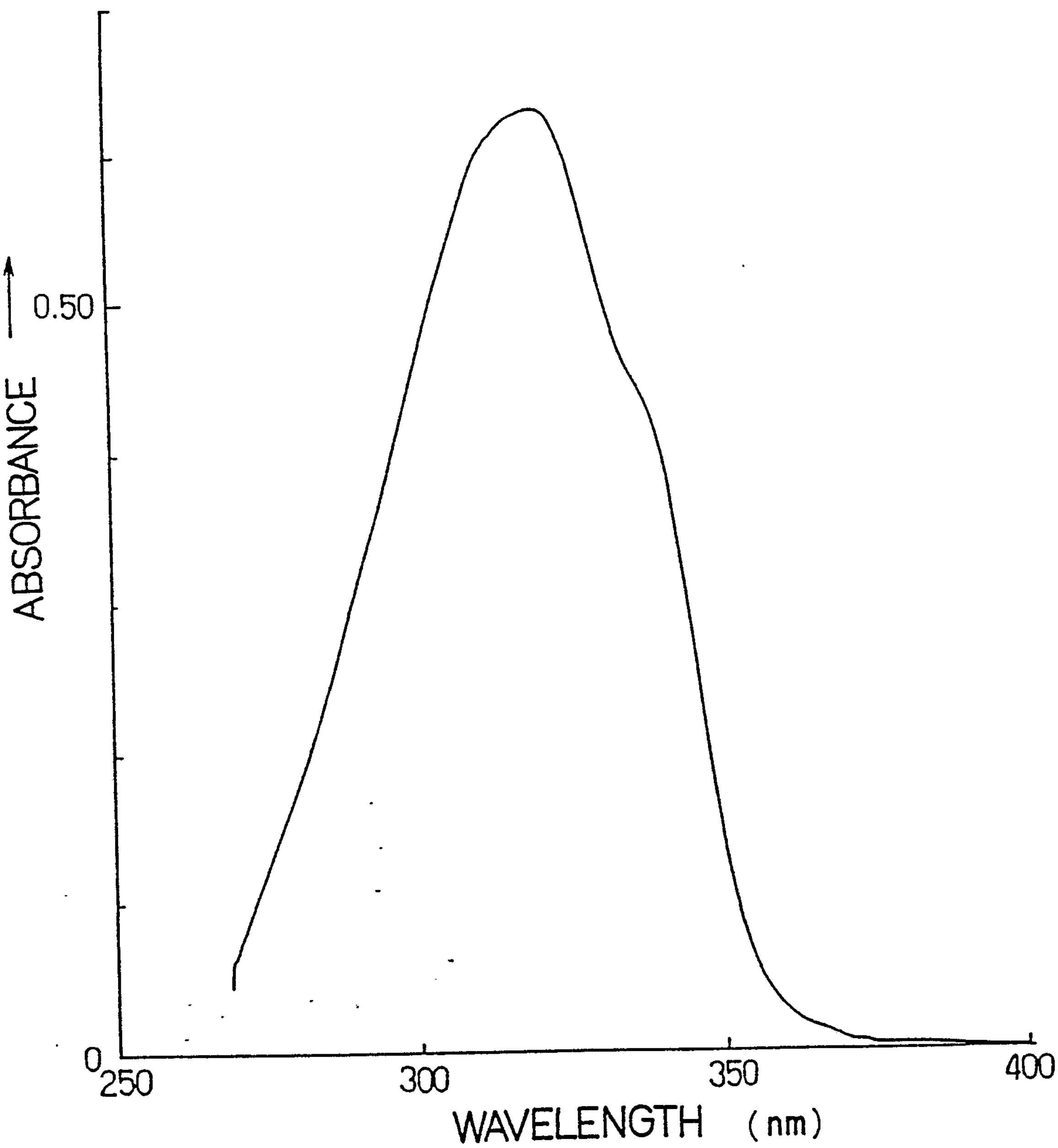

FIG. 51

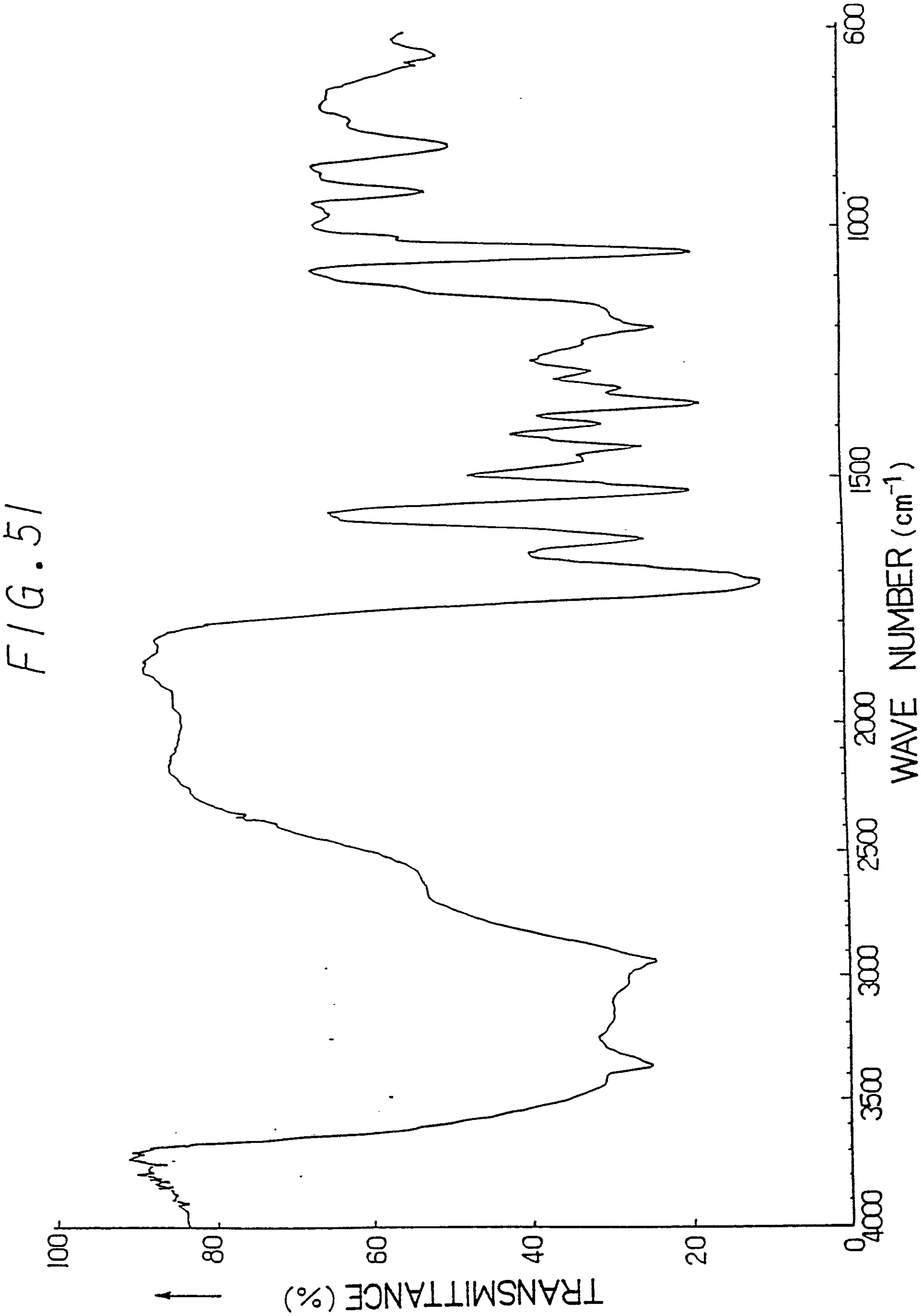
TRANSMITTANCE (%)
0
20
40
60
80
100
WAVE NUMBER (cm$^{-1}$)
4000
3500
3000
2500
2000
1500
1000
600

FIG.52

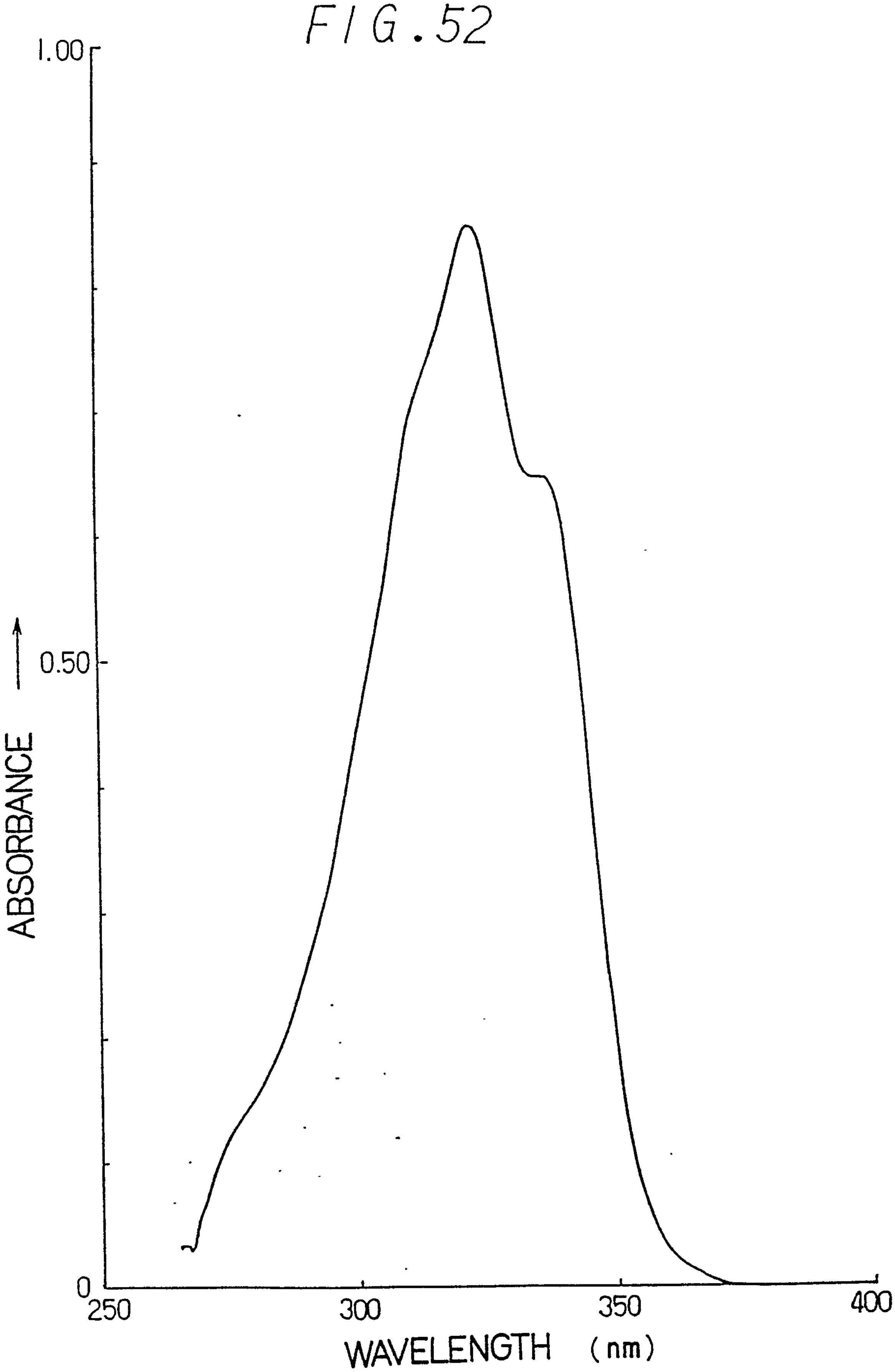

# INTERNATIONAL SEARCH REPORT

International Application No PCT/JP88/01180

**I. CLASSIFICATION OF SUBJECT MATTER** (if several classification symbols apply, indicate all) [6]

According to International Patent Classification (IPC) or to both National Classification and IPC

Int.Cl[4] C07C133/08, C07C159/00, C08F226/00, C09K3/00

**II. FIELDS SEARCHED**

Minimum Documentation Searched [7]

| Classification System | Classification Symbols |
|---|---|
| IPC | C07C133/00-133/08, C07C159/00, C07F226/00, C09K3/00 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 51-131852 (N.V. Philips' Gloeilampenfabrieken) 16 November 1976 (16. 11. 76) Pages 3 to 5 ni Kisai no Kagobutsu & US, A, 3885042 & FR, A, 2171217 & DE, A, 2365948 | 1, 3-4 |
| A | JP, A, 59-164768 (Nippon Soda Co., Ltd.) 17 September 1984 (17. 09. 84) Claim 1 (Family: none) | 1, 3-4 |
| A | JP, A, 56-65865 (Maruho Kabushiki Kaisha) 3 June 1981 (03. 06. 81) Claim (Family: none) | 1-14 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 26, 1989 (26. 01. 89) | February 6, 1989 (06. 02. 89) |
| **International Searching Authority** | **Signature of Authorized Officer** |
| Japanese Patent Office | |